# EUROPEAN PATENT SPECIFICATION

(11) **EP 2 179 292 B1**
(45) Date of publication and mention of the grant of the patent: **28.11.2012**
(21) Application number: 08827819.7
(22) Date of filing: 15.08.2008
(51) Int. Cl.: G01N 33/574

(54) **METABOLOMIC PROFILING OF PROSTATE CANCER**
METABOLOMISCHE PROFILIERUNG VON PROSTATAKREBS
PROFILE METABOLOMIQUE DU CANCER DE LA PROSTATE

(30) Priority: 16.08.2007 US 956239 P; 25.06.2008 US 75540 P; 27.06.2008 US 133279 P
(43) Date of publication of application: 28.04.2010
(73) Proprietor: The Regents of the University of Michigan, Ann Arbor, MI 48104-2592 (US); Metabolon Inc., Durham, NC 27713 (US)
(72) Inventor: CHINNAIYAN, Arul, M., Plymouth, Michigan 48170 (US); SREEKUMAR, Arun, Ann Arbor, Michigan 48105 (US); MITCHELL, Matthew, W., Durham, North Carolina 27707 (US); LAWTON, Kay, A., Raleigh, North Carolina 27612 (US); BERGER, Alvin, Long Lake, MN 55356-8318 (US); BEECHER, Christopher, Ann Arbor, MI 48105 (US)
(74) Representative: Glawe, Delfs, Moll
(86) International application number: PCT/US2008/073318
(87) International publication number: WO 2009/026152

(56) References cited:
- WO-A2-2008/036691
- US-A1- 2007 078 093
- REININGHAUS F ET AL: "PROTON MRS IN HUMAN URINE FROM CANCER PATIENTS A SEARCH FOR TUMOR MARKERS" PROCEEDINGS OF THE ANNUAL MEETING OF THE AMERICAN ASSOCIATION FOR CANCER RESEARCH, AMERICAN ASSOCIATION FOR CANCER RESEARCH, US, vol. 33, 1 January 1992 (1992-01-01), page 201, XP001537345 ISSN: 0197-016X
- MARBERGER H ET AL: "Citric acid in human prostatic secretion and metastasizing cancer of prostate gland" BMJ. BRITISH MEDICAL JOURNAL, LONDON, GB, vol. 1, no. 5281, 24 March 1962 (1962-03-24), pages 835-836, XP002580044 ISSN: 0959-8146
- CHIOU CHIUAN-CHIAN ET AL: "Urinary 8-hydroxydeoxyguanosine and its analogs as DNA marker of oxidative stress: Development of an ELISA and measurement in both bladder and prostate cancers" CLINICA CHIMICA ACTA, ELSEVIER BV, AMSTERDAM, NL LNKD- DOI:10.1016/S0009-8981(03)00191-8, vol. 334, no. 1-2, 1 August 2003 (2003-08-01), pages 87-94, XP002580043 ISSN: 0009-8981 [retrieved on 2003-06-11]
- HASUMI MASARU ET AL: "MR spectroscopy as a reliable diagnostic tool for localization of prostate cancer" ANTICANCER RESEARCH, INTERNATIONAL INSTITUTE OF ANTICANCER RESEARCH, GR, vol. 22, no. 2B, 1 March 2002 (2002-03-01) , pages 1205-1208, XP009132765 ISSN: 0250-7005
- HUZJAN RENATA ET AL: "Magnetic resonance imaging and magnetic resonance spectroscopic imaging of prostate cancer" NATURE CLINICAL PRACTICE UROLOGY, NATURE PUBLISHING GROUP, LONDON, GB, vol. 2, no. 9, 1 September 2005 (2005-09-01), pages 434-442, XP009132764 ISSN: 1743-4270
- JORDAN KATE W ET AL: "NMR-based metabolomics approach to target biomarkers for human prostate cancer" EXPERT REVIEW OF PROTEOMICS, FUTURE DRUGS, LONDON, GB LNKD- DOI:10.1586/14789450.4.3.389, vol. 4, no. 3, 1 June 2007 (2007-06-01), pages 389-400, XP008123021 ISSN: 1744-8387
- STRUYS E A ET AL: "Serum sarcosine is not a marker for prostate cancer" ANNALS OF CLINICAL BIOCHEMISTRY 2010 ROYAL SOCIETY OF MEDICINE PRESS LTD GBR LNKD- DOI:10.1258/ACB.2010.009270, vol. 47, no. 3, May 2010 (2010-05), page 282, XP008124035
- JENTZMIK F ET AL: "Sarcosine in Urine after Digital Rectal Examination Fails as a Marker in Prostate Cancer Detection and Identification of Aggressive Tumours" EUROPEAN UROLOGY 2010 ELSEVIER NLD LNKD- DOI:10.1016/J.EURURO.2010.01.035, vol. 58, no. 1, July 2010 (2010-07), pages 12-18, XP002590535 ISSN: 0302-2838
- SREEKUMAR ARUN ET AL: "Metabolomic profiles delineate potential role for sarcosine in prostate cancer progression." 12 February 2009 (2009-02-12), NATURE 12 FEB 2009 LNKD- PUBMED:19212411, VOL. 457, NR. 7231, PAGE(S) 910 - 914 , XP002590536 ISSN: 1476-4687 * the whole document *
- FUKUDA, HIROYUKI ET AL: 'Increased metabolizing activities of the tricarboxylic acid cycle and decreased drug metabolism in hepatocellular carcinoma' CARCINOGENESIS vol. 23, no. 12, December 2002, pages 2019 - 2023, XP008129823
- SOUTSCHEK, JURGEN ET AL: 'Therapeutic silencing of an endogeneous gene by systemic administracion of modified siRNAs' NATURE vol. 432, no. 7014, November 2004, pages 173 - 178, XP002333747
- FISCHER, TAN ET AL: 'Application of RNAi to cancer research and therapy' FRONTIERS IN BIOSCIENCE vol. 10, no. 2, May 2005, pages 1946 - 1960, XP008129812
- JASON, TLH ET AL: 'Toxicology of antisense therapeutics' TOXICOLOGY AND APPLIED PHARMACOLOGY vol. 201, no. 1, November 2004, pages 66 - 83, XP004616827
- SHARMA, R. ET AL: 'Downregulation of drug transport and metabolism in mice bearing extra-hepatic malignancies' BRITISH JOURNAL OF CANCER vol. 98, no. 1, 04 December 2007, pages 91 - 97, XP008129827

## Description

### FIELD OF THE INVENTION

The present invention is defined in the claims and relates to cancer markers. In particular, the present invention provides metabolites that are differentially present in prostate cancer. The present invention further provides diagnostic, research, and therapeutic applications targeting cancer specific metabolites.

### BACKGROUND OF THE INVENTION

Afflicting one out of nine men over age 65, prostate cancer (PCA) is a leading cause of male cancer-related death, second only to lung cancer (Abate-Shen and Shen, Genes Dev 14:2410 [2000]; Ruijter et al., Endocr Rev, 20:22 [1999]). The American Cancer Society estimates that about 184,500 American men will be diagnosed with prostate cancer and 39,200 will die in 2001.

Prostate cancer is typically diagnosed with a digital rectal exam and/or prostate specific antigen (PSA) screening. An elevated serum PSA level can indicate the presence of PCA. PSA is used as a marker for prostate cancer because it is secreted only by prostate cells. A healthy prostate will produce a stable amounttypically below 4 nanograms per milliliter, or a PSA reading of "4" or less -- whereas cancer cells produce escalating amounts that correspond with the severity of the cancer. A level between 4 and 10 may raise a doctor's suspicion that a patient has prostate cancer, while amounts above 50 may show that the tumor has spread elsewhere in the body.

When PSA or digital tests indicate a strong likelihood that cancer is present, a transrectal ultrasound (TRUS) is used to map the prostate and show any suspicious areas. Biopsies of various sectors of the prostate are used to determine if prostate cancer is present. Treatment options depend on the stage of the cancer. Men with a 10-year life expectancy or less who have a low Gleason number and whose tumor has not spread beyond the prostate are often treated with watchful waiting (no treatment). Treatment options for more aggressive cancers include surgical treatments such as radical prostatectomy (RP), in which the prostate is completely removed (with or without nerve sparing techniques) and radiation, applied through an external beam that directs the dose to the prostate from outside the body or via low-dose radioactive seeds that are implanted within the prostate to kill cancer cells iocaiiy. Anti-androgen hormone therapy is also used, alone or in conjunction with surgery or radiation. Hormone therapy uses luteinizing hormone-releasing hormones (LH-RH) analogs, which block the pituitary from producing hormones that stimulate testosterone production. Patients must have injections of LH-RH analogs for the rest of their lives.

While surgical and hormonal treatments are often effective for localized PCA, advanced disease remains essentially incurable. Androgen ablation is the most common therapy for advanced PCA, leading to massive apoptosis of androgen-dependent malignant cells and temporary tumor regression. In most cases, however, the tumor reemerges with a vengeance and can proliferate independent of androgen signals.

The advent of prostate specific antigen (PSA) screening has led to earlier detection of PCA and significantly reduced PCA-associated fatalities. However, the impact of PSA screening on cancer-specific mortality is still unknown pending the results of prospective randomized screening studies (Etzioni et al., J. Natl. Cancer Inst., 91:1033 [1999]; Maattanen et al., Br. J. Cancer 79:1210 [1999]; Schroder et al., J. Natl. Cancer Inst., 90:1817 [1998]). A major limitation of the serum PSA test is a lack of prostate cancer sensitivity and specificity especially in the intermediate range of PSA detection (4-10 ng/ml). Elevated serum PSA levels are often detected in patients with non-malignant conditions such as benign prostatic hyperplasia (BPH) and prostatitis, and provide little information about the aggressiveness of the cancer detected. Coincident with increased serum PSA testing, there has been a dramatic increase in the number of prostate needle biopsies performed (Jacobsen et al., JAMA 274:1445 [1995]). This has resulted in a surge of equivocal prostate needle biopsies (Epstein and Potter J. Urol., 166:402 [2001]). Thus, development of additional serum and tissue biomarkers to supplement PSA,screening is needed. Some tumor markers are known in the art (Reininghaus et al., Proc. AACR., 33:201 [1992]; Marberger et al., Br Med J., 1(5281):835-6 [1962]; Chiou et al., Clin Chim Acta., 334(1-2):87-94 [2003]; Hasumi et al., Anticancer Res., 22(2B): 1205-8 [2002]; Huzjan et al., Nat Clin Pract Urol., 2(9):434-42 [2005]; Jordan et al., Expert Rev Proteomics. 4(3):389-400 [2007]; and WO 2008/036691 A2).

### SUMMARY OF THE INVENTION

The present invention is defined in the claims and relates to cancer markers. In particular, the present invention provides metabolites that are differentially present in prostate cancer. The present disclosure further relates to diagnostic, research, and therapeutic applications targeting cancer specific metabolites.

For example, in some examples, the present invention relates to a method of diagnosing cancer (e.g., prostate cancer), comprising: detecting the presence or absence of one or more (e.g., 2 or more, 3 or more, 5 or more, 10 or more, etc. measured together in a multiplex or panel format) cancer specific metabolites (e.g., sarcosine, cysteine, glutamate, asparagine, glycine, leucine, proline, threonine, histidine, n-acetyl-aspartic acid (N-acetylaspartate (NAA)), inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, citrate, malate, and N-acetytyrosine or thymine) in a sample (e.g., a tissue (e.g., biopsy) sample, a blood sample, a serum sample, or a urine sample) from a subject; and diagnosing cancer based on the presence of the cancer specific metabolite. In some embodiments, the cancer specific metabolite is present in cancerous samples but not non-cancerous samples. In some embodiments, one or more additional cancer markers are detected (e.g., in a panel or multiplex format) along with the cancer specific metabolites. In some examples, the panel detects citrate, malate, and N-acetyl-aspartic acid, and sarcosine.

The present disclosure further relates to a method of screening compounds, comprising: contacting a cell (e.g., a cancer (e.g., prostate cancer) cell) containing a cancer specific metabolite with a test compound; and detecting the level of the cancer specific metabolite. In some examples, the method further comprises the step of comparing the level of the cancer specific metabolite in the presence of the test compound to the level of the cancer specific metabolite in the absence of the cancer specific metabolite. In some examples, the cell is in vitro, in a non-human mammal, or ex vivo. In some examples, the test compound is a small molecule or a nucleic acid (e.g., antisense nucleic acid, a siRNA, or a miRNA) that inhibits the expression of an enzyme involved in the synthesis or breakdown of a cancer specific metabolite. In some examples, the cancer specific metabolite is sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, n-acetyl tyrosine or thymine. In some embodiments, the method is a high throughput method.

The present disclosure further relates to method of characterizing prostate cancer, comprising: detecting the presence or absence of an elevated level of sarcosine in a sample (e.g., a tissue sample, a blood sample, a serum sample, or a urine sample) from a subject diagnosed with cancer; and characterizing the prostate cancer based on the presence or absence of the elevated levels of sarcosine. In some examples, the presence of an elevated level of sarcosine in the sample is indicative of invasive prostate cancer in the subject.

In some examples the present disclosure relates to a method of inhibiting growth of a cell (e.g., a cancer cell), comprising contacting a cell with a compound under conditions such that the compound increases or decreases the level of a cancer specific metabolite (e.g., sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, glycine-N-methyl transferase, or thymine). In some examples, the compound is a small molecule or a nucleic acid (e.g., antisense nucleic acid, a siRNA, or a miRNA) that inhibits the expression of an enzyme involved in the synthesis or breakdown of a cancer specific metabolite.

Additional examples relating to the present invention are described in the detailed description and experimental sections below.

### DESCRIPTION OF THE FIGURES

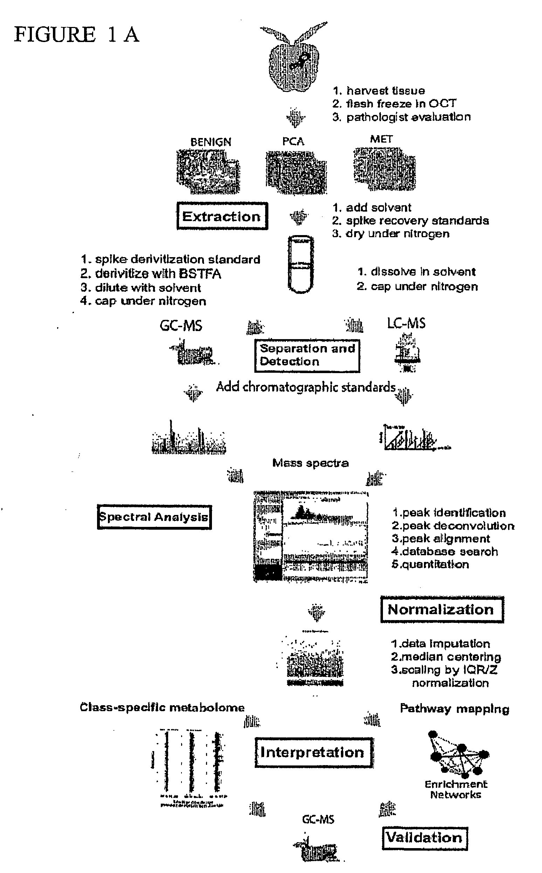

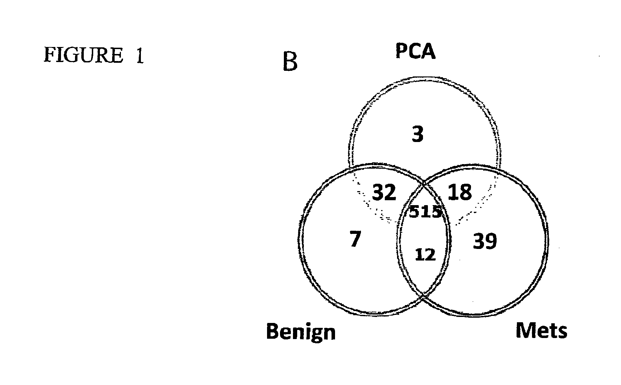

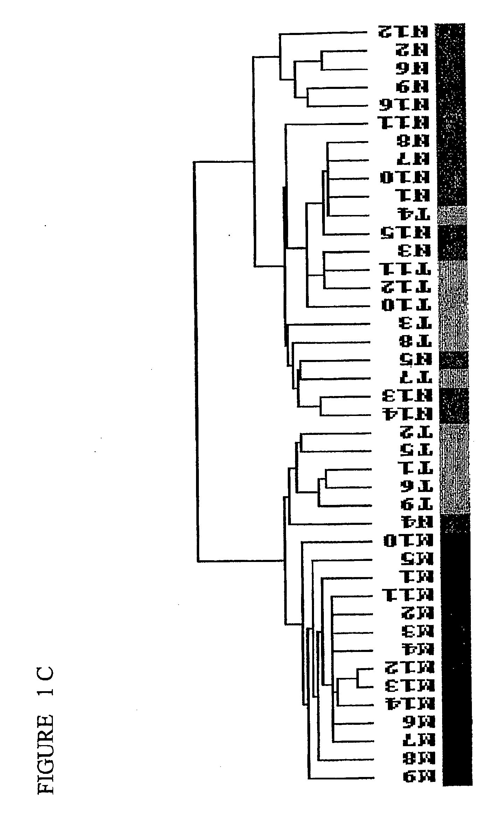

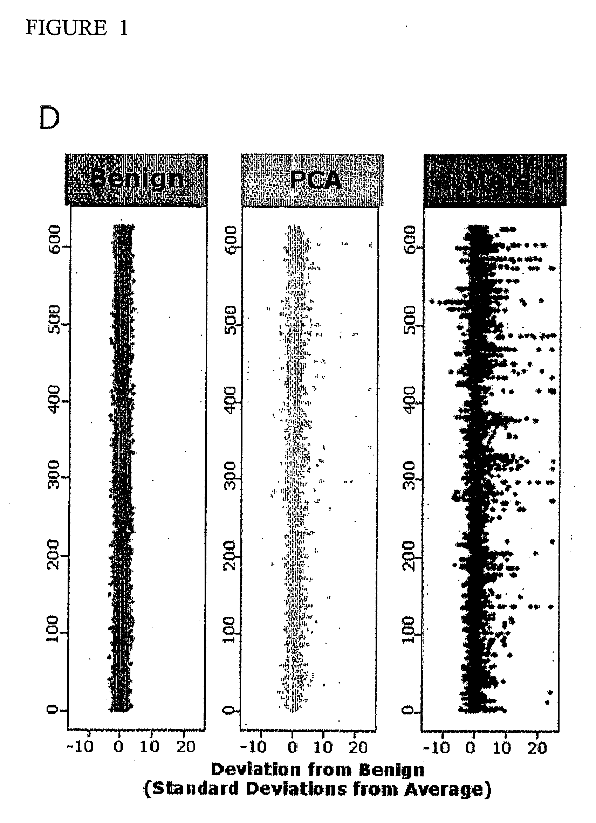

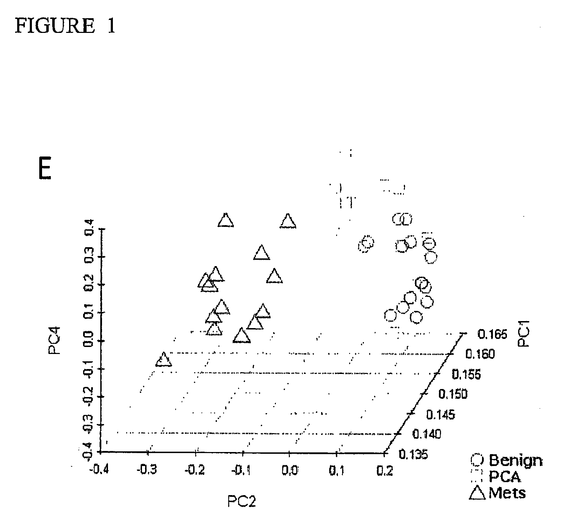

Figure 1 shows metabolomic profiling of prostate cancer progression a, Illustration of the steps involved in metabolomic profiling of prostate-derived tissues, b, Venn diagram representing the distribution of 626 metabolites measured across three classes of prostate-related tissues including benign prostate tissue (n=16), clinically localized prostate cancer (PCA, n=12), and metastatic prostate cancer (Mets, n=14). c, Dendrogram representing unsupervised hierarchical clustering of the prostate-related tissues described in b. N, benign prostate. T, PCA. M, Mets. d, Z-score plots for 626 metabolites monitored in prostate cancer samples normalized to the mean of the benign prostate samples, e, Principal components analysis of prostate tissue samples based on metabolomic alterations.
Figure 2 shows differential metabolomic alterations characteristic of prostate cancer progression. a, Z-score plot of metabolites altered in localized PCA relative to their mean in benign prostate tissues. b, Same as a but for the comparison between metastatic and PCA, with data relative to the mean of the PCA samples.

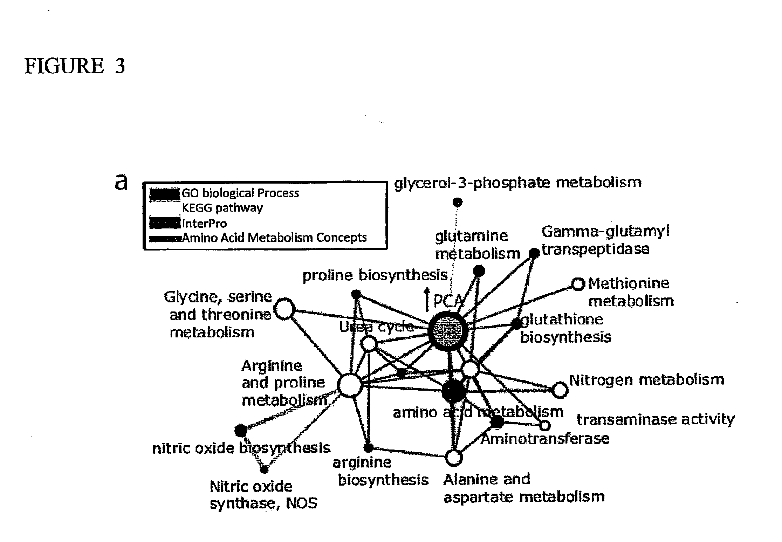

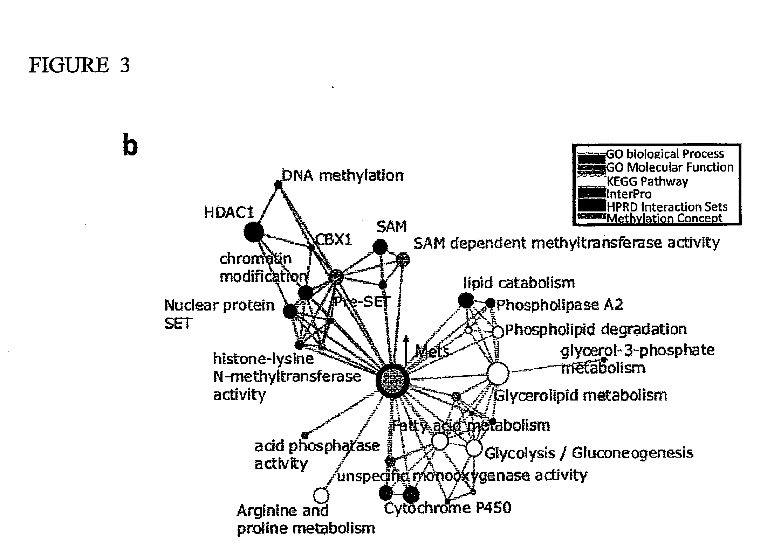

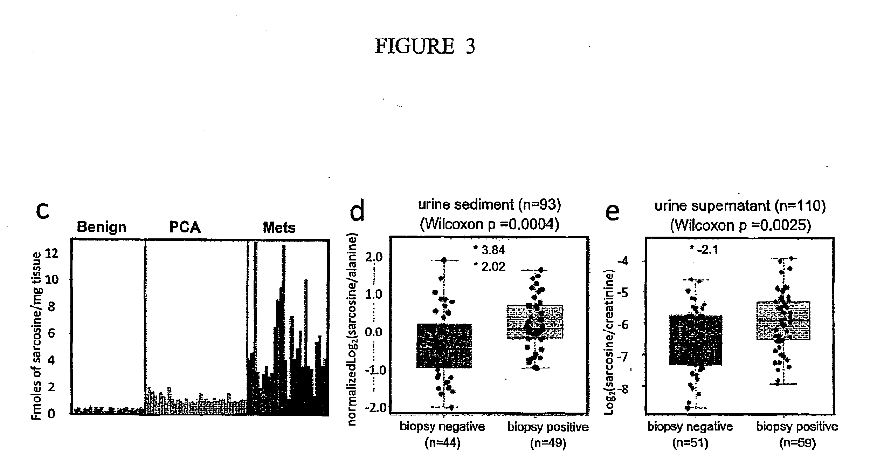

Figure 3 shows integrative analysis of metabolomic profiles of prostate cancer progression and validation of sarcosine as a marker for prostate cancer. a, Network view of the molecular concept analysis for the metabolomic profiles of the "over-expressed in PCA signature". b, Same as a, but for the metabolomic profiles of the "overexpressed in metastatic samples signature". c, Sarcosine levels in independent benign, PCA, and metastatic tissues based on isotope dilution GC/MS analysis. d, Boxplot of sarcosine levels based on isotope dilution GC/MS analysis showing normalized sarcosine to alanine levels in urine sediments from biopsy positive and negative individuals (mean ±SEM: 0.30 ± 0.13 vs -0.35 ± 0.13, Wilcoxon P=0.0004). e, same as d but for urine supernatants showing elevated sarcosine to creatinine levels in biopsy positive prostate cancer patients compared to biopsy negative controls (mean ±SEM: -5.92 ± 0.13 vs .-6.49 ± 0.17, Wilcoxon P = 0.0025)

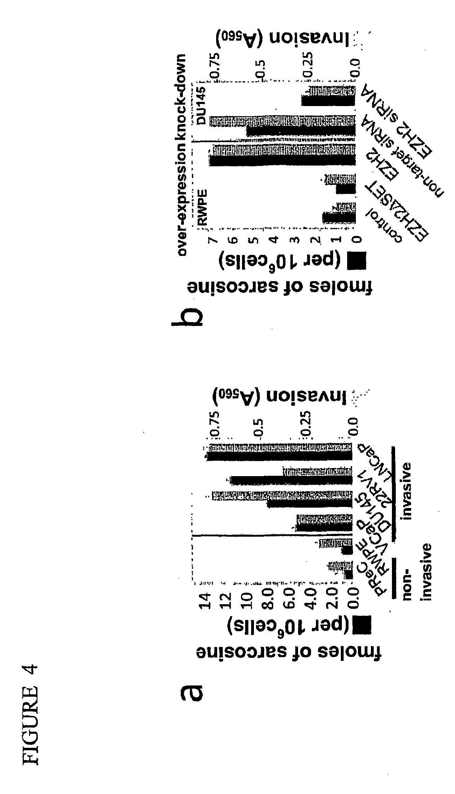

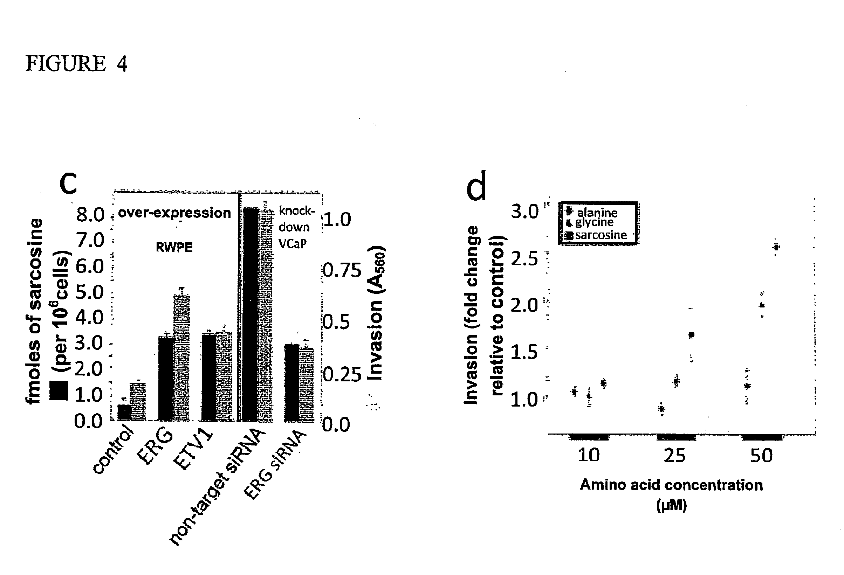

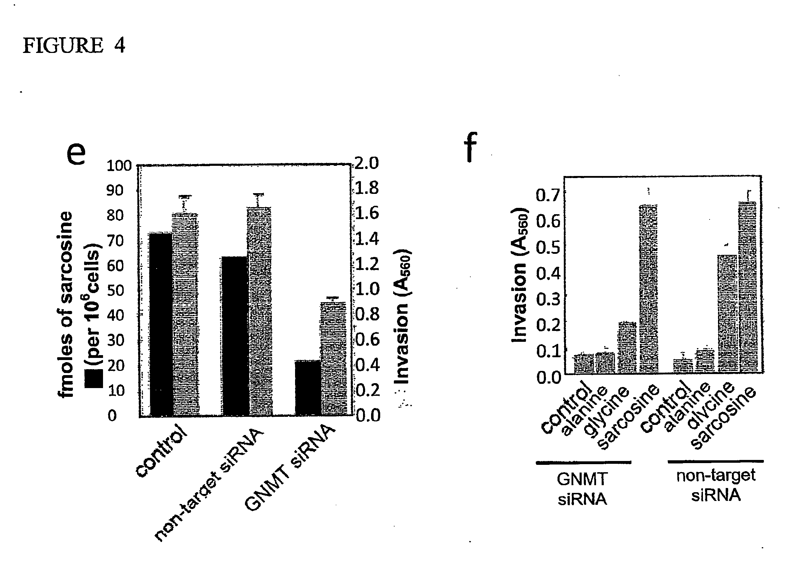

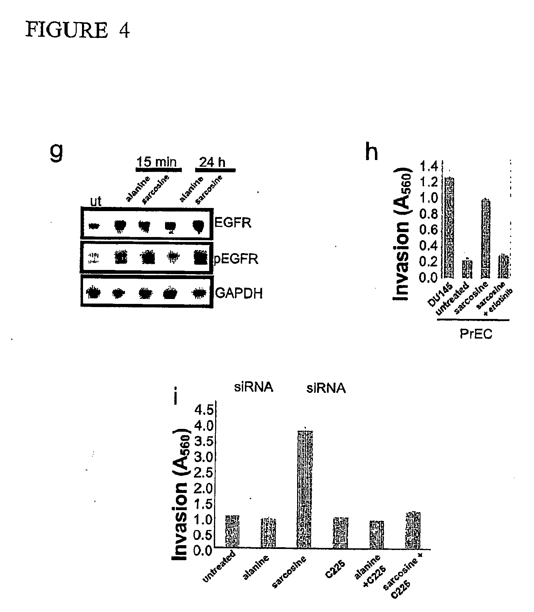

Figure 4 shows that sarcosine is associated with prostate cancer invasion and aggressiveness. a, Assessment of sarcosine and invasiveness of prostate cancer cell lines and benign epithelial cells. b, (Left panel) Overexpression of EZH2 by adenovirus infection in RWPE cells is associated with increased levels of sarcosine and significant increase in invasion (t-test P=0.0001) compared to vector control. (Right panel) Knockdown of EZH2 by siRNA in DU145 cells is associated with decreased levels of sarcosine and significant decrease in invasion relative to non-target siRNA control (t-test P = 0.0115). c, (Left panel) Overexpression of TMPRSS2-ERG or TMPRSS2-ETV 1 in RWPE is associated with increased levels of sarcosine (t-test: P = 0.0035 and P = 0.0016, respectively) and invasion (t-test: P = 0.0019 and P = 0.0057, respectively) relative to wild type control. (Right panel) Knockdown of TMPRSS2-ERG in VCaP cells is associated with decreased levels of sarcosine and significant decrease in invasion relative to non-target siRNA control (t-test: P = 0.0004). d, Assessment of invasion in prostate epithelial cells upon exogenous addition of alanine (circles), glycine (triangles) and sarcosine (squares) measured using a modified Boyden chamber assay. e, Knockdown of GNMT in DU145 cells using GNMT siRNA is associated with a decrease in sarcosine and invasion. (f) Attenuation of GNMT in RWPE cells blocks the ability of exogenous glycine but not sarcosine to induce invasion. g, Immunoblot analysis shows time-dependent phosphorylation of EGFR upon treatment of RWPE cells with 50 µM sarcosine relative to alanine. h, Decrease in sarcosine-induced invasion of PrEC prostate epithelial cells upon pretreatment with 10 µM erlotinib (F-test: P = 0.0003). DU145 cells serve as a positive control for cell invasion. i, Pre-treatment of RWPE cells with C225 decreases sarcosine-induced invasion relative to sarcosine treatment alone (F-test: P = 0.0056).
Figure 5 shows the relative distributions of standardized peak intensities for metabolites and distribution of tissue specimens from each sample class, across two experimental batches profiled. Samples from each of the three tissue classes were equally distributed across the two batches (X-axis). Y-axis shows the standardized peak intensity (m/z) for the 624 metabolites profiled in 42 tissue samples used in this study.
Figure 6 shows an outline of steps involved in analysis of the tissue metabolomic profiles.
Figure 7 shows reproducibility of the metabolomic profiling platform used in the discovery phase.
Figure 8 shows the relative expression of metastatic cancer-specific metabolites across metastatic tissues from different sites.
Figure 9 shows an outline of different steps involved in OCM analyses of the metabolomic profiles of localized prostate cancer and metastatic disease.
Figure 10 shows the reproducibility of sarcosine assessment using isotope-dilution GC-MS. (a) Sarcosine measurement in biological replicates of three prostate-derived cell lines was highly reproducible with a CV of <10 %. (b) Sarcosine measurement for 89 prostate derived tissue samples using two independent GC-MS instruments was highly correlated with Rho>0.9.
Figure 11 shows a comparison of sarcosine levels in tumor bearing tissues and non-tumor controls derived from patients with metastatic prostate cancer using isotope dilution GC/MS. (a) GC/MS trace showing the quantitation of native sarcosine in prostate cancer metastases to the lung. (b) As in (a) but in adjacent control lung tissue. (c) Bar plots showing high levels of sarcosine in metastatic tissues based on isotope dilution GC/MS analysis.

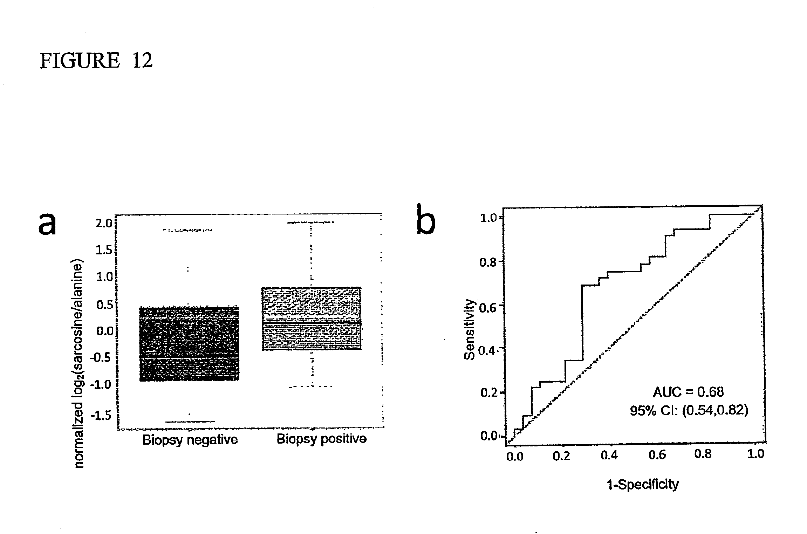

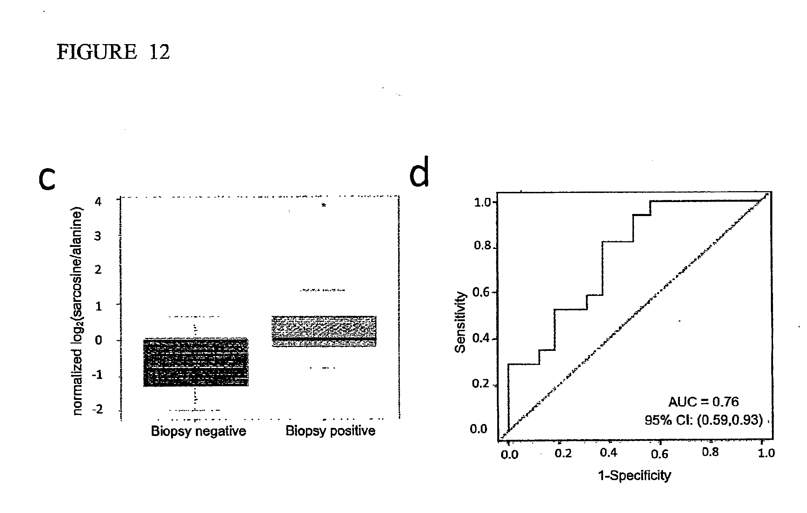

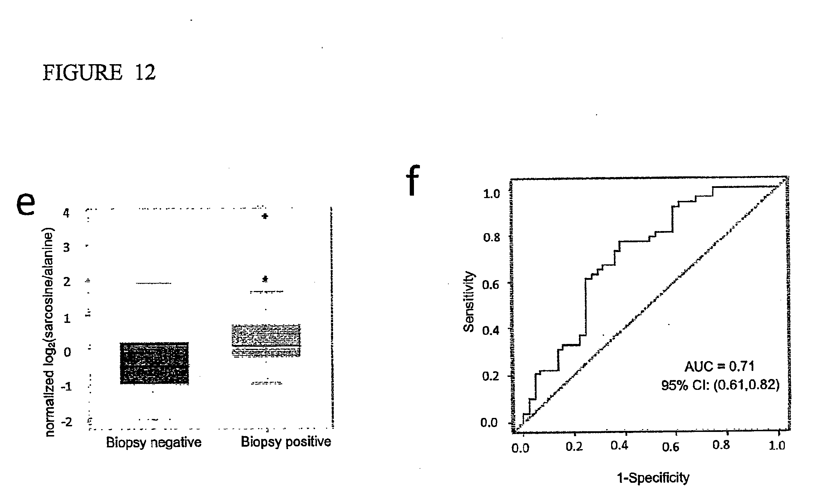

Figure 12 shows an assessment of sarcosine in urine sediments from men with positive and negative biopsies for cancer. (a) Boxplot showing significantly higher sarcosine levels, relative to alanine, in a batch of 60 urine sediments from 32 biopsy positive and 28 biopsy negative individuals (Wilcoxon rank-sum test: P = 0.0188). (b) The Receiver Operator Characteristic (ROC) Curve for the 60 samples in (a) has an AUC f 0.68 (95% CI: 0.54, 0.82). (c) Similar to (a), but in an independent batch of 33 samples (17 biopsy positive and 16 biopsy negative individuals). (d) ROC Curve for the 33 samples in (b) has an AUC of 0.76 (95% CI: 0.59, 0.93). (e) Boxplot for the total set of 93 samples shown in (a) and (c). (f) ROC Curve for the entire dataset (n=93) has an AUC of 0.71 (95% CI: 0.61, 0.82)
Figure 13 shows an assessment of sarcosine in biopsy positive and negative urine supernatants. (a) Box-plot showing significantly (Wilcoxon rank-sum test: P = 0.0025) higher levels of sarcosine relative to creatinine in a batch of 110 urine supernatants from 59 biopsy positive and 51 biopsy negative individuals. (b) Receiver Operator Curve of (a) has an AUC of 0.67 (95 % CI: 0.57, 0.77).

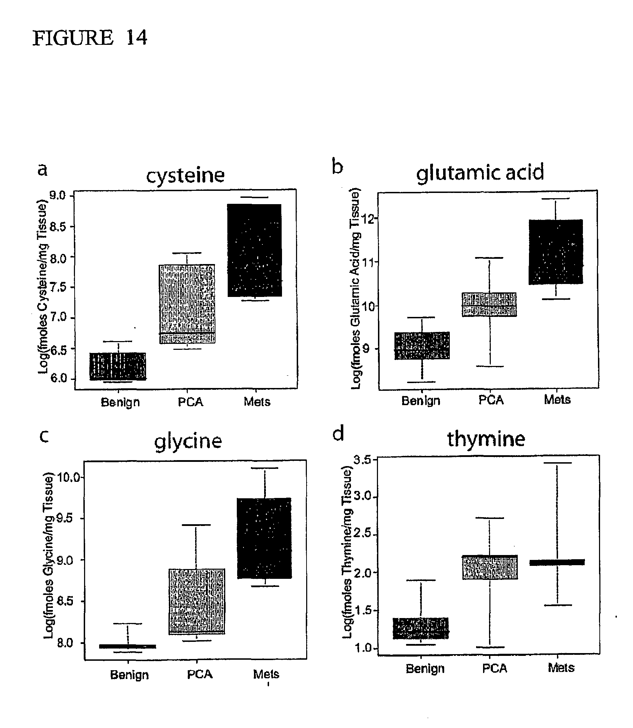

Figure 14 shows confirmation of additional prostate cancer-associated metabolites in prostate-derived tissue samples. (a) Box-plot showing elevated levels of cysteine during progression from benign to clinically localized to metastatic disease (n=5 each, mean ± SEM : 6.19 ± 0.13 vs 7.14 ± 0.34 vs 8.00 ± 0.37 for Benign vs PCA vs Mets) (b) same as a, but for glutamic acid (mean + SEM : 9.00 ± 0.26 vs 9.92 ± 0.41 vs 11.15 ± 0.44 for Benign vs PCA vs Mets) (c) same as a, but for glycine (mean ± SEM : 8.00 ± 0.06 vs 8.51 ± 0.28 vs 9.28 ± 0.28 for Benign vs PCA vs Mets). (d) same as a, but for thymine (mean + SEM : 1.33 ± 0.15 vs 2.01 ± 0.28 vs 2.27 ± 0.31 for Benign vs PCA vs Mets).
Figure 15 shows an immunoblot confirmation of EZH2 over-expression and knock-down in prostate-derived cell lines.
Figure 16 shows real-time PCR-based quantitation of knock-down of the ERG gene fusion product in VCaP cells.
Figure 17 shows an assessment of internalized sarcosine in prostate and breast epithelial cell lines.
Figure 18 shows cell cycle analysis and assessment of proliferation in amino acid-treated prostate epithelial cells. (a) Cell cycle profile of untreated prostate cell line RWPE or treated for 24 h with 50 µM of either (b) alanine (c) glycine (d) sarcosine. (e) Assessment of cell numbers using coulter counter for (a-d).
Figure 19 shows real-time PCR-based quantitation of GNMT knockdown in prostate cell lines. (a) In DU145 cells, siRNA mediated knockdown resulted in approximately 25 % decrease in GNMT mRNA levels (b) in RWPE cells, siRNA mediated knockdown resulted in approximately 42 % decrease in GNMT mRNA levels.
Figure 20 shows glycine-induced invasion, but not sarcosine-induced invasion is blocked by knock-down of GNMT.
Figure 21 shows Oncomine concept maps of genes over-expressed in sarcosine treated prostate epithelial cells compared to alanine-treated.
Figure 22 shows downstream read-outs of the EGFR pathway are activated by sarcosine.
Figure 23 shows that Erlotinib inhibits sarcosine mediated invasion in PrEC cells. (a) Immunoblot analysis showing inhibition of EGFR phosphorylation by 10 µM Erlotinib. (b) Pre-treatment of PrEC cells with 10 µM Erlotinib results in a significant decrease in sarcosine-induced invasion. (c) colorimetric quantitation of (b).

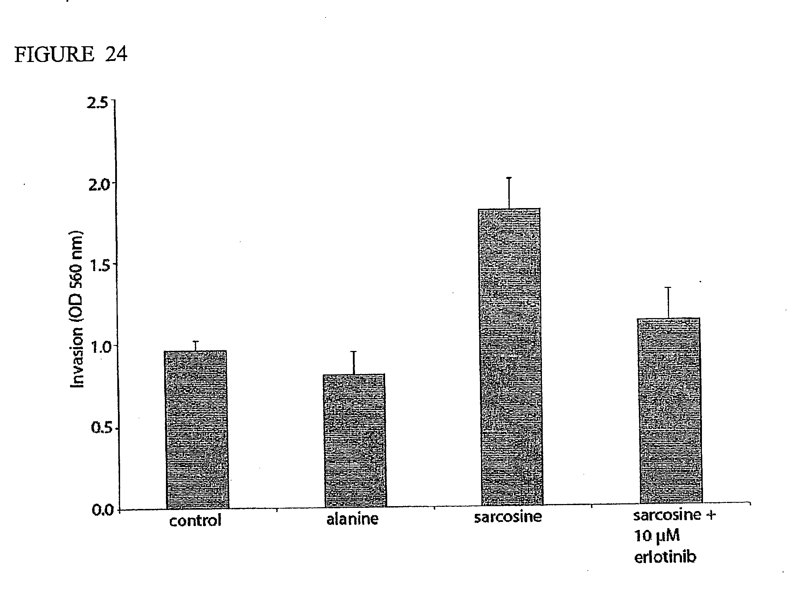

Figure 24 shows that Erlotinib inhibits sarcosine mediated invasion in RWPE cells. (a) Pre-treatment of RWPE cells with 10 µM Erlotinib results in a 2-fold decrease in sarcosine-induced invasion.
Figure 25 shows that C225 inhibits sarcosine mediated invasion in RWPE cells. (a) Pre-treatment of RWPE cells with 50 mg/ml of C225 results in a significant decrease in sarcosine-induced invasion. (b) Immunoblot analysis showing inhibition of EGFR phosphorylation by 50 mg/ml of C225.
Figure 26 shows that knock-down of EGFR attenuates sarcosine mediated cell invasion. (a) Photomicrograph of cells. (b) Colorometic assessment of invasion. (c) Confirmation of EGFR knock-down by QRT-PCR.

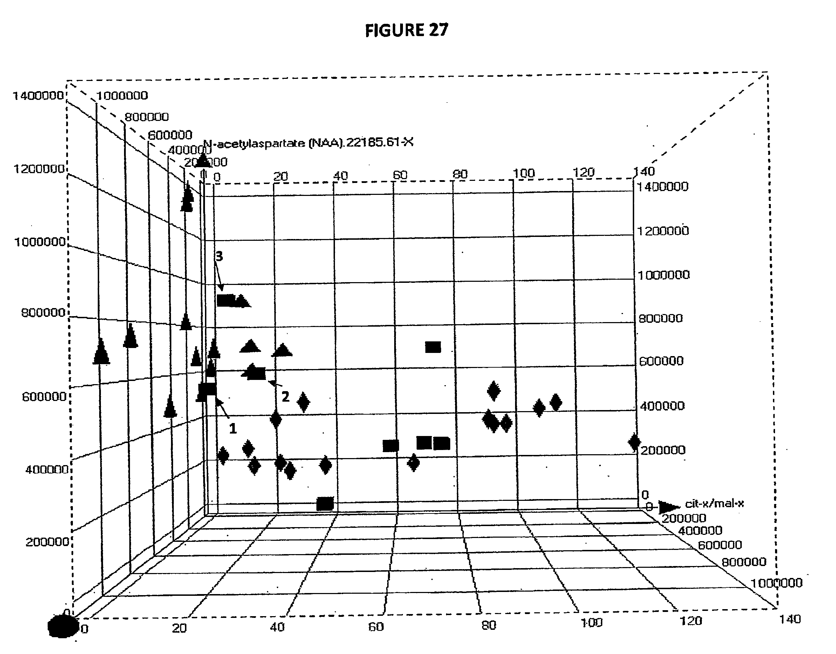

Figure 27 shows a three dimensional plot of a panel of biomarkers useful to determine cancer tumor aggressivity in a range of tumors from non-aggressive to very aggressive. Benign (diamonds), metastatic (isosceles triangles), GS3 (squares), GS4 (equilateral triangles). X-axis, citrate/malate; Y-axis, NAA; Z-axis, sarcosine. Several metastatic samples are off-scale and are not visible on the graph as presented.
Figure 28 shows a Z score plot showing elevated levels of sarcosine and associated metabolites in the methionine pathway during prostate cancer progression.
Figure 29 shows validation of sarcosine in prostate cancer and metastatic cancer using isotope dilution GCMS.
Figure 30 shows knock down of SARDH in RWPE cells. (a) GC MS assessment of sarcosine. (b) colorimetric assessment of invasion. (c) photomicrographs of b.

### DEFINITIONS

To facilitate an understanding of the present invention, a number of terms and phrases are defined below:
"Prostate cancer" refers to a disease in which cancer develops in the prostate, a gland in the male reproductive system. "Low grade" or "lower grade" prostate cancer refers to non-metastatic prostate cancer, including malignant tumors with low potential for metastasis (i.e. prostate cancer that is considered to be less aggressive). "High grade" or "higher grade" prostate cancer refers to prostate cancer that has metastasized in a subject, including malignant tumors with high potential for metastasis (prostate cancer that is considered to be aggressive).

As used herein, the term "cancer specific metabolite" refers to a metabolite that is differentially present in cancerous cells compared to non-cancerous cells. For example, in some embodiments, cancer specific metabolites are present in cancerous cells but not non-cancerous cells. In other embodiments, cancer specific metabolites are absent in cancerous cells but present in non-cancerous cells. In still further embodiments, cancer specific metabolites are present at different levels (e.g., higher or lower) in cancerous cells as compared to non-cancerous cells. For example, a cancer specific metabolite may be differentially present at any level, but is generally present at a level that is increased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more; or is generally present at a level that is decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by 100% (i.e., absent). A cancer specific metabolite is preferably differentially present at a level that is statistically significant (i.e., a p-value less than 0.05 and/or a q-value of less than 0.10 as determined using either Welch's T-test or Wilcoxon's rank-sum Test). Exemplary cancer specific metabolites are described in the detailed description and experimental sections below.

The term "sample" in the present specification and claims is used in its broadest sense. On the one hand it is meant to include a specimen or culture. On the other hand, it is meant to include both biological and environmental samples. A sample may include a specimen of synthetic origin.

Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagamorphs, rodents, etc. A biological sample may contain any biological material suitable for detecting the desired biomarkers, and may comprise cellular and/or non-cellular material from a subject. The sample can be isolated from any suitable biological tissue or fluid such as, for example, prostate tissue, blood, blood plasma, urine, or cerebral spinal fluid (CSF).

Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present disclosure.

A "reference level" of a metabolite means a level of the metabolite that is indicative of a particular disease state, phenotype, or lack thereof, as well as combinations of disease states, phenotypes, or lack thereof. A "positive" reference level of a metabolite means a level that is indicative of a particular disease state or phenotype. A "negative" reference level of a metabolite means a level that is indicative of a lack of a particular disease state or phenotype. For example, a "prostate cancer-positive reference level" of a metabolite means a level of a metabolite that is indicative of a positive diagnosis of prostate cancer in a subject, and a "prostate cancer-negative reference level" of a metabolite means a level of a metabolite that is indicative of a negative diagnosis of prostate cancer in a subject. A "reference level" of a metabolite may be an absolute or relative amount or concentration of the metabolite, a presence or absence of the metabolite, a range of amount or concentration of the metabolite, a minimum and/or maximum amount or concentration of the metabolite, a mean amount or concentration of the metabolite, and/or a median amount or concentration of the metabolite; and, in addition, "reference levels" of combinations of metabolites may also be ratios of absolute or relative amounts or concentrations of two or more metabolites with respect to each other. Appropriate positive and negative reference levels of metabolites for a particular disease state, phenotype, or lack thereof may be determined by measuring levels of desired metabolites in one or more appropriate subjects, and such reference levels may be tailored to specific populations of subjects (e.g., a reference level may be age-matched so that comparisons may be made between metabolite levels in samples from subjects of a certain age and reference levels for a particular disease state, phenotype, or lack thereof in a certain age group). Such reference levels may also be tailored to specific techniques that are used to measure levels of metabolites in biological samples (e.g., LC-MS, GC-MS, etc.), where the levels of metabolites may differ based on the specific technique that is used.

As used herein, the term "cell" refers to any eukaryotic or prokaryotic cell (e.g., bacterial cells such as E. coli, yeast cells, mammalian cells, avian cells, amphibian cells, plant cells, fish cells, and insect cells), whether located in vitro or in vivo.

As used herein, the term "processor" refers to a device that performs a set of steps according to a program (e.g., a digital computer). Processors, for example, include Central Processing Units ("CPUs"), electronic devices, or systems for receiving, transmitting, storing and/or manipulating data under programmed control.

As used herein, the term "memory device," or "computer memory" refers to any data storage device that is readable by a computer, including, but not limited to, random access memory, hard disks, magnetic (floppy) disks, compact discs, DVDs, magnetic tape, flash memory, and the like.

The term "proteomics", as described in Liebler, D. Introduction to Proteomics: Tools for the New Biology, Humana Press, 2003, refers to the analysis of large sets of proteins. Proteomics deals with the identification and quantification of proteins, their localization, modifications, interactions, activities, and their biochemical and cellular function. The explosive growth of the proteomics field has been driven by novel, high-throughput laboratory methods and measurement technologies, such as gel electrophoresis and mass spectrometry, as well as by innovative computational tools and methods to process, analyze, and interpret huge amounts of data.

"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ration of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.

The term "lysis" refers to cell rupture caused by physical or chemical means. This is done to obtain a protein extract from a sample of serum or tissue.

The term "separation" refers to separating a complex mixture into its component proteins or metabolites. Common laboratory separation techniques include gel electrophoresis and chromatography.

The term "gel electrophoresis" refers to a technique for separating and purifying molecules according to the relative distance they travel through a gel under the influence of an electric current. Techniques for automated gel spots excision may provide data in large dataset format that may be used as input for the methods and systems described herein.

The term "capillary electrophoresis" refers to an automated analytical technique that separates molecules in a solution by applying voltage across buffer-filled capillaries. Capillary electrophoresis is generally used for separating ions, which move at different speeds when the voltage is applied, depending upon the size and charge of the ions. The solutes (ions) are seen as peaks as they pass through a detector and the area of each peak is proportional to the concentration of ions in the solute, which allows quantitative determinations of the ions.

The term "chromatography" refers to a physical method of separation in which the components to be separated are distributed between two phases, one of which is stationary (stationary phase) while the other (the mobile phase) moves in a definite direction. Chromatographic output data may be used for manipulation by the present invention.

The term "chromatographic time", when used in the context of mass spectrometry data, refers to the elapsed time in a chromatography process since the injection of the sample into the separation device. A "mass analyzer" is a device in a mass spectrometer that separates a mixture of ions by their mass-to-charge ratios.

A "source" is a device in a mass spectrometer that ionizes a sample to be analyzed.

A "detector" is a device in a mass spectrometer that detects ions.

An "ion" is a charged object formed by adding electrons to or removing electrons from an atom.

A "mass spectrum" is a plot of data produced by a mass spectrometer, typically containing m/z values on x-axis and intensity values on y-axis.

A "peak" is a point on a mass spectrum with a relatively high y-value.

The term "m/z" refers to the dimensionless quantity formed by dividing the mass number of an ion by its charge number. It has long been called the "mass-to-charge" ratio.

The term "metabolism" refers to the chemical changes that occur within the tissues of an organism, including "anabolism" and "catabolism". Anabolism refers to biosynthesis or the buildup of molecules and catabolism refers to the breakdown of molecules.

A "metabolite" is an intermediate or product resulting from metabolism. Metabolites are often referred to as "small molecules".

The term "metabolomics" refers to the study of cellular metabolites.

A "biopolymer" is a polymer of one or more types of repeating units. Biopolymers are typically found in biological systems and particularly include polysaccharides (such as carbohydrates), and peptides (which term is used to include polypeptides and proteins) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another.

As used herein, the term "post-surgical tissue" refers to tissue that has been removed from a subject during a surgical procedure. Examples include, but are not limited to, biopsy samples, excised organs, and excised portions of organs.

As used herein, the terms "detect", "detecting", or "detection" may describe either the general act of discovering or discerning or the specific observation of a detestably labeled composition.

As used herein, the term "clinical failure" refers to a negative outcome following prostatectomy. Examples of outcomes associated with clinical failure include, but are not limited to, an increase in PSA levels (e.g., an increase of at least 0.2 ng ml⁻¹) or recurrence of disease (e.g., metastatic prostate cancer) after prostatectomy.

As used herein, the term "siRNAs" refers to small interfering RNAs. In some examples, siRNAs comprise a duplex, or double-stranded region, of about 18-25 nucleotides long; often siRNAs contain from about two to four unpaired nucleotides at the 3' end of each strand. At least one strand of the duplex or double-stranded region of a siRNA is substantially homologous to, or substantially complementary to, a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; non-limiting examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

### DETAILED DESCRIPTION OF THE INVENTION

The present invention is defined in the claims and relates to cancer markers. In particular embodiments, the present invention provides metabolites that are differentially present in prostate cancer. Experiments conducted during the course of development of embodiments of the present invention identified a series of metabolites as being differentially present in prostate cancer versus normal prostate. Experiments conducted during the course of development of embodiments of the present invention indentified, for example, sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, n-acetyl tyrosine and thymine. Tables 3, 4, 10 and 11 provide additional metabolites present in localized and metastatic cancer. The disclosed markers find use as diagnostic and therapeutic targets. In some examples, the present disclosure relates to methods of identifying invasive prostate cancers based on the presence of elevated levels of sarcosine (e.g. in tumor tissue or other bodily fluids).

### I. Diagnostic Applications

In some examples the present disclosure relates to methods and compositions for diagnosing cancer, including but not limited to, characterizing risk of cancer, stage of cancer, risk of or presence of metastasis, invasiveness of cancer, etc. based on the presence of cancer specific metabolites or their derivates, precursors, metabolites, etc. Exemplary diagnostic methods are described below.

Thus, for example, a method of diagnosing (or aiding in diagnosing) whether a subject has prostate cancer comprises (1) detecting the presence or absence or a differential level of one or more cancer specific metabolites selected from sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, n-acetyl tyrosine, and thymine in a sample from a subject; and b) diagnosing cancer based on the presence, absence or differential level of the cancer specific metabolite. When such a method is used to aid in the diagnosis of prostate cancer, the results of the method may be used along with other methods (or the results thereof) useful in the clinical determination of whether a subject has prostate cancer.

In another example, methods of characterizing prostate cancer comprise detecting the presence or absence or amount of an elevated level of a metabolite, for example sarcosine, in a sample from a subject diagnosed with cancer; and b) characterizing the prostate cancer based on the presence of said elevated levels of the metabolite (e.g. sarcosine).

### A. Sample

Any patient sample suspected of containing cancer specific metabolites is tested according to the methods described herein. By way of non-limiting examples, the sample may be tissue (*e.g*., a prostate biopsy sample or post-surgical tissue), blood, urine, or a fraction thereof (*e.g*., plasma, serum, urine supernatant, urine cell pellet or prostate cells). In some embodiments, the sample is a tissue sample obtained from a biopsy or following surgery (e.g., prostate biopsy).

In some embodiments, the patient sample undergoes preliminary processing designed to isolate or enrich the sample for cancer specific metabolites or cells that contain cancer specific metabolites. A variety of techniques known to those of ordinary skill in the art may be used for this purpose, including but not limited: centrifugation; immunocapture; and cell lysis.

### B. Detection of Metabolites

Metabolites may be detected using any suitable method including, but not limited to, liquid and gas phase chromatography, alone or coupled to mass spectrometry (See e.g., experimental section below), NMR (See e.g., US patent publication 20070055456), immunoassays, chemical assays, spectroscopy and the like. In some embodiments, commercial systems for chromatography and NMR analysis are utilized.

In other embodiments, metabolites (i.e. biomarkers and derivatives thereof) are detected using optical imaging techniques such as magnetic resonance spectroscopy (MRS), magnetic resonance imaging (MRI), CAT scans, ultra sound, MS-based tissue imaging or X-ray detection methods(e.g., energy dispersive x-ray fluorescence detection).

Any suitable method may be used to analyze the biological sample in order to determine the presence, absence or level(s) of the one or more metabolites in the sample. Suitable methods include chromatography (*e.g*., HPLC, gas chromatography, liquid chromatography), mass spectrometry (e.g., MS, MS-MS), enzyme-linked immunosorbent assay (ELISA), antibody linkage, other immunochemical techniques, biochemical or enzymatic reactions or assays, and combinations thereof. Further, the level(s) of the one or more metabolites may be measured indirectly, for example, by using an assay that measures the level of a compound (or compounds) that correlates with the level of the biomarker(s) that are desired to be measured.

The levels of one or more of the recited metabolites may be determined in the methods relating to the present disclosure. For example, the level(s) of one metabolites, two or more metabolites, three or more metabolites, four or more metabolites, five or more metabolites, six or more metabolites, seven or more metabolites, eight or more metabolites, nine or more metabolites, ten or more metabolites, etc., including a combination of some or all of the metabolites including, but not limited to, sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, n-acetyl tyrosine and thymine, may be determined and used in such methods. Determining levels of combinations of the metabolites may allow greater sensitivity and specificity in the methods, such as diagnosing prostate cancer and aiding in the diagnosis of prostate cancer, and may allow better differentiation or characterization of prostate cancer from other prostate disorders (e.g. benign prostatic hypertrophy (BPH), prostatitis, etc.) or other cancers that may have similar or overlapping metabolites to prostate cancer (as compared to a subject not having prostate cancer). For example, ratios of the levels of certain metabolites in biological samples may allow greater sensitivity and specificity in diagnosing prostate cancer and aiding in the diagnosis of prostate cancer and allow better differentiation or characterization of prostate cancer from other cancers or other disorders of the prostate that may have similar or overlapping metabolites to prostate cancer (as compared to a subject not having prostate cancer).

### C. Data Analysis

In some embodiments, a computer-based analysis program is used to translate the raw data generated by the detection assay (*e.g*., the presence, absence, or amount of a cancer specific metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.

The present disclosure relates to any method capable of receiving, processing, and transmitting the information to and from laboratories conducting the assays, information provides, medical personal, and subjects. For example, in some examples of the present disclosure, a sample (*e.g*., a biopsy or a blood, urine or serum sample) is obtained from a subject and submitted to a profiling service (*e.g*., clinical lab at a medical facility, etc.), located in any part of the world (*e.g*., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g*., a urine sample) and directly send it to a profiling center. Where the sample comprises previously determined biological information, the information may be directly sent to the profiling service by the subject (*e.g*., an information card containing the information may be scanned by a computer and the data transmitted to a computer of the profiling center using an electronic communication systems). Once received by the profiling service, the sample is processed and a profile is produced (*i.e*., metabolic profile), specific for the diagnostic or prognostic information desired for the subject.

The profile data is then prepared in a format suitable for interpretation by a treating clinician. For example, rather than providing raw data, the prepared format may represent a diagnosis or risk assessment (*e.g*., likelihood of cancer being present) for the subject, along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.

In some examples, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some examples, the subject is able to directly access the data using the electronic communication system. The subject may chose further intervention or counseling based on the results. In some examples, the data is used for research use. For example, the data may be used to further optimize the inclusion or elimination of markers as useful indicators of a particular condition or stage of disease.

When the amount(s) or level(s) of the one or more metabolites in the sample are determined, the amount(s) or level(s) may be compared to prostate cancer metabolite- reference levels, such as prostate-cancer-positive and/or prostate cancer-negative reference levels to aid in diagnosing or to diagnose whether the subject has prostate cancer. Levels of the one or more metabolites in a sample corresponding to the prostate cancer-positive reference levels (e.g., levels that are the same as the reference levels, substantially the same as the reference levels, above and/or below the minimum and/or maximum of the reference levels, and/or within the range of the reference levels) are indicative of a diagnosis of prostate cancer in the subject. Levels of the one or more metabolites in a sample corresponding to the prostate cancer-negative reference levels (e.g., levels that are the same as the reference levels, substantially the same as the reference levels, above and/or below the minimum and/or maximum of the reference levels, and/or within the range of the reference levels) are indicative of a diagnosis of no prostate cancer in the subject. In addition, levels of the one or more metabolites that are differentially present (especially at a level that is statistically significant) in the sample as compared to prostate cancer-negative reference levels are indicative of a diagnosis of prostate cancer in the subject. Levels of the one or more metabolites that are differentially present (especially at a level that is statistically significant) in the sample as compared to prostate cancer-positive reference levels are indicative of a diagnosis of no prostate cancer in the subject.

The level(s) of the one or more metabolites may be compared to prostate cancer-positive and/or prostate cancer-negative reference levels using various techniques, including a simple comparison (e.g., a manual comparison) of the level(s) of the one or more metabolites in the biological sample to prostate cancer-positive and/or prostate cancer-negative reference levels. The level(s) of the one or more metabolites in the biological sample may also be compared to prostate cancer-positive and/or prostate cancer-negative reference levels using one or more statistical analyses (e.g., t-test, Welch's T-test, Wilcoxon's rank sum test, random forest).

### D. Compositions & Kits

Compositions for use (e.g., sufficient for, necessary for, or useful for) in the diagnostic methods relating to some embodiments of the present invention include reagents for detecting the presence or absence of cancer specific metabolites. Any of these compositions, alone or in combination with other compositions relating to the present disclosure, may be provided in the form of a kit. Kits may further comprise appropriate controls and/or detection reagents.

### E. Panels

Embodiments of the present disclosure relate to multiplex or panel assays that simultaneously detect one or more of the markers of the present disclosure (e.g., sarcosine, cysteine, glutamate, asparagine, glycine, leucine, proline, threonine, histidine, n-acetyl-aspartic acid, inosine, inositol, adenosine, taurine, creatine, uric acid, glutathione, uracil, kynurenine, glycerol-s-phosphate, glycocholic acid, suberic acid, thymine, glutamic acid, xanthosine, 4-acetamidobutyric acid, n-acetyltyrosine and thymine), alone or in combination with additional cancer markers known in the art. For example, in some embodiments, panel or combination assays are provided that detected 2 or more, 3 or more, 4 or more, 5 or more, 6 or more, 7 or more, 8 or more, 9 or more, 10 or more, 15 or more, or 20 or more markers in a single assay. In some embodiments, assays are automated or high throughput.

In some embodiments, additional cancer markers are included in multiplex or panel assays. Markers are selected for their predictive value alone or in combination with the metabolic markers described herein. Exemplary prostate cancer markers include, but are not limited to: AMACR/P504S (U.S. Pat. No. 6,262,245); PCA3 (U.S. Pat. No. 7.008.765); PCGEM1 (U.S. Pat. No. 6,828,429): prostein/P501S, P503S, P504S, P509S, P510S, prostase/P703P, P710P (U.S. Publication No. 20030185830); and, those disclosed in U.S. Pat. Nos. 5,854,206 and 6,034,218, and U.S. Publication No. 20030175736. Markers for other cancers, diseases, infections, and metabolic conditions are also contemplated for inclusion in a multiplex or panel format.

### II. Therapeutic Methods

In some examples, the present disclosure relates to therapeutic methods (e.g., that target the cancer specific metabolites described herein). In some examples, the therapeutic methods target enzymes or pathway components of the cancer specific metabolites described herein.

For example, in some examples, the present disclosure relates to compounds that target the cancer specific metabolites of the present disclosure. The compounds may decrease the level of cancer specific metabolite by, for example, interfering with synthesis of the cancer specific metabolite (e.g., by blocking transcription or translation of an enzyme involved in the synthesis of a metabolite, by inactivating an enzyme involved in the synthesis of a metabolite (e.g., by post translational modification or binding to an irreversible inhibitor), or by otherwise inhibiting the activity of an enzyme involved in the synthesis of a metabolite) or a precursor or metabolite thereof, by binding to and inhibiting the function of the cancer specific metabolite, by binding to the target of the cancer specific metabolite (e.g., competitive or non competitive inhibitor), or by increasing the rate of break down or clearance of the metabolite. The compounds may increase the level of cancer specific metabolite by, for example, inhibiting the break down or clearance of the cancer specific metabolite (e.g., by inhibiting an enzyme involved in the breakdown of the metabolite), by increasing the level of a precursor of the cancer specific metabolite, or by increasing the affinity of the metabolite for its target. Exemplary therapeutic targets include, but are not limited to, glycine-N-methyl transferase (GNMT) and sarcosine.

### A. Metabolic Pathways

The metabolic pathways of exemplary cancer specific metabolites are described below. Additional metabolites are contemplated for use in the compositions and methods relating to the present invention and are described, for example, in the Experimental section below.

### i. Sarcosine Metabolism

For example, sarcosine is involved in choline metabolism in the liver. The oxidative degradation of choline to glycine in the mammalian liver takes place in the mitochondria, where it enters by a specific transporter. The two last steps in this metabolic pathway are catalyzed by dimethylglycine dehydrogenase (Me2GlyDH), which converts dimethylglycine into sarcosine, and sarcosine dehydrogenase (SarDH), which converts sarcosine (N-methylglycine) into glycine. Both enzymes are located in the mitochondrial matrix. Accordingly, in some examples, therapeutic compositions target Me2GlyDH and/or SarDH. Exemplary compounds are identified, for example, by using the drug screening methods described herein.

### ii. Glycholic Acid Metabolism

The end products of cholesterol utilization are the bile acids, synthesized in the liver. Synthesis of bile acids is the predominant mechanisms for the excretion of excess cholesterol. However, the excretion of cholesterol in the form of bile acids is insufficient to compensate for an excess dietary intake of cholesterol. The most abundant bile acids in human bile are chenodeoxycholic acid (45%) and cholic acid (31 %). The carboxyl group of bile acids is conjugated via an amide bond to either glycine or taurine before their secretion into the bile canaliculi. These conjugation reactions yield glycocholic acid and taurocholic acid, respectively. The bile canaliculi join with the bile ductules, which then form the bile ducts. Bile acids are carried from the liver through these ducts to the gallbladder, where they are stored for future use. The ultimate fate of bile acids is secretion into the intestine, where they aid in the emulsification of dietary lipids. In the gut the glycine and taurine residues are removed and the bile acids are either excreted (only a small percentage) or reabsorbed by the gut and returned to the liver. This process is termed the enterohepatic circulation.

### iii. Suberic Acid Metabolism

Suberic acid, also octanedioic acid, is a dicarboxylic acid, with formula C₆H₁₂(COOH)₂. The peroxisomal metabolism of dicarboxylic acids results in the production of the mediumchain dicarboxylic acids adipic acid, suberic acid, and sebacic acid, which are excreted in the urine.

### iv. Xanthosine Metabolism

Xanthosine is involved in purine nucleoside metabolism. Specifically, xanthosine is an intermediate in the conversion of inosine to guanosine. Xanthylic acid can be used in quantitative measurements of the Inosine monophosphate dehydrogenase enzyme activities in purine metabolism, as recommended to ensure optimal thiopurine therapy for children with acute lymphoblastic leukaemia (ALL).

### B. Small Molecule Therapies

In some examples, small molecule therapeutics are utilized. In certain examples, small molecule therapeutics targeting cancer specific metabolites. In some examples, small molecule therapeutics are identified, for example, using the drug screening methods relating to the present disclosure.

### C. Nucleic acid Based Therapies

In other examples, nucleic acid based therapeutics are utilized. Exemplary nucleic acid based therapeutics include, but are not limited to antisense RNA, siRNA, and miRNA. In some examples, nucleic acid based therapeutics target the expression of enzymes in the metabolic pathways of cancer specific metabolites (e.g., those described above).

In some examples, nucleic acid based therapeutics are antisense. siRNAs are used as gene-specific therapeutic agents (Tuschl and Borkhardt, Molecular Intervent. 2002; 2(3):158-67). The transfection of siRNAs into animal cells results in the potent, long-lasting post-transcriptional silencing of specific genes (Caplen et al, Proc Natl Acad Sci U.S.A. 2001; 98: 9742-7; Elbashir et al., Nature. 2001; 411:494-8; Elbashir et al., Genes Dev. 2001;15; 188-200; and Elbashir et al., EMBO J. 2001; 20: 6877-88. Methods and compositions for performing RNAi with siRNAs are described, for example, in U.S. Pat. 6,506,559.

In other examples, expression of genes involved in metabolic pathways of cancer specific metabolites is modulated using antisense compounds that specifically hybridize with one or more nucleic acids encoding the enzymes (See e.g., Georg Sczakiel, Frontiers in Bioscience 5, d194-201 January 1, 2000; Yuen et al., Frontiers in Bioscience d588-593, June 1, 2000; Antisense Therapeutics, Second Edition, Phillips, M. Ian, Humana Press, 2004.

### D. Gene Therapy

The present dislosure relates to the use of any genetic manipulation for use in modulating the expression of enzymes involved in metabolic pathways of cancer specific metabolites described herein. Examples of genetic manipulation include, but are not limited to, gene knockout (*e.g*., removing the gene from the chromosome using, for example, recombination), expression of antisense constructs with or without inducible promoters, and the like. Delivery of nucleic acid construct to cells *in vitro* or *in* vivo may be conducted using any suitable method. A suitable method is one that introduces the nucleic acid construct into the cell such that the desired event occurs (e.g., expression of an antisense construct). Genetic therapy may also be used to deliver siRNA or other interfering molecules that are expressed in vivo (e.g., upon stimulation by an inducible promoter).

Introduction of molecules carrying genetic information into cells is achieved by any of various methods including, but not limited to, directed injection of naked DNA constructs, bombardment with gold particles loaded with said constructs, and macromolecule mediated gene transfer using, for example, liposomes, biopolymers, and the like. Preferred methods use gene delivery vehicles derived from viruses, including, but not limited to, adenoviruses, retroviruses, vaccinia viruses, and adeno-associated viruses. Because of the higher efficiency as compared to retroviruses, vectors derived from adenoviruses are the preferred gene delivery vehicles for transferring nucleic acid molecules into host cells *in vivo.* Adenoviral vectors have been shown to provide very efficient *in vivo* gene transfer into a variety of solid tumors in animal models and into human solid tumor xenografts in immune-deficient mice. Examples of adenoviral vectors and methods for gene transfer are described in PCT publications WO 00/12738 and WO 00/09675 and U.S. Pat. Appl. Nos. 6,033,908, 6,019,978, 6,001,557, 5,994,132, 5,994,128, 5,994,106, 5,981,225, 5,885,808, 5,872,154, 5,830,730, and 5,824,544.

Vectors may be administered to subject in a variety of ways. For example, in some examples relating to the present disclosure, vectors are administered into tumors or tissue associated with tumors using direct injection. In other examples, administration is via the blood or lymphatic circulation (*See e.g.,* PCT publication 99/02685 .Exemplary dose levels of adenoviral vector are preferably 10⁸ to 10¹¹ vector particles added to the perfusate.

### E. Antibody Therapy

In some examples, the present disclosure relates to antibodies that target cancer specific metabolites or enzymes involved in their metabolic pathways. Any suitable antibody (*e.g*., monoclonal, polyclonal, or synthetic) may be utilized in the therapeutic methods disclosed herein. In preferred examples, the antibodies used for cancer therapy are humanized antibodies. Methods for humanizing antibodies are well known in the art (*See e.g.,* U.S .Pat. Nos. 6,180,370, 5,585,089, 6,054,297, and 5,565,332.

In some examples, antibody based therapeutics are formulated as pharmaceutical compositions as described below. In preferred examples, administration of an antibody composition relating to the present disclosure results in a measurable decrease in cancer (*e,g*. , decrease or elimination of tumor).

### F. Pharmaceutical Compositions

The presents disclosure further relates to pharmaceutical compositions (*e.g*., comprising pharmaceutical agents that modulate the level or activity of cancer specific metabolites. The pharmaceutical compositions relating to some embodiments of the present disclosure may be administered in a number of ways depending upon whether local or systemic treatment is desired and upon the area to be treated. Administration may be topical (including ophthalmic and to mucous membranes including vaginal and rectal delivery), pulmonary (*e.g*., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, *e.g*., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and Formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions relating to the present disclosure include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations relating to the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions relating to the present disclosure may be formulated into any of many possible dosage forms such as, but not limited to, tablets capsules, liquid syrups, soft gels, suppositories, and enemas. The compositions relating to the present disclosure may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

In one examples relating to the present disclosure the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

Agents that enhance uptake of oligonucleotides at the cellular level may also be added to the pharmaceutical and other compositions of the present disclosure. For example, cationic lipids, such as lipofectin (U.S. Pat. No. 5,705,188), cationic glycerol derivatives, and polycationic molecules, such as polylysine (WO 97/30731), also enhance the cellular uptake of oligonucleotides.

The compositions relating to the present disclosure may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present disclosure , such as dyes, flavoring agents, preservatives, antioxidants, opacifiers, thickening agents and stabilizers. However, such materials, when added, should not unduly interfere with the biological activities of the components of the compositions of the present disclosure. The formulations can be sterilized and, if desired, mixed with auxiliary agents, *e.g*., lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Certain examples of the disclosure relate to pharmaceutical compositions containing (a) one or more nucleic acid compounds and (b) one or more other chemotherapeutic agents that function by different mechanisms. Examples of such chemotherapeutic agents include, but are not limited to, anticancer drugs such as daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, vincristine, vinblastine, etoposide, teniposide, cisplatin and diethylstilbestrol (DES). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions of the disclosure. Other non-antisense chemotherapeutic agents are also within the scope of this disclosure. Two or more combined compounds may be used together or sequentially.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models or based on the examples described herein. In general, dosage is from 0.01 µg to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the pharmaceutical composition is administered in maintenance doses, ranging from 0.01 µg to 100 g per kg of body weight, once or more daily, to once every 20 years.

### III. Drug Screening Applications

In some examples the present disclosure relates to drug screening assays (*e.g*., to screen for anticancer drugs). The screening methods relating to the present disclosure utilize cancer specific metabolites described herein. As described above, in some examples, test compounds are small molecules, nucleic acids, or antibodies. In some examples, test compounds target cancer specific metabolites directly. In other examples,they target enzymes involved in metabolic pathways of cancer specific metabolites.

In preferred examples, drug screening methods are high throughput drug screening methods. Methods for high throughput screening are well known in the art and include, but are not limited to, those described in U.S. 6468736, WO06009903, and U.S. 5972639.

The test compounds of some examples relating to the present disclosure can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g.*, Zuckennann et al., J. Med. Chem. 37: 2678-85 [1994]); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al.. J. Med. Chem. 37:1233 [1994].

Libraries of compounds may be presented in solution (*e.g*., Houghten, Biotechniques 13:412-421 [1992]), or on beads (Lam, Nature 354:82-84 [1991]), chips (Fodor, Nature 364:555-556 [1993]), bacteria or spores (U.S. Pat. No. 5,223,409; plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. NatI. Acad. Sci. 87:6378-6382 [1990]; Felici, J. Mol. Biol. 222:301 [1991]).

In some examples ,the markers described herein are used to produce a model system for the identification of therapeutic agents for cancer. For example, a cancer-specific biomarker metabolite (for example, sarcosine which activates cell proliferation) can be added to a cell-line to increase the cancer aggressivity of the cell line. The cell line will have an improved dynamic range of response (e.g., 'readout') which is useful to screen for anti-cancer agents. While an in vitro example is described, the model assay system may be in vitro, in vivo or ex vivo.

### VII. Transgenic Animals

The present disclosure relates to the generation of non-human transgenic animals comprising an exogenous gene (*e.g.,* resulting in altered levels of a cancer specific metabolite). In preferred examples the non-human transgenic animal displays an altered phenotype (*e.g*., increased or decreased presence of metabolites) as compared to wild-type animals. Methods for analyzing the presence or absence of such phenotypes include but are not limited to, those disclosed herein. In some preferred examples, the non-human transgenic animals further display an increased or decreased growth of tumors or evidence of cancer.

The transgenic animals relating to the present disclosure find use in drug (*e.g*., cancer therapy) screens. In some examples, test compounds (*e.g*., a drug that is suspected of being useful to treat cancer) and control compounds (*e.g.*, a placebo) are administered to the non-human transgenic animals and the control animals and the effects evaluated_{.}

The non-human transgenic animals can be generated via a variety of methods. In some examples, embryonal cells at various developmental stages are used to introduce transgenes for the production of transgenic animals. Different methods are used depending on the stage of development of the embryonal cell. The zygote is the best target for micro-injection. In the mouse, the male pronucleus reaches the size of approximately 20 micrometers in diameter that allows reproducible injection of 1-2 picoliters (pl) of DNA solution. The use of zygotes as a target for gene transfer has a major advantage in that in most cases the injected DNA will be incorporated into the host genome before the first cleavage (Brinster et al., Proc. Natl. Acad. Sci. USA 82:4438-4442 [1985]). As a consequence, all cells of the transgenic non-human animal will carry the incorporated transgene. This will in general also be reflected in the efficient transmission of the transgene to offspring of the founder since 50% of the germ cells will harbor the transgene. U.S. Pat. No. 4,873,191 describes a method for the micro-injection of zygotes.

In other examples retroviral infection is used to introduce transgenes into a non-human animal. In some examples, the retroviral vector is utilized to transfect oocytes by injecting the retroviral vector into the perivitelline space of the oocyte (U.S. Pat. No. 6,080,912.In other examples, the developing non-human embryo can be cultured in *vitro* to the blastocyst stage. During this time, the blastomeres can be targets for retroviral infection (Janenich, Proc. Natl. Acad. Sci. USA 73:1260 [1976]). Efficient infection of the blastomeres is obtained by enzymatic treatment to remove the zona pellucida (Hogan et al., in Manipulating the Mouse Embryo, Cold Spring Harbor Laboratory Press, Cold Spring Harbor, N.Y. [1986]). The viral vector system used to introduce the transgene is typically a replication-defective retrovirus carrying the transgene (Jahner et al., Proc. Natl. Acad Sci. USA 82:6927 [1985]). Transfection is easily and efficiently obtained by culturing the blastomeres on a monolayer of virus-producing cells (Stewart, et al., EMBO J., 6:383 [1987]). Alternatively, infection can be performed at a later stage. Virus or virus-producing cells can be injected into the blastocoele (Jahner et al., Nature 298:623 [1982]). Most of the founders will be mosaic for the transgene since incorporation occurs only in a subset of cells that form the transgenic animal. Further, the founder may contain various retroviral insertions of the transgene at different positions in the genome that generally will segregate in the offspring. In addition, it is also possible to introduce transgenes into the germline, albeit with low efficiency, by intrauterine retroviral infection of the midgestation embryo (Jahner *et al., supra* [1982]). Additional means of using retroviruses or retroviral vectors to create transgenic animals known to the art involve the micro-injection of retroviral particles or mitomycin C-treated cells producing retrovirus into the perivitelline space of fertilized eggs or early embryos (PCT International Application WO 90/08832 [1990], and Haskell and Bowen, Mol. Reprod. Dev., 40:386 [1995]).

In other examples, the transgene is introduced into embryonic stem cells and the transfected stem cells are utilized to form an embryo. ES cells are obtained by culturing pre-implantation embryos *in vitro* under appropriate conditions (Evans et al., Nature 292:154 [1981]; Bradley et al., Nature 309:255 [1984]; Gossler et al., Proc. Acad. Sci. USA 83:9065 [1986]; and Robertson et al., Nature 322:445 [1986]). Transgenes can be efficiently introduced into the ES cells by DNA transfection by a variety of methods known to the art including calcium phosphate co-precipitation, protoplast or spheroplast fusion, lipofection and DEAE-dextran-mediated transfection. Transgenes may also be introduced into ES cells by retrovirus-mediated transduction or by micro-injection. Such transfected ES cells can thereafter colonize an embryo following their introduction into the blastocoel of a blastocyst-stage embryo and contribute to the germ line of the resulting chimeric animal (for review, See, Jaenisch, Science 240:1468 [1988]). Prior to the introduction of transfected ES cells into the blastocoel, the transfected ES cells may be subjected to various selection protocols to enrich for ES cells which have integrated the transgene assuming that the transgene provides a means for such selection. Alternatively, the polymerase chain reaction may be used to screen for ES cells that have integrated the transgene. This technique obviates the need for growth of the transfected ES cells under appropriate selective conditions prior to transfer into the blastocoel.

In still other examples, homologous recombination is utilized to knock-out gene function or create deletion mutants (*e.g.*, truncation mutants). Methods for homologous recombination are described in U.S. Pat. No. 5,614,396.

### EXPERIMENTAL

The following examples are provided in order to demonstrate and further illustrate certain preferred embodiments and aspects of the present disclosure and are not to be construed as limiting the scope thereof.

### Example 1

### A. Methods:

*Clinical Samples:* Benign prostate and localized prostate cancer tissues were obtained from a radical prostatectomy series at the University of Michigan Hospitals and the metastatic prostate cancer biospecimens were from the Rapid Autopsy Program, which are both part of University of Michigan Prostate Cancer Specialized Program of Research Excellence (S.P.O.R.E) Tissue Core. Samples were collected with informed consent and prior institutional review board approval at the University of Michigan. Detailed clinical information on each of the tissue samples used in the profiling phase of this study is provided in Table 1. Analogous information for tissues and urine samples used to validate sarcosine are given in Tables 5 and 6 respectively. All the samples were stripped of identifiers prior to metabolomic assessment. For the profiling studies, tissue samples were sent to Metabolon, Inc. without any accompanying clinical information. Upon receipt, each sample was accessioned by Metabolon into a LIMS system and assigned unique 10 digit identifier. The sample was bar coded and this anonymous identifier alone was used to track all sample handling, tasks, results etc. All samples were stored at -80°C until use.

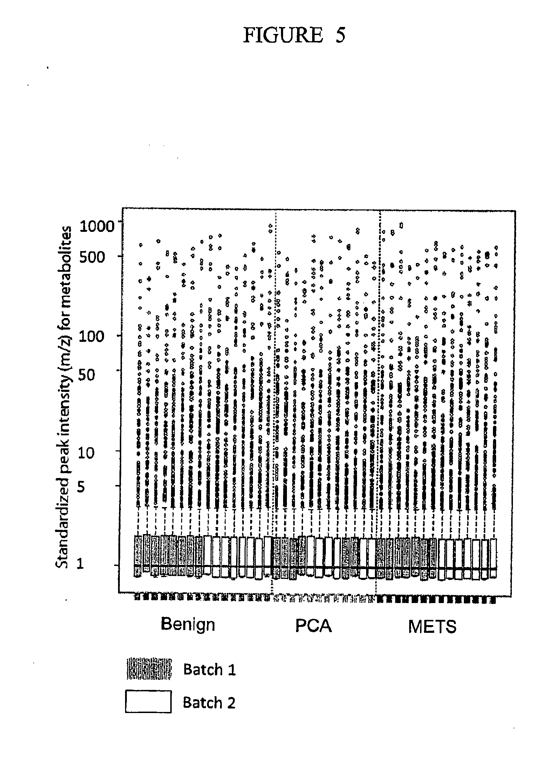

*General Considerations:* The metabolomic profiling analysis of all samples was carried out in collaboration with Metabolon using the following general protocol. All samples were randomized prior to mass spectrometric analyses to avoid any experimental drifts (Fig. 5). A number of internal standards, including injection standards, process standards, and alignment standards were used to assure QA/QC targets were met and to control for experimental variability (see Table 2 for description of standards). The tissue specimens were processed in two batches of 21 samples each. Samples from each of the three tissue diagnostic classes-benign prostate, PCA, and metastatic tumor-were equally distributed across the two batches (Fig. 5). Thus, in each batch there were 8 benign prostates, 6 PCAs, and 7 metastatic tumor samples (Fig. 5). The samples were subsequently processed as described below.

*Sample Preparation:* Samples were kept frozen until assays were to be performed. The sample preparation was programmed and automated. It was performed on a MicroLab STAR® sample prep system from Hamilton Company (Reno, NV). Sample extraction consisted of sequential organic and aqueous extractions. A recovery standard was introduced at the start of the extraction process. The resulting pooled extract was equally divided into a liquid chromatography (LC) fraction and a gas chromatography (GC) fraction. Samples were dried on a Turbo Vap® evaporator (Zymark, Claiper Life Science, Hopkinton, MA) to remove the organic solvent. Finally, samples were frozen and lyophilized to dryness. As discussed specifically below, all samples were adjusted to final solvent strength and volumes prior to injection. Injection standards were introduced during the final resolvation. In addition to controls and blanks, an additional well-characterized sample (a QC control, for QC verification) was included multiple times into the randomization scheme such that sample preparation and analytical variability could be constantly assessed.

*Liquid Chromatography*/*Mass Spectroscopy (LC*/*MS):* The LC/MS portion of the platform is based on a Surveyor HPLC and a Thermo-Finnigan LTQ-FT mass spectrometer (Thermo Fisher Corporation, Waltham, MA). The LTQ side data was used for compound quantitation. The FT side data, when collected, was used only to confirm the identity of specific compounds. The instrument was set for continuous monitoring of both positive and negative ions. Some compounds are redundantly visualized across more than one of these data-streams, however, not only is the sensitivity and linearity vastly different from interface to interface but these redundancies, in some instances, are actually used as part of the QC program.

The vacuum-dried sample was re-solubilized in 100 µl of injection solvent that ontains no less than five injection standards at fixed concentrations. The chromatography was standardized and was never allowed to vary. Internal standards were used both to assure injection and chromatographic consistency. The chromatographic system was operated using a gradient of Acetonitrile (ACN): Water (both solvents were modified by the addition of 0.1 % TFA) from 5% to 100% over an 8 minute period, followed by 100% ACN for 8 min. The column was then reconditioned back to starting conditions. The columns (Aquasil C-18, Thermo Fisher Corporation, Waltham, MA) were maintained in temperature-controlled chambers during use and were exchanged, washed and reconditioned after every 50 injections. As part of Metabolon's general practice, all columns were purchased from a single manufacturer's lot at the outset of these experiments. All solvents were similarly purchased in bulk from a single manufacturer's lot in sufficient quantity to complete all related experiments. All samples were bar-coded by LIMS and all chromatographic runs were LIMS-scheduled tasks. The raw data files were tracked and processed by their LIMS identifiers and archived to DVD at regular intervals. The raw data was processed as described later.

A similar LC/MS protocol as described above was used to assess sarcosine and creatinine in urine supernatants.

*Gas chromatography*/*Mass Spectrometry (GC*/*MS):* For the metabolomic profiling studies, the samples destined for GC were re-dried under vacuum desiccation for a minimum of 24 hours prior to being derivatized under dried nitrogen using bistrimethylsilyl-triflouroacetamide (BSTFA). Samples were analyzed on a Thermo-Finnigan Mat-95 XP (Thermo Fisher Corporation, Waltham, MA) using electron impact ionization and high resolution. The column used for the assay was (5% phenyl)-methyl polysiloxane. During the course of the run, temperature was ramped from 40° to 300° C in a 16 minute period. The resulting spectra were compared against libraries of authentic compounds. As noted above, all samples were scheduled by LIMS and all chromatographic runs were LIMS schedule-based tasks. The raw data files were identified by their LIMS identifiers and archived to DVD at regular intervals. The raw data was processed as described later.

For isotope dilution GC/MS analysis of sarcosine and alanine (in case of urine sediments, Fig 3d), residual water was removed from the samples by forming an azeotrope with 100 µL of dimethylformamide (DMF), and drying the suspension under vacuum. All of the samples were injected using an on column injector and a Agilent 6890N gas chromatograph equipped with a 15-m DB-5 capillary column (inner diameter, 0.2 mm; film thickness, 0.33 micron; J & W Scientific Folsom, CA) interfaced with a Agilent 5975 MSD mass detector. The t-butyl dimethylsilyl derivatives of sarcosine were quantified by selected ion monitoring (SIM), using isotope dilution electron-impact ionization GC/MS. The levels of alanine and sarcosine that eluted at 3.8 and 4.07 minutes respectively, were quantified using their respective ratio between the ion of m/z 232 derived from native metabolite ([M-O-t-butyl-dimethylsilyl]-) and the ions of m/z 233 and 235 respectively for alanine and sarcosine, derived from the isotopically labeled deuteriated internal standard [²H₃] for the compounds. A similar strategy was used for assessment of sarcosine, cysteine, thymine, glycine and glutamic acid in the tissues. The m/z for native and labeled molecular peaks for these compounds were: 158 and 161 (sarcosine), 406 and 407 (cysteine), 432 and 437 (glutamic acid), 297 and 301 (thymine), and 218 and 219 (glycine) respectively. In case of urine supernatants (Fig 3e), sarcosine was measured and normalized to creatinine. Relative area counts for each compound were obtained by manual integration of chromatogram peaks corresponding to each compound using Xcalibur software (Thermo Fisher Corporation, Waltham, MA). The data are presented as the log of the ratio, (sarcosine ion counts)/(creatinine ion counts). For metabolite validation, all the samples were assessed by single runs on the instrument except for sarcosine validation of tissues wherein each sample was run in quadruplicates and the average ratio was used for calculate sarcosine levels. The limit of detection (signal/noise > 10) was ~ 0.1 femtomole for sarcosine using isotope dilution GC/MS.

*Metabolomic Libraries:* These were used to search the mass spectral data. The library was created using approximately 800 commercially available compounds that were acquired and registered into the Metabolon LIMS. All compounds were analyzed at multiple concentrations under the conditions as the experimental samples, and the characteristics of each compound were registered into a LIMS-based library. The same library was used for both the LC and GC platforms for determination of their detectable characteristics. These were then analyzed using custom software packages. Initial data visualization used SAS and Spotfire.

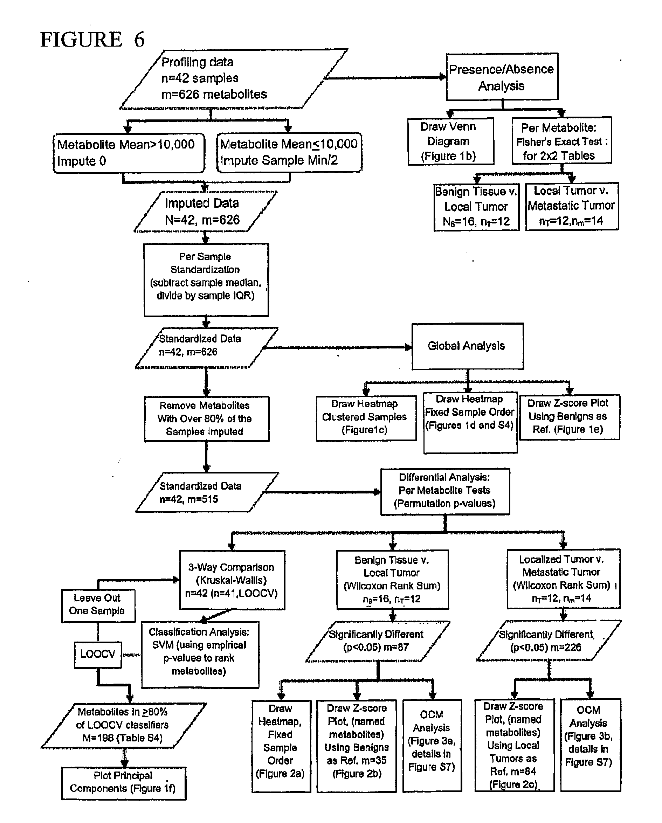

### Statistical Analysis (See Figure 6 for outline):

### a) Metabolomic Data

Data Imputation: The metabolic data is left censored due to thresholding of the mass spectrometer data. The missing values were input based on the average expression of the metabolite across all subjects. If the mean metabolite measure across samples was greater than 100,000, then zero was imputed, otherwise one half of the minimum measure for that sample was imputed. In this way, it was distinguished which metabolites had missing data due to absence in the sample and which were missing due to instrument thresholds. Sample minimums were used for the imputed values since the mass spectrometer threshold for detection may differ between samples and it was preferred that that threshold level be captured.

Sample Normalization: To reduce between-sample variation the imputed metabolic measures for each tissue sample was centered on its median value and scaled by its interquartile range (IQR).

### Analysis:

z-score: This z-score analysis scaled each metabolite according to a reference distribution. Unless otherwise specified, the benign samples were designated as the reference distribution. Thus the mean and standard deviation of the benign samples was determined for each metabolite. Then each sample, regardless of diagnosis, was centered by the benign mean and scaled by the benign standard deviation, per metabolite. In this way, one can look at how the metabolite expressions deviate from the benign state.

Hierarchical Clustering: Hierarchical clustering was performed on the log transformed normalized data. A small value (unity) was added to each normalized value to allow log transformation. The log transformed data was median centered, per metabolite, prior to clustering for better visualization. Pearson's correlation was used for the similarity metric. Clustering was performed using the Cluster program and visualized using Treeview 1. A maize/blue color scheme was used in heat maps of the metabolites.

Comparative Tests: To look at association of metabolite detection with diagnosis, the measure were dichotomized as present or absent (i.e., undetected). Chi-square tests were used to assess difference in rates of presence/absence of measurements for each metabolite between diagnosis groups. To assess the association between metabolite expression levels between diagnosis groups, two-tailed Wilcoxon rank sum tests were used for two-sample tests; benign vs. PCA, PCA vs. Mets. Kruskal-Wallis tests were used for three-way comparisons between all diagnosis groups; benign vs. PCA vs. Mets. Non-parametric tests were used reduce the influence of the imputed values. Tests were run per metabolite on those metabolites that had detectable expression in at least 20% of the samples. Significance was determined using permutation testing in which the sample labels were shuffled and the test was recomputed. This was repeated 1000 times. Tests in which the original statistic was more extreme than the permuted test statistic increased evidence against the null hypothesis of no difference between diagnosis groups. False discovery rates were determined from the permuted P-value using the q-value conversion algorithm of Storey et al 2 as implemented in the R package "q-value". Pairwise differences in expression in the cell line data and small scale tissue data were tested using two-tailed t-tests with Satterthwaite variance estimation. Comparisons involving multiple cell lines used repeated measures analysis of variance (ANOVA) to adjust for the multiple measures per cell line. Fold change was estimated using ANOVA on a log scale, following the model log(Y) = A + B*Treatment + E. In this way exp(B) is an estimate of (Y |Treatment = 1)/(Y | Treatment = 0) and the standard error of exp(B) can be estimated from SE(B) using the delta method.

Classification: Metabolites were added to classifiers based on increasing empirical p P-value. Support vector machines (SVM) were used to determine an optimal classifier. Leave-one-out cross validation (LOOCV) was employed to estimate error rates among classifiers. To avoid bias, comparative tests to determine the empirical *P*-value ranking, were repeated for each leave-one-out sample set. SVM selected the optimal empirical P-value for inclusion in the classifier. Those metabolites that appeared in at least 80% of the LOOCV samples at or below the chosen empirical *P*-value were selected as the classification set. A principal components analysis was used to help visualize the separation provided by the resulting classification set of metabolites. Principal components one, two, and four were used for plotting.

Validation of Sarcosine in Urine: Urine sediment experiments were performed across three batches; batch-level variation was removed using two adjustments. First, two batches (n=15 and n=18) with available measurements on cell line controls DU145 and RWPE were combined by estimating batch-level differences using only this cell line data in an ANOVA model with the log-transformed ratio of sarcosine to alanine as the response. The second adjustment put the resulting combined batches (n=33) together with the remaining third batch (n=60) by centering (by the median) and scaling (by the median absolute deviation) within each of these two batches. As seen in Figure 12, the ratio of sarcosine to alanine was predictive of biopsy status not only in the combined dataset but also in each of these two smaller batches separately.

Urine supernatant experiments measured sarcosine in relation to creatinine. Analysis was performed using a log base 2 scale to indicate fold change from creatinine. Urine sediments and supernatants were tested for differences between biopsy status using a two-tailed Wilcoxon rank-sum test. Associations with clinical parameters were assessed by Pearson correlation coefficients for continuous variables and two-tailed Wilcoxon rank-sum tests for categorical variables.

### b) Gene Expression:

Expression profiling of sarcosine-treated PrEC prostate epithelial cells. Expression profiling of PrEC cells treated with either 50 µM alanine or sarcosine for 6h, was performed using the Agilent Whole Human Genome Oligo Microarray (Santa Clara, CA). Total RNA isolated using Trizol from the treated cells was purified using the Qiagen RNAeasy Micro kit (Valencia, CA). Total RNA from untreated PrEC cells were used as the reference. One µg of total RNA was converted to cRNA and labeled according to the manufacturer's protocol (Agilent). Hybridizations were performed for 16 hrs at 65°C, and arrays were scanned on an Agilent DNA microarray scanner. Images were analyzed and data extracted using Agilent Feature Extraction Software 9.1.3.1, with linear and lowess normalization performed for each array. A technical replicate was included for each of the two treatments. Fold change was determined as the ratio of sarcosine to alanine for each of two replicates. Genes considered further - showed a two fold change, either up or down, in both replicates.

Mapping of "Omics" data to a common identifier. The metabolites profiled in example were mapped to the metabolic maps in KEGG using their compound IDs, followed by identification of all the anabolic and catabolic enzymes in the mapped pathways. This was followed by retrieval of the official enzyme commission number (EC number) for the enzymes that were mapped to its official gene ID using KEGG's DBGET integrated data retrieval system.

Enrichment of Molecular Concepts. In order to explore the network of interrelationships among various molecular concepts and the integrated data (containing information from metabolome), the Oncomine Concepts Map bioinformatics tool was used (Rhodes et al., Neoplasia 9, 443-454 (2007); Tomlins et al., Nat Genet 39, 41-51 (2007)). In addition to being the largest collection of gene sets for association analysis, the Oncomine Concepts Map (OCM) is unique in that computes pair-wise associations among all gene sets in the database, allowing for the identification and visualization of "enrichment networks" of linked concepts. Integration with the OCM allows one to systematically link molecular signatures (i.e., in this case metabolomic signatures) to over 14,000 molecular concepts. To study the enrichments resulting from the metabolomic data alone involved generation of a list of gene IDs from the metabolites that were significant with a *P*-value less than 0.05 for the comparisons being made. This signature was used to seed the analysis. On a similar note for gene expression-based enrichment analysis, we used gene IDs for transcripts that were significant (p<0.05) for the comparisons being made. Once seeded, each pair of molecular concepts was tested for association using Fisher's exact test. Each concept was then analyzed independently and the most significant concept reported. Results were stored if a given test had an odds ratio > 1.25 and P-value < 0.01. Adjustment for multiple comparisons was made by computing q-values for all enrichment analyses. All concepts that had a P-value less than 1x10⁻⁴ were considered significant. Additionally, OCM was used to reveal the biological nuance underlying sarcosine-induced invasion of prostate epithelial cells. For this the list of genes that were up regulated by 2-fold upon sarcosine treatment compared to alanine treatment, in both the replicates were used for the enrichment.

### B. Results

A number of groups have employed gene expression microarrays to profile prostate cancer tissues (Dhanasekaran et al., Nature 412, 822-826. (2001); Lapointe et al., Proc Natl Acad Sci U S A 101, 811-816 (2004); LaTulippe et al., Cancer Res 62, 4499-4506 (2002); Luo et al., Cancer Res 61, 4683-4688. (2001); Luo et al., Mol Carcinog 33, 25-35. (2002); Magee et al., Cancer Res 61, 5692-5696. (2001); Singh et al., Cancer Cell 1, 203-209. (2002); Welsh et al., Cancer Res 61, 5974-5978. (2001); Yu et al., J Clin Oncol 22, 2790-2799 (2004)) as well as other tumors (Golub, T.R., et al. Science 286, 531-537 (1999); Hedenfalk et al. The New England Journal of Medicine 344, 539-548 (2001); Perou et al., Nature 406, 747-752 (2000); Alizadeh et al., Nature 403, 503-511 (2000)) at the transcriptome level, and to a more limited extent, at the proteome level (Ahram et al., Mol Carcinog 33, 9-15 (2002); Hood et al., Mol Cell Proteomics 4, 1741-1753 (2005); Prieto et al., Biotechniques Suppl, 32-35 (2005); Varambally et al., Cancer Cell 8, 393-406 (2005); Martin et al., Cancer Res 64, 347-355 (2004); Wright et al., Mol Cell Proteomics 4, 545-554 (2005); Cheung et al., Cancer Res 64, 5929-5933 (2004)). However, in contrast to genomics and proteomics, metabolomics (i.e., examining metabolites with a global, unbiased perspective) is an emerging science, and represents the distal read-out of the cellular state as well as associated pathophysiology. As part of a systems biology perspective, metabolomic profiling is a useful complement to other approaches.

Metabolomic profiling has long relied on the use of high pressure liquid chromatography (HPLC), nuclear magnetic resonance (NMR) (Brindle et al., J Mol Recognit 10, 182-187 (1997)), mass spectrometry (Gates and Sweeley, Clin Chem 24, 1663-1673 (1978)) (GC/MS and LC/MS) and Enzyme Linked Immuno Sorbent Assay (ELISA). Using such techniques in a focused approach, most of the early studies on neoplastic metabolism have interrogated tumor adaptation to hypoxia (Dang and Semenza, Trends Biochem Sci 24, 68-72 (1999); Kress et al., J Cancer Res Clin Oncol 124, 315-320 (1998)). These investigations revealed heterogeneity within the tumor constituted by varying gradients of metabolites (e.g., glucose or oxygen) and growth factors, which allow neoplastic cells to thrive under conditions of low oxygen tension (Dang and Semenza, supra). Among these targeted approaches are studies that have implicated citrate and choline in the process of prostate cancer progression (Mueller-Lisse et al., European radiology 17, 371-378 (2007); Wu et al., Magn Reson Med 50, 1307-1311 (2003)). Multiple groups have also used cell line models to understand changes in the energy utilization pathways with different degrees of tumor aggressiveness (Vizan et al., Cancer Res 65, 5512-5515 (2005); Al-Saffar et al., Cancer Res 66, 427-434 (2006)). Ramanathan et al. have used metabolic profiling as a tool to correlate different stages of tumor progression with bioenergetic pathways (Proc Natl Acad Sci U S A 102, 5992-5997 (2005). More recently, holistic interrogation of the metabolome using nuclear magnetic resonance (Wu et al., supra; Cheng et al., Cancer Res 65, 3030-3034 (2005); Bums et al., Magn Reson Med 54, 34-42 (2005); Kurhanewicz et al., J Magn Reson Imaging 16, 451-463 (2002)) and gas chromatography, coupled with time-of-flight mass spectrometry (Denkert et al., Cancer Res 66, 10795-10804 (2006); Ippolito et al., Proc Natl Acad Sci U S A 102, 9901-9906 (2005)), have revealed the power of metabolomic signatures in classifying tumor populations. Despite this increase in power, however, the number of metabolites monitored in these studies is limited.

Prostate cancer is the second most common cause of cancer-related death in men in the western world and afflicts one out of nine men over the age of 65 (Abate-Shen and Shen, Genes Dev 14, 2410-2434 (2000); Ruijter et al, Endocr Rev 20, 22-45 (1999)). To better understand the complex molecular events that characterize prostate cancer initiation, unregulated growth, invasion, and metastasis, it is important to delineate the distinct sets of genes, proteins, and metabolites that dictate its progression from precursor lesion, to localized disease, and subsequent metastasis. With the advent of global profiling strategies, such a systematic analysis of molecular alterations is now possible.

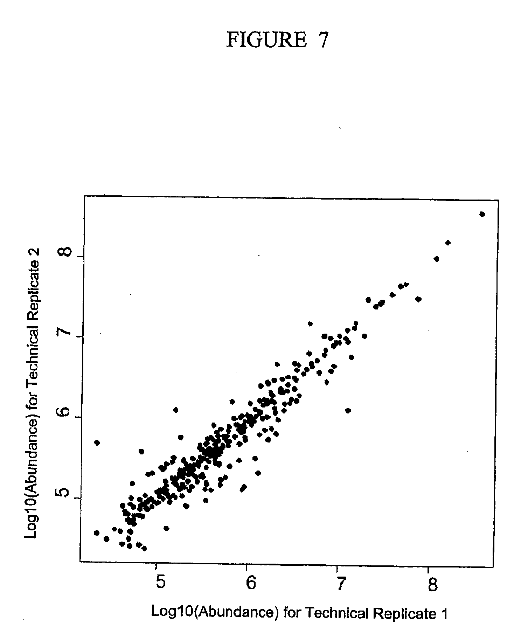

In order to profile the metabolome during prostate cancer progression, a combination of liquid and gas chromatography, coupled with mass spectrometry, was used to interrogate the relative levels of metabolites across 42 prostate-related tissue specimens. Figure 1a outlines the strategy employed for metabolomic profiling. Specifically, this study included benign adjacent prostate specimens (n=16), clinically localized prostate cancers (PCA, n=12), and metastatic prostate cancers (Mets, n=14) (Fig. 1b). Additionally, selection of metastatic tissue samples from different sites minimized the contribution from nonprostatic tissue (see Table 1 for clinical information). Tissue specimens were processed in two groups, each of which were comprised of equally distributed specimens from the three classes (Fig. 5). The technology component of the metabolomics platform used in this study is described in Lawton et al. (Pharmacogenomics 9: 383 (2008)) and outlined in Figure 1a. This process involved: sample extraction, separation, detection, spectral analysis, data normalization, delineation of class-specific metabolites, pathway mapping, validation and functional characterization of candidate metabolites (Fig. 6 provides an outline of the data analysis strategy). The reproducibility of the profiling process was addressed at two levels; one by measuring only instrument variation, and the other by measuring overall process variation (refer to Table 2 for a list of controls used to assess reproducibility). Instrument variation was measured from a series of internal standards (n=14 in this study) added to each sample just prior to injection. The median coefficient of variation (CV) value for the internal standard compounds was 3.9%. To address overall process variability, metabolomic studies were augmented to include a set of nine experimental sample technical replicates (also called matrix, abbreviated as MTRX), which were spaced evenly among the injections for each day. Reproducibility analysis for the n=339 compounds detected in each of these nine replicate samples gave a measure of the combined variation for all process components including extraction, recovery, derivatization, injection, and instrument steps. The median CV value for the experimental sample technical replicates (tissue profiling part of this study) was 14.6%. Figure 7 shows the reproducibility of these experimental-sample technical replicates; Spearman's rank correlation coefficient between pairs of technical replicates ranged from 0.93 to 0.97.

The above authenticated process was used to quantify the metabolomic alterations in prostate-derived tissues. In total, high throughput profiling of prostate tissues identified 626 metabolites (175 named, 19 isobars, and 432 metabolites without identification) that were quantitatively detected in the tissue samples across the three tissue classes (see Table 3 for a complete list of metabolites profiled). Of these, 515 metabolites were shared across all the three classes (Fig. 1b). There were 60 metabolites found in PCA and/or metastatic tumors but not in benign prostate.

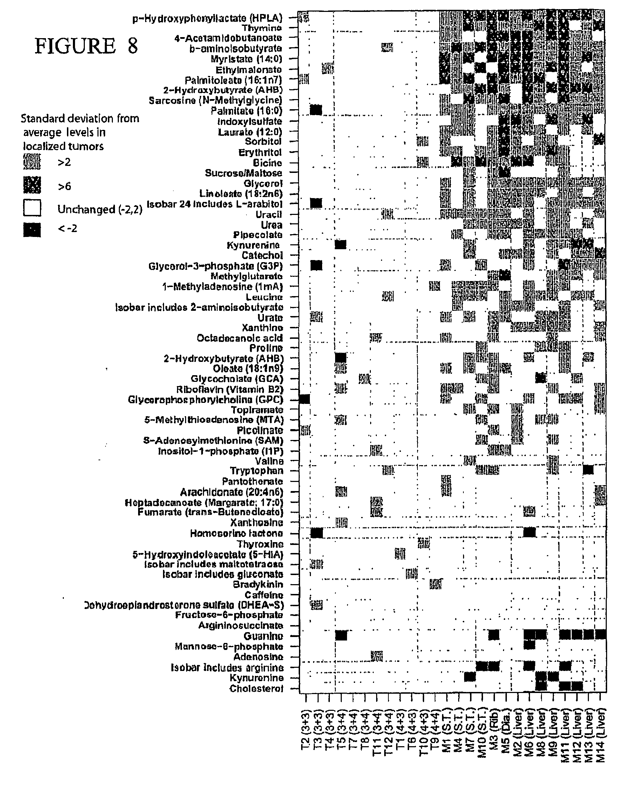

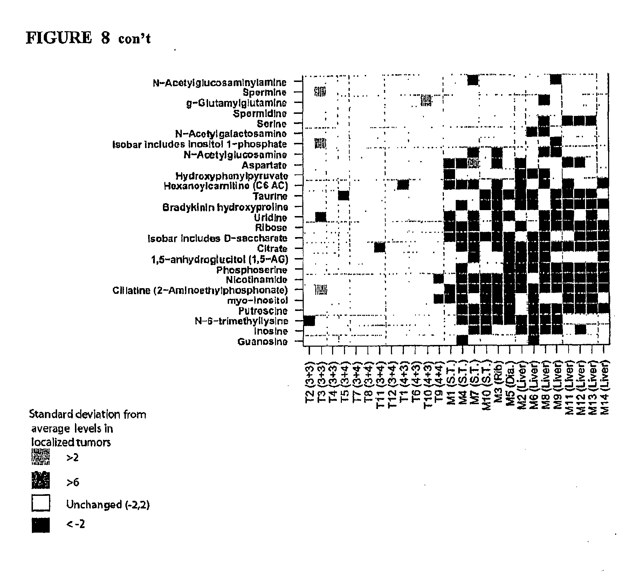

Three analyses were performed to provide a global perspective of the data. The first employed unsupervised hierarchical clustering on the normalized data (refer to Fig. 6 for detailed outline of data analysis methods for procedural details). This analysis separated the metastatic samples from both the benign and PCA tissues, but it did not accurately cluster the clinically localized prostate cancers from the benign prostates (Fig. 1c). This indicated a higher degree of metabolomic alteration in the metastatic samples relative to benign and PCA specimens highlighted by the heat map representation of the data. This finding is consistent with earlier observations based on gene expression analyses (Dhanasekaran et al., supra; Tomlins et al., Nat Genet 39, 41-51 (2007). Further, as shown in Figure 8, this pattern ofmetabolomic alterations was shared across multiple metastatic samples derived from different sites of origin.

In the second analysis, each metabolite was centered on the mean and scaled on the standard deviation of the normalized benign metabolite levels to create z-scores based on the distribution of the benign samples (see Fig. 6 and methods for details). Figure 1d shows the 626 metabolites plotted on the vertical-axis, and the benign-based z-score for each sample plotted on the horizontal-axis for each class of sample. As illustrated by the figure, changes in metabolomic content occur most robustly in metastatic tumors (z-score range: -13.6 to 81.9). In particular, there were 105 metabolites that had a z-score of two or greater in at least 33% of the metastatic samples analyzed. In contrast, the changes in clinically localized prostate cancer samples were less than in metastatic disease (z-score range: -7.7-45.8) such that only 38 metabolites had a z-score of two or greater in at least 33% of the samples.

To investigate the classification potential of metabolomic profiles, the third analysis used a support vector machine (SVM) classification algorithm with leave-one out cross-validation (see Methods). This predictor correctly identified all of the benign and metastatic samples, with misclassification of 2/12 PCA samples as benign. The two misclassified cancer samples had a low Gleason grade of 3+3, which indicates less aggressive tumors. In addition, a list of 198 metabolites that were significant at a P=0.05 level in at least 80% of the leave-one-out cross-validated datasets was generated. (See Table 4 for the list of 198 metabolites). For visualization, principal component analysis was employed on this data matrix of 198 metabolites (Fig. 1e). The resulting figure was similar to the classification obtained using SVM; the samples were well delineated using only three principal components.

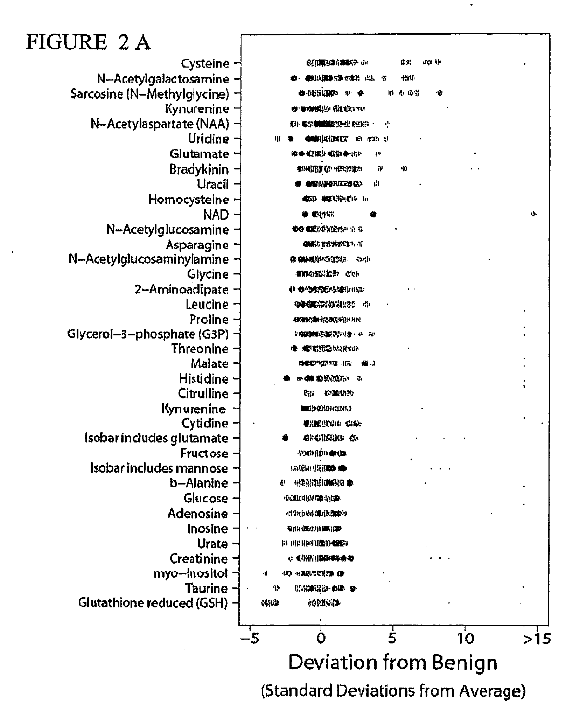

To further delineate the metabolomic elements that distinguish the three classes of samples analyzed, differential alterations between the PCA and benign samples were selected using a Wilcoxon rank-sum test coupled with a permutation test (n = 1,000). A total of 87/518 metabolites were differential across these two classes at a P-value cutoff of 0.05, corresponding to a false discovery rate of 23%. For visualizing the relationship between 87 dysregulated metabolites across disease states, hierarchical clustering was used to arrange the metabolites based on their relative levels across samples. Among the perturbed metabolites, 50 were elevated in PCA while 37 were downregulated. Figure 2a displays the relative levels of the 37 named metabolites that were differential between benign prostate and PCA. Among the upregulated metabolites were a number of amino acids, namely cysteine, glutamate, asparagine, glycine, leucine, proline, threonine, and histidine or their derivatives like sarcosine, n-acetyl-aspartic acid, etc. Those that were down-regulated included inosine, inositol, adenosine, taurine, creatinine, uric acid, and glutathione.

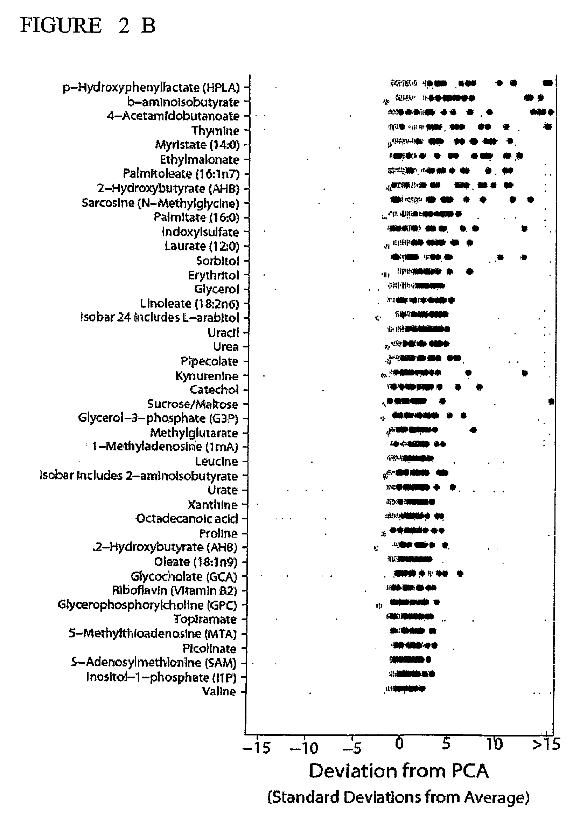

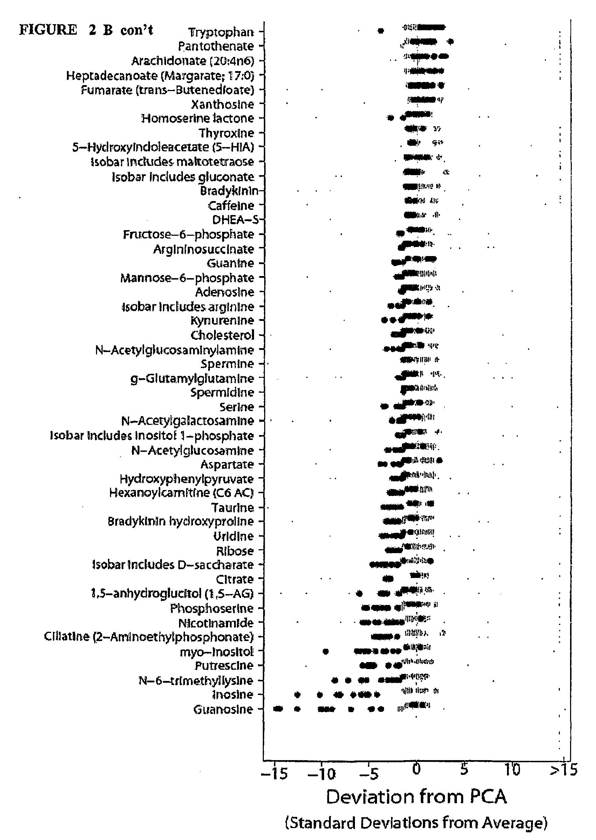

A similar approach was used to identify differential metabolites in metastatic prostate cancer and resulted in 124 metabolites that were elevated in the metastatic state compared to the organ-confined state, with 102 compounds down-regulated and 289/518 (56%) unchanged (at a P-value cutoff of 0.05, corresponding to an false discovery rate of 4%). Figure 2b displays the levels of the 81 named metabolites that were dysregulated during cancer progression. This includes metabolites that were only detected in metastatic prostate cancer: 4-acetamidobutryic acid, thymine, and two unnamed metabolites. A subset of six metabolites was significantly elevated upon disease advancement. These included sarcosine, uracil, kynurenine, glycerol-3-phosphate, leucine and proline. By virtue of their occurrence in a subset of the PCA samples and a majority of the metastatic samples, these metabolites serve as biomarkers for progressive disease

Upon defining class-specific metabolomic patterns, these changes were evaluated in the context of biochemical pathways and delineation of altered biochemical processes during prostate cancer development and progression. The metabolomic profiles were first mapped to their respective pathways as outlined in the Kyoto Encyclopedia of Genes and Genomes (KEGG, release 41.1). This revealed an increase in amino acid metabolism and nitrogen breakdown pathways during cancer development, supporting the gene expression based prediction of androgen-modulated increased protein synthesis as an early event during prostate cancer development (Tomlins et al, 2007; supra). These trends persisted, and even increased, during the progression to the metastatic disease.

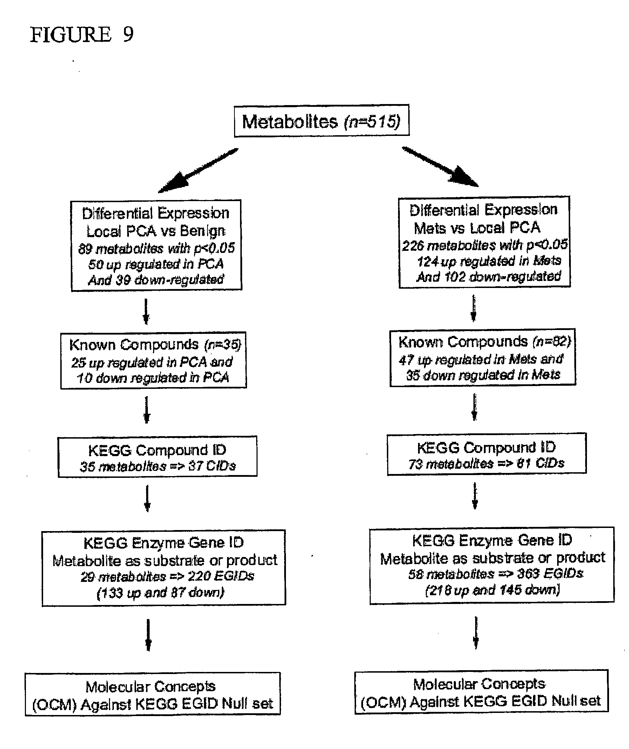

Additionally, the class-specific coordinated metabolite patterns were examined using the bioinformatics tool, Oncomine Concept Maps that permitted systematic linkages of metabolomic signatures to molecular concepts, generating novel hypotheses about the biological progression of prostate cancer (refer to Fig. 9 for an outline of the analyses for localized prostate cancer and metastatic prostate cancer and to the Methods for a description of OCM) (Rhodes et al., Neoplasia 9, 443-454 (2007)). Consistent with the KEGG analysis, the Oncomine analysis expanded upon this theme and (Fig. 3a) and identified an enrichment network of amino acid metabolism in these specimens (Fig. 3a). These included the most enriched GO Biological processes; amino acid metabolism (P = 6 X 10⁻¹³) and KEGG pathway for glutamate metabolism (P = 6.1 X 10-24). KEGG pathways for glycine, serine and threonine metabolism (P = 2.8 X 10⁻¹⁴), alanine and aspartate metabolism (P= 3.3 X 10⁻¹¹), arginine and proline metabolism (P = 2.3 X 10⁻¹⁰) and urea cycle and metabolism of amino groups (P= 1.7 X 10-6) also showed strong enrichment.

The metabolomic profiles for compounds "over-expressed in metastatic samples" (Fig. 3b) showed strong enrichment for elevated methyltransferase activity (Fig. 3b). This increased methylation potential was supported by multiple enrichments of S-adenosyl methionine (SAM) mediated methyltransferase activity including: the enriched InterPro concept, SAM binding motif (P = 1.1 X 10⁻¹¹) and GO Molecular Function, methyltransferase activity (P = 7.7 X 10⁻⁸). These enrichments were a result of significant elevation in the levels of S-adenosyl methionine (P = 0.004) in the metastatic samples compared to the PCA samples. The resulting enhancement in the methylation potential of the tumor was further supported by additional concepts that described increased chromatin modification (GO Biological Process, P = 2.9 X 10⁻⁶), involvement of SET domain containing proteins (InterPro, P = 7.4 X 10⁻⁷) and histone-lysine N-methyltransferase activity (GO Molecular Function, P = 6.3 X 10⁻⁶) in the metastatic samples (Fig. 3b). This corroborates earlier studies showing elevation of the SET domain containing histone methyltransferase EZH2 in metastatic tumors (Rhodes et al., Neoplasia 9, 443-454 (2007); Varambally et al., Nature 419, 624-629 (2002); van der Vlag and Otte, Nat Genet 23, 474-478. (1999); Laible et al., Embo J 16, 3219-3232. (1997); Cao et al., Science 298, 1039-1043. (2002); Kleer et al., Proc Natl Acad Sci U S A 100, 11606-11611 (2003).

In light of the enrichment of the amino acid precursors and the methylation potential of the tumor, metabolomic biomarkers that typified both of these mechanisms were characterized. The amino acid metabolite sarcosine, an N-methyl derivative of glycine, fit this criteria in that it is methylated and expected to increase in the presence of an excess amino acid pool and increased methylation (Mudd et al., Metabolism: clinical and experimental 29, 707-720 (1980)). Indeed, the metabolomic profile of metastatic samples showed markedly elevated levels of sarcosine in 79 % of the specimens analyzed (Chi-Square test, P = 0.0538), whereas 42% of the PCA samples showed a step-wise increase in the levels of this metabolite (Fig. 2 a-b). None of the benign samples had detectable levels of sarcosine.

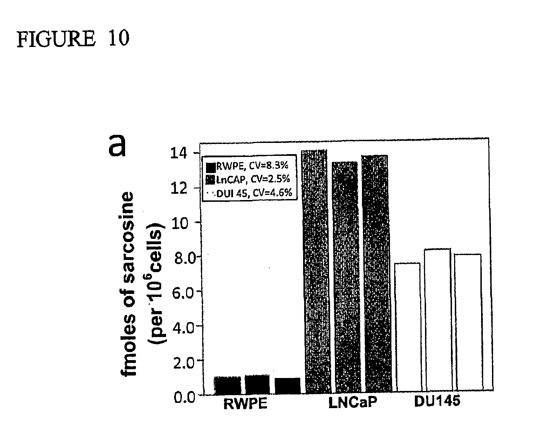

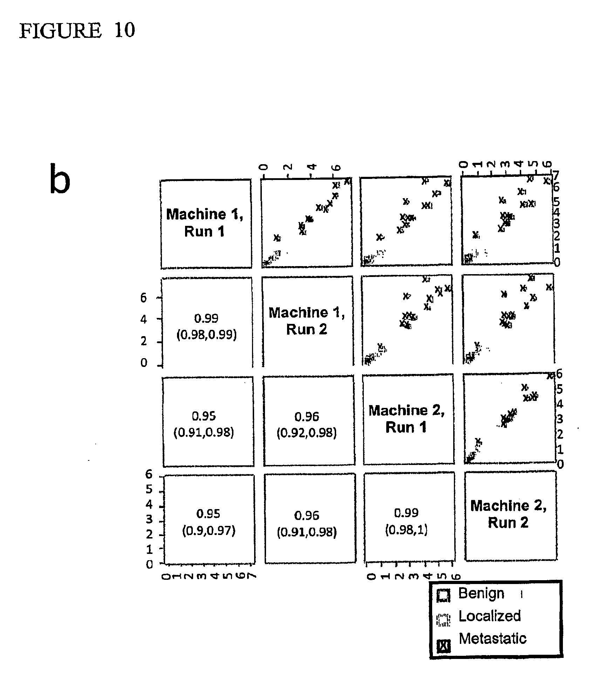

The level of sarcosine in the metastatic samples was significantly greater than PCA samples (Wilcoxon rank-sum test, P=0.005) (Fig. 2b), rendering it clinically useful as a metabolite marker, and for the monitoring of disease progression and aggressiveness. For confirmation, a highly sensitive and specific isotope dilution GC/MS method of accurately quantifying sarcosine from tissue and cellular samples (limit of detection = 0.1 fmole) was developed. Figure 10 illustrates the reproducibility of the GC-MS platform using both prostate-derived cell lines and tissues.

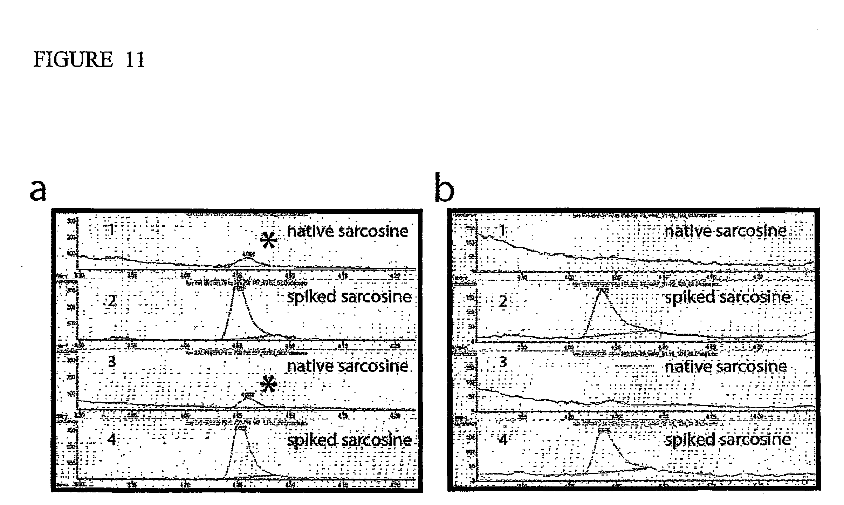

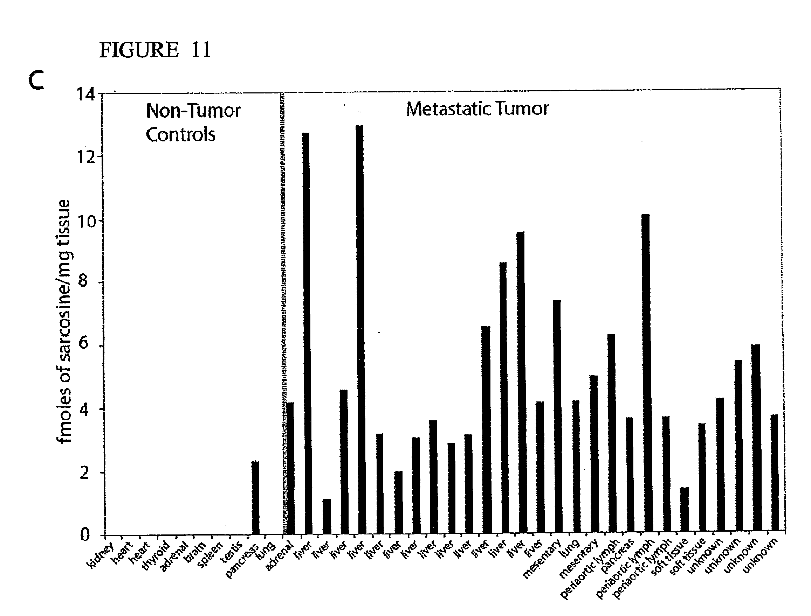

Using this method, the utility of sarcosine as a biomarker was validated in an independent set of 89 tissue samples (25 benign, 36 PCA and 28 metastatic prostate cancers (see Table 5 for sample information). As shown in Figure 3c, sarcosine levels were significantly elevated in PCA samples compared to benign samples (Wilcoxon rank-sum, P = 4.34 X10⁻¹¹). Additionally, sarcosine levels displayed an even greater elevation in the metastatic samples compared to organ-confined disease (Wilcoxon rank-sum, P = 6.02 X 10⁻¹¹). No association of sarcosine with the site of tumor growth was evident, as noted by its absence in organs derived from metastatic patients that were negative for neoplasm (Fig. 11. a-c). The increase of four additional metabolites in prostate cancer progression were validated these using targeted mass spectrometric assays. As shown in Figure 14, levels of cysteine, glutamic acid, glycine and thymine were all elevated upon progression from benign to localized prostate cancer and advancement into metastatic disease.

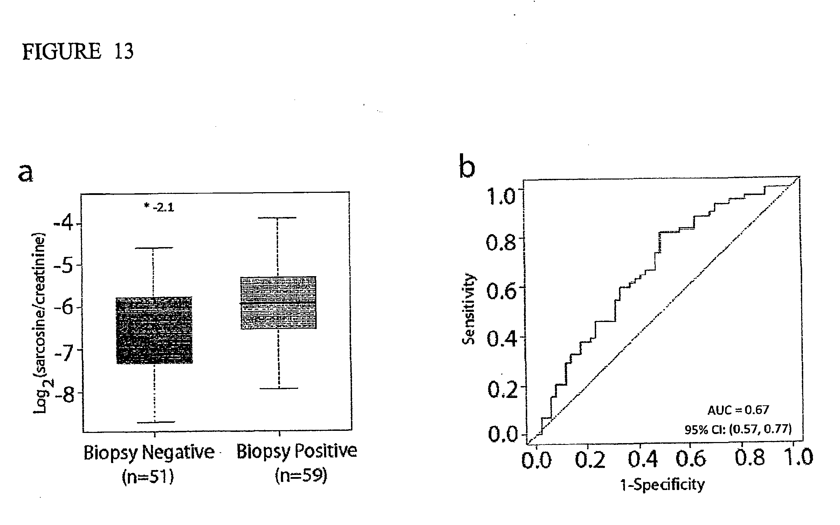

A biomarker panel for early disease detection was developed. As a first step, the ability of sarcosine to function as a non-invasive prostate cancer marker, in the urine of biopsy positive and negative individuals was assayed. Sarcosine was independently assessed in both urine sediments and supernatants derived from this clinically relevant cohort (203 samples derived from 160 patients, with 43 patients contributing both urine sediment and supernatant, see Table 6 for clinical information). Sarcosine levels were reported as a log ratio to either alanine levels (in case of urine sediments) or creatinine levels (in case of urine supernatants). Both alanine and creatinine served as controls for variations in urine concentration. The average standardized (to alanine or creatinine) log ratio for sarcosine was significantly higher in both the urine sediments (n= 49) and supernatants (n=59) derived from biopsy-proven prostate cancer patients as compared to biopsy negative controls (n = 44 urine sediments and n= 51 urine supernatants, Fig. 3d, for urine sediment, Wilcoxon P=0.0004 and Fig. 3e, for urine supernatant, Wilcoxon P = 0.0025). As shown in Figure 12f, receiver operator characteristic (ROC) curves for sarcosine assessment in urine sediments (n=93) gave an AUC of 0.71. Similarly, sarcosine assessment in urine supernatants (n=110) resulted in a comparable AUC of 0.67 (Fig.13b), indicated that sarcosine finds use as a non-invasive marker for detection of prostate cancer. Further sarcosine levels, both in urine sediment and supernatant were not correlated to various clinical parameters like age, PSA and gland weight (Table 7). As a single marker, these performance criteria are equal or superior to currently available prostate cancer biomarkers.

To investigate the biological role of sarcosine elevation in prostate cancer, prostate cancer cell lines VCaP, DU145, 22RV1 and LNCaP and their benign epithelial counterparts, primary benign prostate epithelial cells PrEC and immortalized benign RWPE prostate cells were used. The sarcosine levels of these cell lines was analyzed using isotope dilution GC/MS and cellular invasion was assayed using a modified Boyden chamber matrigel invasion assay (Kleer et al., Proc Natl Acad Sci U S A 100, 11606-1161 (2003). As shown in Figure 4a, the prostate cancer cell lines displayed significantly higher levels of sarcosine (P = 0.0218, repeated measures ANOVA) compared to their benign epithelial counterparts (mean ± SEM in fmoles/million cells: 9.3 ± 1.04 vs. 2.7 ± 1.49). Further, sarcosine levels in these cells correlated well with the extent of their invasiveness (Fig. 4a, Spearman's correlation coefficient: 0.943, P=0.0048).

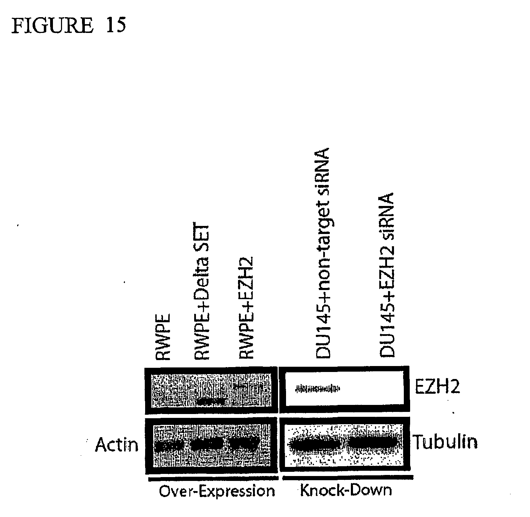

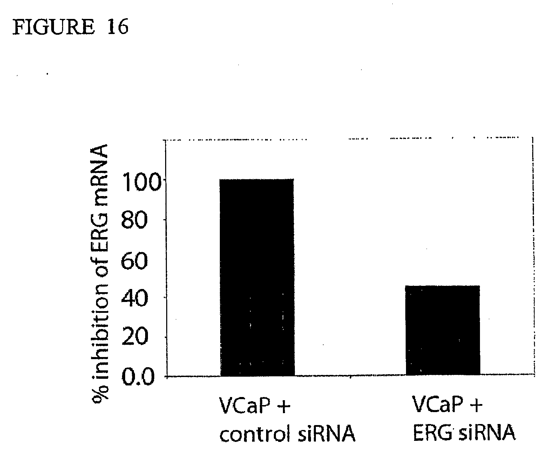

Based on earlier findings that EZH2 over-expression in benign cells could mediate cell invasion and neoplastic progression (Varambally et al., 2002, supra; Kleer et al., 2003, supra), sarcosine levels were compared to EZH2 expression. Sarcosine levels were elevated by 4.5 fold upon EZH2-induced invasion in benign prostate epithelial cells. By contrast, DU145 cells are an invasive prostate cancer cell line in which transient knock-down of EZH2 attenuated cell invasion that was accompanied by approximately 2.5 fold decrease in sarcosine levels (Fig. 4B and Fig. 15). Thus, over-expression of oncogenic EZH2 induces sarcosine production while knock-down of EZH2 attenuates sarcosine production. The association of sarcosine with prostate cancer was further strengthened by studies using TMPRSS2-ERG and TMPRSS2-ETV1 gene fusion models of prostate cancer. Recurrent gene fusions involving ETS family of transcription factors (ERG and ETV1) with TMPRSS2 are integral for prostate cancer development (Tomlins et al., Cancer Res 66, 3396-3400 (2006); Tomlins et al., Science 310, 644-648 (2005)). Sarcosine levels upon constitutive over-expression or attenuation of the fusion products in prostate-derived cell lines were tested. Both TMPRSS2-ERG and TMPRSS2-ETV induced invasion (P = 0.0019 for TMPRSS2-ERG vs control, and 0.0057 for TMPRSS2-ETV1 vs control) associated with a 3-fold sarcosine elevation in benign prostate epithelial cells (Fig. 4c, over-expression, mean ± SEM in fmoles/million cells: 3.3 ± 0.1 for TMPRSS2-ERG and 3.4 ± 0.2 for TMPRSS2-ETV1 vs 0.5 ± 0.3 for control, P = 0.0035 for ERG vs control and 0.0016 for ETV1 vs control). Similarly, knock- down of TMPRSS2-ERG gene fusion in VCaP cells (which harbor this gene fusion) resulted in > 3 fold decrease in the levels of the metabolite with a similar decrease in the invasive phenotype (Fig 4c, knock-down, see Figure 16 for transcript levels of ERG upon siRNA-mediated knock-down).

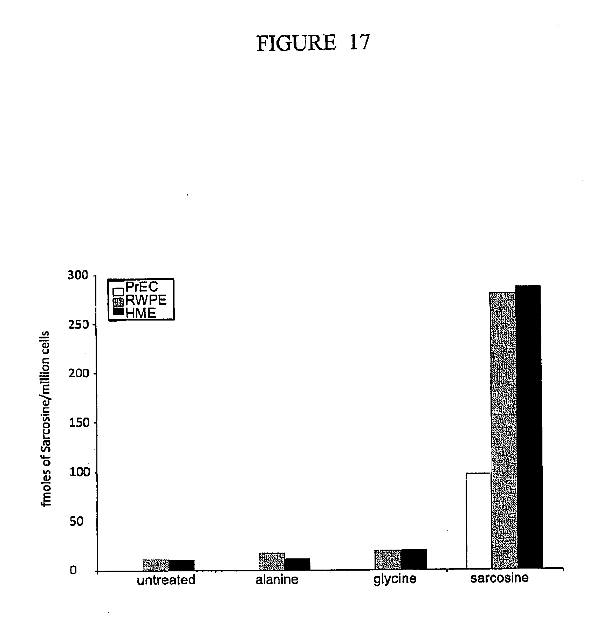

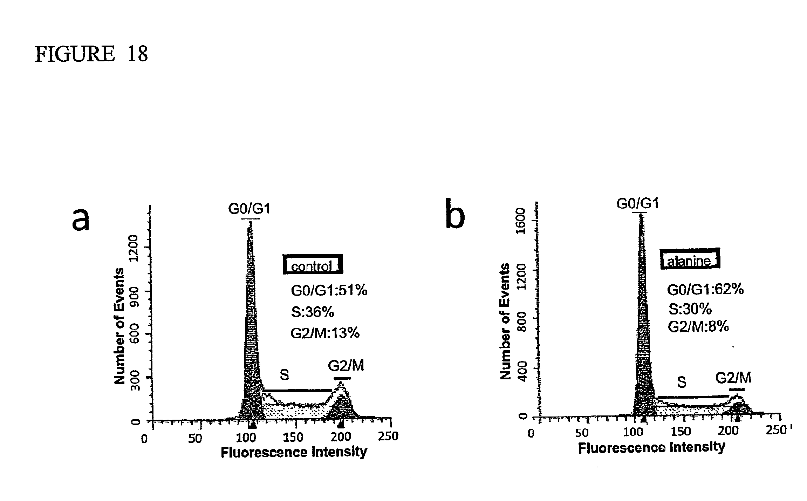

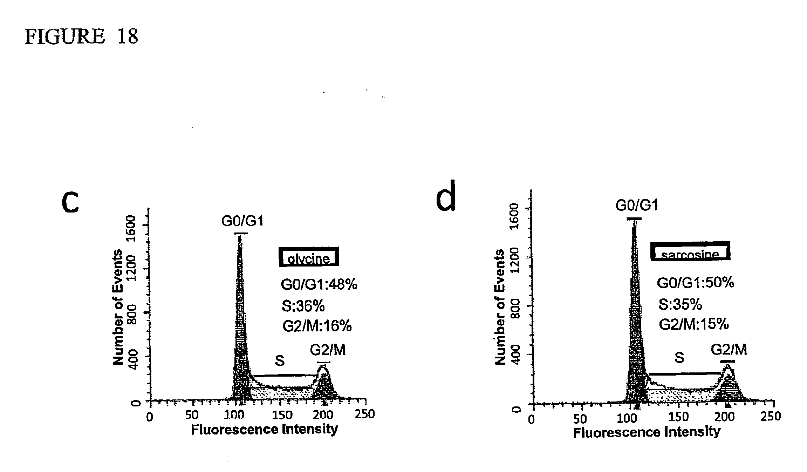

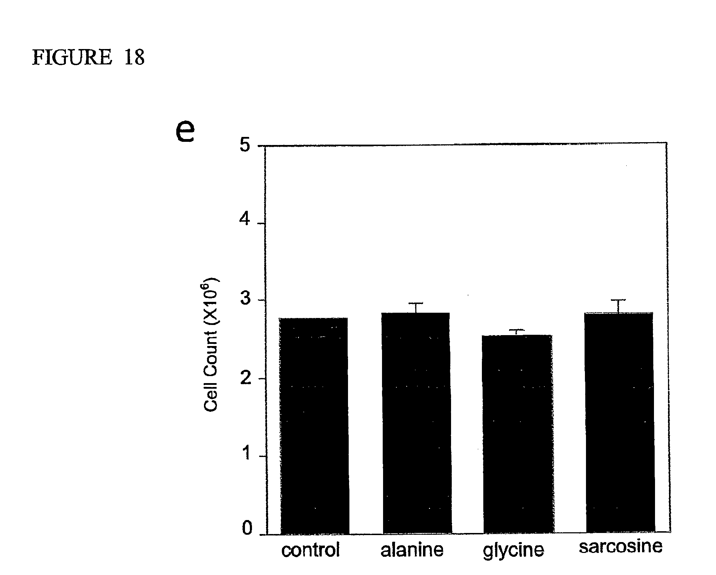

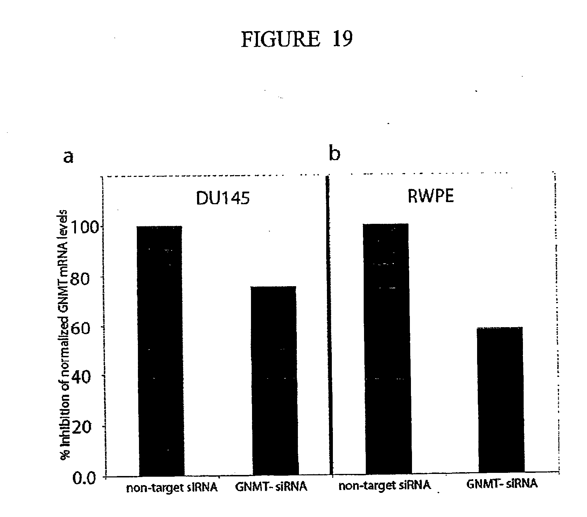

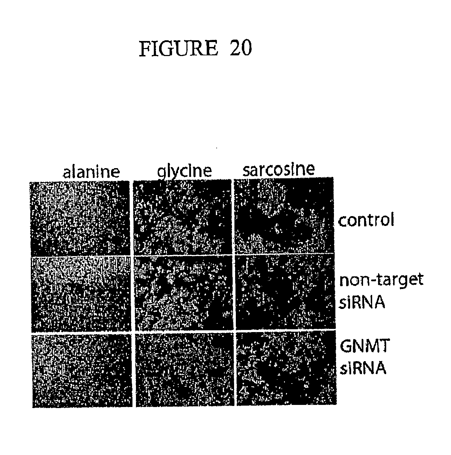

Taken together, the results indicate that sarcosine levels were associated with cancer cell invasion. To determine if sarcosine plays a role in this process, it was added directly to non-invasive benign prostate epithelial cells. Alanine (an isomer of sarcosine) was used as a control for these experiments. Intracellular sarcosine levels were highly elevated, as assessed by isotope dilution GC-MS, confirming sarcosine uptake by the cells (Fig. 17). The addition of sarcosine imparted an invasive phenotype to benign prostate epithelial cells (Fig. 4d, increased invasion upon sarcosine addition compared to control, 25 µM: 1.64-fold, p=0.065 and 50 µM: 2.57-fold, P <0.001). Similar results were obtained with primary prostate epithelial cells and benign immortalized breast epithelial cells. Exposure of the cells to the amino acids did not affect their ability to progress through the different stages of cell cycle (Fig. 18a-d) or affect proliferation (Fig. 18e). Notably, glycine (the precursor of sarcosine) also induced invasion in these cells, although to a lesser degree than sarcosine (Fig. 4d). The present invention is not limited to a particular mechanism. Indeed, an understanding of the mechanism is not necessary to practice the present invention. Nonetheless, it is contemplated that this indicated that glycine was being converted to sarcosine by the cell thus leading to invasion. To test this hypothesis, we blocked the conversion of glycine to sarcosine using RNA interference-mediated knock-down of glycine-N-methyl transferase (GNMT) (Takata et al., Biochemistry 42, 8394-8402 (2003)), the enzyme responsible for converting glycine to sarcosine, in invasive DU145 prostate cancer cells (Fig. 19). GNMT knockdown resulted in a significant reduction in invasion (P= 0.0073, t-test) with a concomitant 3-fold decrease in the intracellular sarcosine levels compared to control non-target siRNA-transfected cells (Fig 4e, 10.2 vs 31.9 fmoles/million cells). In a similar knockdown experiment performed in benign prostate epithelial cells (Fig. 19, RWPE), it was demonstrated that attenuation of GNMT did not affect the ability of exogenous sarcosine to induce invasion (Fig. 4f and Fig. 20 a,b, mean ±SEM for sarcosine addition, 0.64 ± 0.07 vs 0.65 ± 0.05, for GNMT knockdown vs control non-target siRNA-transfected cells). In this case, the ability of exogenous glycine to induce invasion was significantly hampered (Fig. 4f and Fig. 20 a,b, mean ±SEM for glycine addition, 0.20 ± 0.03 vs 0.46 ± 0.04, for GNMT knockdown vs control non-target siRNA-transfected cells, P=0.0082). These findings substantiate the role of sarcosine in mediating tumor invasion and may provide a biological explanation for why it is elevated in invasive prostate cancer.

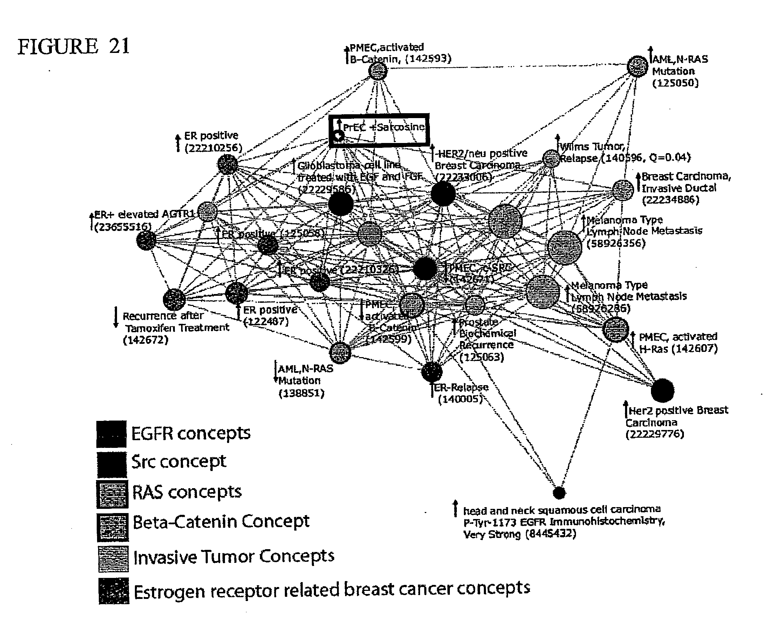

To determine the pathways that sarcosine activates in order to mediate invasion, gene expression analysis of sarcosine-treated prostate epithelial cells was compared to alanine-treated cells. Oncomine Concepts was used to evaluate whether the genes induced by sarcosine map to other molecular concepts (Fig. 21 and Table 8). Concepts of interest that were found to be significantly associated with sarcosine-induced genes included: (1) genes associated with estrogen receptor (ER) positive breast tumors, (2) genes associated with metastatic or aggressive variants of melanoma, and (3) genes associated with EGF receptor pathway activation in tumors).

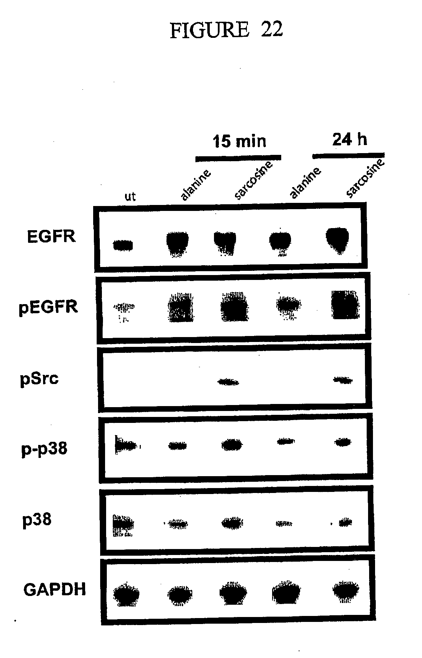

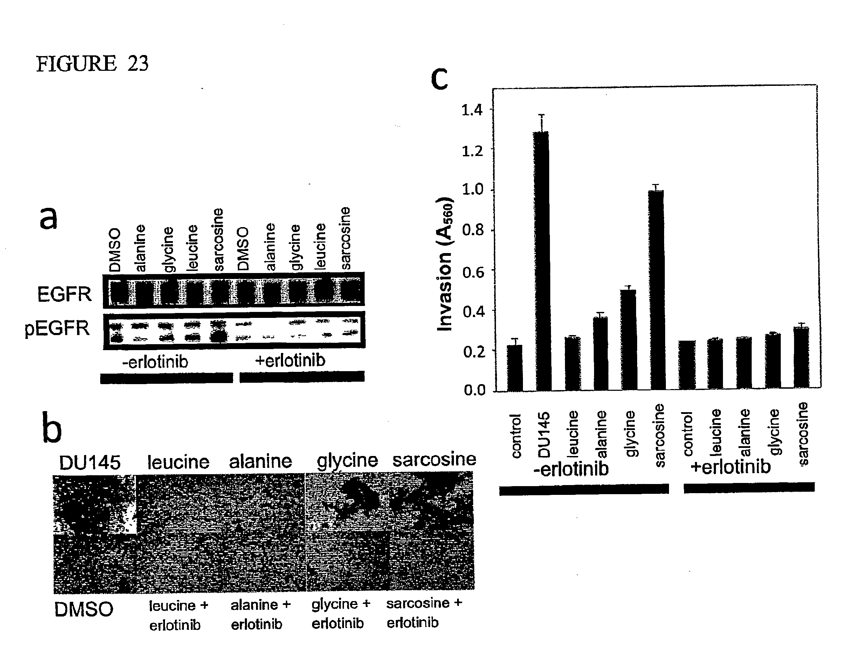

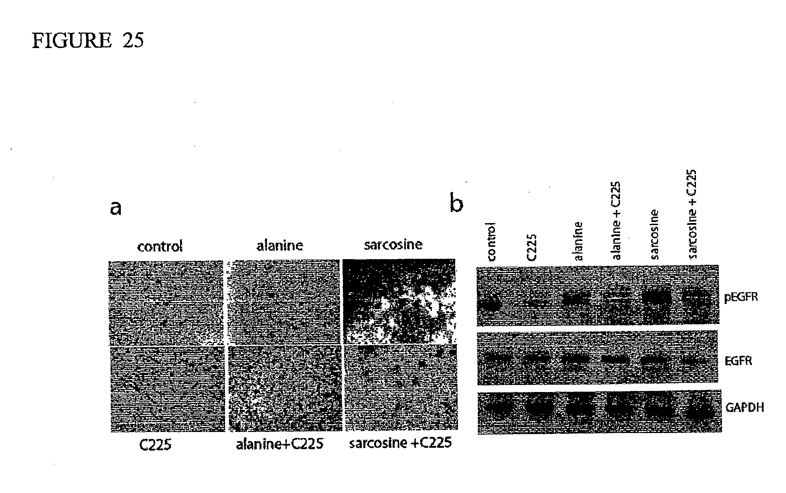

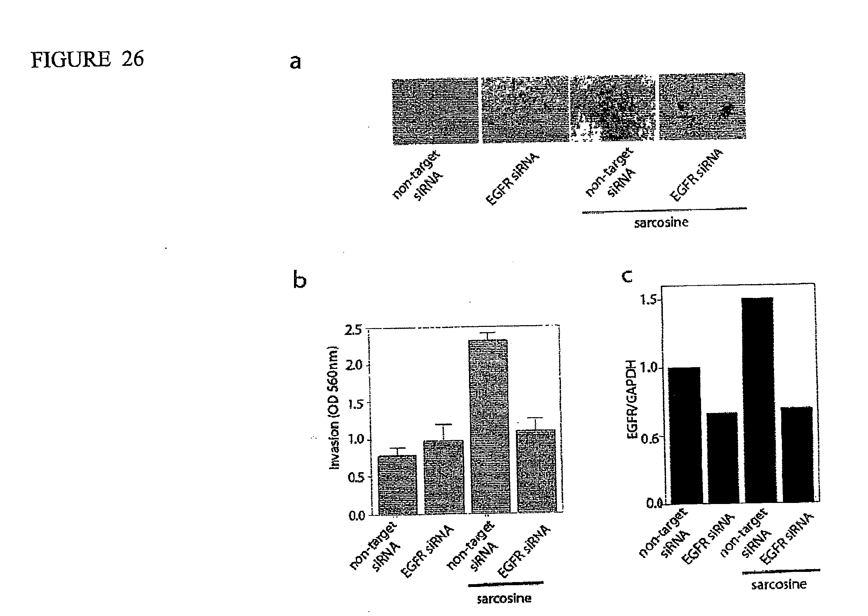

As the EGFR pathway and a number of its downstream mediators, including src and p38MAPK, have been implicated in ER positive breast cancer (Gross and Yee, Breast Cancer Res 4, 62-64 (2002); Lazennec et al., Endocrinology 142, 4120-4130 (2001); Rakovic et al., Arch Oncol 14, 146-150 (2006)) and invasive melanoma (Fagiani et al., Cancer Res 67, 3064-3073 (2007)), this pathway was examined in the context of sarcosine-induced cell invasion. Immunoblot analyses confirmed a time-dependent increase in EGFR (Fig.4g) and src phosphorylation (Fig. 22) in sarcosine-treated prostate epithelial cells (PrEC) compared to alanine-treated controls. Concordant with this was the finding of phosphorylation of p38MAPK in these samples (Fig. 22). It was reported that p38MAPK played a significant role in EGFR-Src-mediated invasion (Park et al., Cancer Res 66, 8511-8519 (2006); Hiscox et al., Clin Exp Metastasis 24, 157-167 (2007); Hiscox et al., Breast Cancer Res Treat 97, 263-274 (2006)). Also total EGFR levels were elevated upon treatment with alanine or sarcosine. The invasion induced by sarcosine was decreased by 70% (P = 0.0003) upon pre-treatment of PrEC cells with 10 µM concentration of erlotinib, a small molecule inhibitor of EGFR56-58 (Figs. 4h and Fig. 23,a-c). Similar attenuation of sarcosine-induced invasion was also seen in the immortalized prostate epithelial cells RWPE (t-test: P = 0.00007, See Fig. 26). This observation was further strengthened using both antibody-mediated inhibition of EGFRactivity and siRNA-mediated knock-down of receptor levels. Specifically, 50 µg/ml of C225 completely blocked sarcosine induced invasion in RWPE (Fig. 4i and Figs. 25 a,b) and PrEC cells. Similar attenuation of sarcosine-induced invasion was obtained using siRNA-mediated knock-down of EGFR compared to non-target control (P= 0.0058, Fig. 25 a-c).

Changes in metabolic activity and cancer progression are highly interrelated events. Changes in the levels of sarcosine reflect the inherent changes in the biochemistry of the tumor as it develops and progresses to a more advanced state. This is evident from data described above where cancer progression has been shown to be associated with an increase in amino acid metabolism and methylation potential of the tumor. Furthermore, one of the factors leading to an increased methylation potential is the increase in levels of S-adenosyl methionine (SAM) and its pathway components during tumor progression. This translates into elevated levels of methylated metabolites like N-methyl-glycine (sarcosine), methyl-guanosine, methyladenosine (known markers of DNA methylation) etc. in tumors compared to their benign counterparts. Notably, one of the major pathways for sarcosine generation involves the transfer of the methyl group from SAM to glycine, a reaction catalyzed by glycine-N-methyl transferase (GNMT). Using siRNA directed against GNMT, it was shown that sarcosine generation is important for the cell invasion process. This supports the hypothesis that elevated levels of sarcosine are a result of a change in the tumor's metabolic activity that is closely associated with the process of tumor progression. Sarcosine produced from tumor progression-associated changes in metabolic activity, by itself promotes tumor invasion.

The data described herein shows that sarcosine levels are reflective of two important hallmarks associated with prostate cancer development; namely increased amino acid metabolism and enhanced methylation potential leading to epigenetic silencing. The former is evident from the metabolomic profiles of localized prostate cancer that show high levels of multiple amino acids. This is also well corroborated by gene expression studies (Tomlins et al., Nat Genet, 2007. 39(1): 41-51) that describe increased protein biosynthesis in indolent tumors. Increased methylation has been known to play a major role in epigenetic silencing. Increased levels of EZH2, a methyltransferase belonging to the polycomb complex, are found in aggressive prostate cancer and metastatic disease (Varambally et al., Nature, 2002.419(6907):624-9). The current study expands understanding in this realm by implicating tumor progression to be associated with elevated methylation potential. This is supported by the finding of elevated levels of S-adenosyl methionine (the major methylation currency of the cell) and its associated pathway components during prostate cancer progression. This is further reflected by elevated levels of methylated metabolites in the dataset. Included among these is the methylated derivative of glycine (i.e., sarcosine) that shows a progressive elevation in its levels from localized tumor to metastatic disease. Notably, one of the major pathways for sarcosine generation involves the methylation reaction wherein the enzyme glycine-N-methyltransferase catalyses the transfer of methyl groups from SAM to glycine (an essential amino acid). Thus elevated levels of sarcosine can be attributed to an increase in both amino acid levels (in this case glycine) and an increase in methylation, both of which form the hallmarks of prostate cancer progression.

This Example describes unbiased metabolomic profiling of prostate cancer tissues to shed light into the metabolic pathways and networks dysregulated during prostate cancer progression. The present invention is not limited to a particular mechanism. Indeed, an understanding of the mechanism is not necessary to practice the present invention. Nonetheless, it is contemplated that the dysregulation of the metabolome during tumor progression could result from a myriad of causes that include perturbation in activities of their regulatory enzymes, changes in nutrient access or waste clearance during tumor development/progression

**Table 1**

| **Characteristic** | | **Value ⁺** |
|---|---|---|
| **Benign: Benign adjacent prostate tissues from patients with prostate cancer** | | |
| No. of patients | | 16 |
| Age at biopsy (years) | | 56 ± 6.7 [40, 63] |
| Race | | |
| | White(non-Hispanic origin) | 12 (92.3%) |
| | Other | 1 (7.7%) |

| **PCA: Patients with clinically localized prostate cancer** | | |
|---|---|---|
| No. of patients | | 11 |
| Age at biopsy (years) | | 57 ± 7.7 [40, 63] |
| Sample Gleason Grade (minor + major) | | |
| | 3 + 3 | 3 (25%) |
| | 3 + 4 | 5 (41.7%) |
| | 4 + 3 | 3 (25%) |
| | 4 + 4 | 1 (8.3%) |
| Baseline PSA | | 10.4 ± 8.1 [2.4. 24.6] |
| Stage | | |
| | T2a | 3 (30%) |
| | T2b | 4 (40%) |
| | T3a | 2 (20%) |
| | T3b | 0 (0%) |
| | T4 | 1 (10%) |
| Race | | |
| | White (non-Hispnnic origin) (%) | 8 (80%) |
| | Other (%) | 2 (20%) |

| **Mets: Patients with metastatic prostate cancer.** | | |
|---|---|---|
| No. of patients | | 13 |
| Age at death (years) | | 66 ± 12.1 [40, 82] |
| Sample Location, | | |
| | Soft tissue | 4 (28.6%) |
| | Liver | 8 (57.1%) |
| | Rib | 1 (7.1%) |
| | Diaphragm | 1 (7.1%) |
| Race | | |
| | White (non-Hispanic origin) (%) | 13 (100%) |

**Table 2**

| Standard | Description | Purpose |
|---|---|---|
| MTRX | Large pool of human plasma maintained at Metabolon, characterized extensively | Assure all aspects of profiling process are within specifications |
| PRCS | Aliquot of ultra-pure water | Process blank to assess contribution to compound signals from process |
| SOLV | Aliquot of extraction solvents | Solvent blank used to segregate contamination sources in extraction |
| DS | Derivatization Standard | Assess variability of derivatization for GC/MS samples |
| IS | Internal Standard | Assess variability performance of instrument |
| RS | Recovery Standard | Assess variability, verify performance of extraction instrumentation |

**Table 3. List of named metabolites and isobars measured in benign, PCA and metastatic prostate cancer tissues using either liquid chromatography (LC) or gas phase chromatography (GC) coupled to mass spectrometry**

| **Mass spectrometry method used for identification** | **Biochemical** |
|---|---|
| GC | 1,5-anhydroglucitol (1,5-AG) |
| LC | 1-Methyladenosine (1mA) |
| GC | 2-Aminoadipate |
| LC | 2'-Deoxyuridine-5'-triphosphate (dUTP) |
| GC | 2-Hydroxybutyrate (AHB) |
| LC | 2-Hydroxybutyrate (AHB) |
| LC | 3-Methyl-2-oxopentanoate |
| LC | 3-Methylhistidine (1-Methylhistidine) |
| GC | 3-Phosphoglycerate |
| GC | 3,4-Dihydroxyphenylethyleneglycol (DOPEG) |
| LC | 4-Acetamidobutanoate |
| LC | 4-Guanidinobutanoate |
| LC | 4-Methyl-2-oxopentanoate |
| GC | 5-Hydroxyindoleacetate (5-HIA) |
| LC | 5-Hydroxytryptophan |
| LC | 5-Methylthioadenosine (MTA) |
| LC | 5-Sulfosalicylate |
| LC | 5,6-Dihydrothymine |
| GC | 5,6-Dihydrouracil |
| LC | 6-Phosphogluconate |
| LC | Acetylcarnitine (ALC; C2 AC) |
| GC | Aconitate |
| GC | Adenine |
| LC | Adenosine |
| LC | α-Ketoglutarate |
| GC | Alanine |
| LC | Alanylalanine |
| GC | Arachidonate (20:4n6) |
| LC | Argininosuccinate |
| GC | Ascorbate (Vitamin C) |
| GC | Asparagine |
| GC | Aspartate |
| LC | Assymetric Dimethylarginine (ADMA) |
| GC | α-Tocopherol |
| LC | Azelate (Nonanedioate) |
| GC | ß-Alanine |
| GC | ß-aminoisobutyrate |
| GC | ß-Hydroxybutyrate (BHBA) |
| LC | Bicine |
| LC | Biliverdin |
| LC | Biotin |
| LC | Bradykinin |
| GC | Cadaverine |
| LC | Caffeine |
| LC | Carnitine |
| LC | Catechol |
| GC | Cholesterol |
| LC | Ciliatine (2-Aminoethylphosphonate) |
| GC | Citrate |
| GC | Citrulline |
| LC | Creatinine |
| GC | Cystathionine |
| GC | Cysteine |
| LC | Cytidine |
| LC | Cytidine monophosphate (CMP) |
| LC | Deoxyuridine |
| LC | Dihydroxyacetonephosphate (DHAP) |
| GC | Dimethylbenzimidazole |
| GC | Erythritol |
| LC | Ethylmalonate |
| GC | Fructose |
| GC | Fructose-6-phosphate |
| GC | Fumarate (trans-Butenedioate) |
| GC | Glucose |
| LC | γ-Glutamylcysteine |
| LC | γ-Glutamylglutamine |
| GC | Glutamate |
| GC | Glutamine |
| LC | Glutarate (Pentanedioate) |
| LC | Glutathione reduced (GSH) |
| GC | Glycerate |
| GC | Glycerol |
| GC | Glycerol-3-phosphate (G3P) |
| LC | Glycerophosphorylcholine (GPC) |
| GC | Glycine |
| LC | Glycocholate (GCA) |
| GC | Guanine |
| LC | Guanosine |
| GC | Heptadecanoate (Margarate; 17:0) |
| LC | Hexanoylcarnitine (C6 AC) |
| LC | Hippurate (Benzoylglycine) |
| LC | Histamine |
| GC | Histidine |
| LC | Histidinol |
| LC | Homocysteine |
| LC | Homoserine lactone |
| LC | Hydroxyphenylpyruvate |
| GC | Hydroxyproline |
| GC | Hypotaurine |
| LC | Hypoxanthine |
| GC | Imidazolelactate |
| LC | Indolelactate |
| LC | Inosine |
| LC | Indoxylsulfate |
| GC | Inositol-1-phosphate (I1P) |
| GC | Isoleucine |
| LC | Kynurenate |
| LC | Kynurenine |
| GC | Lactate |
| GC | Laurate (12:0) |
| GC | Leucine |
| GC | Linoleate (18:2n6) |
| GC | Lysine |
| GC | Malate |
| GC | Mannose |
| GC | Mannose-6-phosphate |
| LC | Methionine |
| LC | Methylglutarate |
| GC | myo-Inositol |
| GC | Myristate (14:0) |
| LC | N-6-trimethyllysine |
| LC | N-Acetylaspartate (NAA) |
| GC | N-Acetylgalactosamine |
| GC | N-Acetylglucosamine |
| GC | N-Acetylglucosaminylamine |
| LC | N-Acetylneuraminate |
| LC | N-Carbamoylaspartate |
| LC | Nicotinamide |
| LC | Nicotinamide adenine dinucleotide (NAD+) |
| LC | Nicotinamide Ribonucleotide (NMN) |
| GC | Octadecanoic acid |
| LC | Ofloxacin |
| GC | Oleate (18:1n9) |
| GC | Ornithine |
| LC | Orotidine-5'-phosphate |
| GC | Orthophosphate (Pi) |
| LC | Oxalate (Ethanedioate) |
| GC | Oxoproline |
| GC | Palmitate (16:0) |
| GC | Palmitoleate (16:1n7) |
| LC | Pantothenate |
| LC | Paraxanthine |
| LC | Phenylalanine |
| GC | Phosphoenolpyruvate (PEP |
| GC | Phosphoethanolamine |
| LC | Phosphoserine |
| GC | p-Hydroxyphenylacetate (HPA) |
| GC | p-Hydroxyphenyllactate (HPLA) |
| LC | Picolinate |
| LC | Pipecolate |
| GC | Proline |
| GC | Putrescine |
| LC | Pyridoxamine |
| GC | Pyrophosphate (PPi) |
| LC | Quinolinate |
| LC | Riboflavin (Vitamin B2) |
| GC | Ribose |
| LC | S-Adenosylhomocysteine (SAH) |
| LC | S-Adenosylmethionine (SAM) |
| GC | Sarcosine (N-Methylglycine) |
| GC | Serine |
| GC | Sorbitol |
| GC | Spermidine |
| GC | Spermine |
| LC | Suberate (Octanedioate) |
| GC | Succinate |
| GC | Sucrose/Maltose |
| LC | Tartarate |
| LC | Taurine |
| LC | trans-2, 3, 4-Trimethoxycinnamate |
| GC | Threonine |
| GC | Thymine |
| LC | Thyroxine |
| LC | Topiramate |
| LC | Tryptophan |
| LC | Tyrosine |
| LC | UDP-N-acetylmuraminate (UDP-MurNAc) |
| GC | Uracil |
| LC | Urate |
| GC | Urea |
| LC | Uridine |
| GC | Valine |
| LC | Xanthine |
| LC | Xanthosine |
| GC | Xylitol |

| | ISOBARS |
|---|---|
| LC | Isobar includes mannose, fructose, glucose, galactose |
| LC | Isobar includes arginine, N-alpha-acetyl-omithine |
| LC | Isobar includes D-fructose 1-phosphate, beta-D-fructose 6-phosphate |
| LC | Isobar includes D-saccharic acid, 1,5-anhydro-D-glucitol |
| LC | Isobar includes 2-aminoisobutyric acid,3-amino-isobutyrate |
| LC | Isobar includes gamma-aminobutyryl-L-histidine |
| LC | Isobar includes glutamic acid, O-acetyl-L-serine |
| LC | Isobar includes L-arabitol, adonitol |
| LC | Isobar includes L-threonine, L-allothreonine, L-homoserine |
| LC | Isobar includes R,S-hydroorotic acid, 5,6-dihydroorotic acid |
| LC | Isobar includes inositol 1-phosphate, mannose 6-phosphate |
| LC | Isobar includes maltotetraose, stachyose |
| LC | Isobar includes 1-kestose,maltotriose, melezitose |
| LC | Isobar includes N-acetyl-D-glucosamine, N-acetyl-D-mannosamine |
| LC | Isobar includes D-arabinose 5-phosphate, D-ribulose 5-phosphate |
| LC | Isobar includes Gluconic acid, DL-arabinose, D-ribose |
| LC | Isobar includes Maltotetraose, stachyose |
| LC | Isobar includes valine, betaine |
| LC | Isobar includes glycochenodeoxycholic acid/glycodeoxycholic acid |

**Table 4: List of 198 metabolites that make up the three-class-predictor derived from LOOCV**

| Metabolite | Permuted | LOOCV |
|---|---|---|
| | *P*-value | Frequency |
| 1,5-anhydroglucitol (1,5-AG) | <0.001 | 100.00% |
| 1-Methyladenosine (1mA) | <0.001 | 100.00% |
| 2-Hydroxybutyrate (AHB) | <0.001 | 100.00% |
| 4-Acetamidobutanoate | <0.001 | 100.00% |
| 5-Hydroxyindoleacetate (5-HIA) | 0.002 | 100.00% |
| Adenosine | <0.001 | 100.00% |
| Arachidonate (20:4n6) | 0.005 | 100.00% |
| Aspartate | 0.001 | 100.00% |
| Assymetric Dimethylarginine (ADMA) | 0.001 | 100.00% |
| β-aminoisobutyrate | <0.001 | 100.00% |
| Bicine | <0.001 | 100.00% |
| Biliverdin | 0.003 | 83.30% |
| Bradykinin hydroxyproline | <0.001 | |
| 100.00% | | |
| Caffeine | 0.007 | 97.60% |
| Catechol | <0.001 | 100.00% |
| Ciliatine (2-Aminoethylphosphonate) | <0.001 | 100.00% |
| Citrate | <0.001 | 100.00% |
| Creatinine | 0.008 | 85.70% |
| Cysteine | <0.001 | 100.00% |
| Dehydroepiandrosterone sulfate (DHEA-S) | <0.001 | 100.00% |
| Erythritol | <0.001 | 100.00% |
| Ethylmalonate | <0.001 | 100.00% |
| Fumarate (trans-Butenedioate) | 0.004 | 100.00% |
| γ-Glutamylglutamine | <0.001 | 100.00% |
| Glutamate | 0.01 | 85.70% |
| Glutathione reduced (GSH) | <0.001 | 100.00% |
| Glycerol | <0.001 | 100.00% |
| Glycerol-3-phosphate (G3P) | <0.001 | 100.00% |
| Glycine | 0.008 | 97.60% |
| Glycocholate (GCA) | 0.002 | 100.00% |
| Guanosine | <0.001 | 100.00% |
| Heptadecanoate (Margarate; 17:0) | <0.001 | 100.00% |
| Hexanoylcarnitine (C6 AC) | <0.001 | 100.00% |
| Histamine | 0.003 | 100.00% |
| Histidine | 0.002 | 100.00% |
| Homocysteine | <0.001 | 100.00% |
| Homoserine lactone | 0.001 | 100.00% |
| Hydroxyphenylpyruvate | <0.001 | 100.00% |
| Inosine | <0.001 | 100.00% |
| Inositol-1-phosphate (I1P) | <0.001 | 100.00% |
| Kynurenine | <0.001 | 100.00% |
| Laurate (12:0) | <0.001 | 100.00% |
| Leucine | <0.001 | 100.00% |
| Linoleate (18:2n6) | <0.001 | 100.00% |
| Methylglutarate | 0.002 | 100.00% |
| myo-Inositol | <0.001 | 100.00% |
| Myristate (14:0) | <0.001 | 100.00% |
| N-6-trimethyllysine | 0.001 | 100.00% |
| N-Acetylaspartate (NAA) | 0.003 | 100.00% |
| N-Acetylgalactosamine | <0.001 | 100.00% |
| N-Acetylglucosamine | <0.001 | 100.00% |
| N-Acetylglucosaminylamine | 0.002 | 100.00% |
| Nicotinamide | <0.001 | 100.00% |
| Nicotinamide adenine dinucleotide (NAD+) | 0.002 | 100.00% |
| Octadecanoic acid | <0.001 | 100.00% |
| Oleate (18:1n9) | <0.001 | 100.00% |
| Orthophosphate (Pi) | <0.001 | 100.00% |
| Palmitate (16:0) | <0.001 | 100.00% |
| Palmitoleate (16:1n7) | <0.001 | 100.00% |
| Pantothenate | 0.004 | 92.90% |
| Phosphoserine | <0.001 | 100.00% |
| p-Hydroxyphenyllactate (HPLA) | <0.001 | 100.00% |
| Pipecolate | <0.001 | 100.00% |
| Proline | <0.001 | 100.00% |
| Putrescine | <0.001 | 100.00% |
| Pyridoxamine | 0.001 | 95.20% |
| Riboflavin (Vitamin B2) | <0.001 | 100.00% |
| Ribose | <0.001 | 100.00% |
| S-Adenosylmethionine (SAM) | 0.001 | 100.00% |
| Sarcosine (N-Methylglycine) | <0.001 | 100.00% |
| Sorbitol | 0.001 | 100.00% |
| Spermidine | <0.001 | 100.00% |
| Spermine | <0.001 | 100.00% |
| Taurine | <0.001 | 100.00% |
| Thymine | <0.001 | 100.00% |
| Tryptophan | <0.001 | 100.00% |
| Uracil | <0.001 | 100.00% |
| Urate | <0.001 | 100.00% |
| Urea | <0.001 | 100.00% |
| Uridine | <0.001 | 100.00% |
| Valine | <0.001 | 100.00% |
| Xanthine | <0.001 | 100.00% |
| Xanthosine | <0.001 | 100.00% |

| **Isobars and Un-named** | | |
|---|---|---|
| Isobar includes mannose, fructose, glucose, galactose | 0.001 | 100.00% |
| Isobar includes arginine, N-alpha-acetyl-ornithine | 0.005 | 83.30% |
| Isobar includes D-saccharic acid,1,5-anhydro-D-glucitol | <0.001 | 100.00% |
| Isobar includes 2-aminoisobutyric acid,3-aminoisobutyrate | <0.001 | 100.00% |
| Isobar includes L-arabitol, adonitol | <0.001 | 100.00% |
| Isobar includes inositol 1-phosphate, mannose 6-phosphate | <0.001 | 100.00% |
| Isobar includes Maltotetraose, stachyose | 0.003 | 100.00% |
| X-1104 | <0.001 | 100.00% |
| X-1111 | <0.001 | 100.00% |
| X-1114 | 0.002 | 100.00% |
| X-1142 | 0.004 | 100.00% |
| X-1186 | 0.001 | 97.60% |
| X-1329 | <0.001 | 100.00% |
| X-1333 | 0.002 | 100.00% |
| X-1342 | 0.003 | 100.00% |
| X-1349 | <0.001 | 100.00% |
| X-1351 | <0.001 | 100.00% |
| X-1465 | <0.001 | 100.00% |
| X-1575 | 0.01 | 100.00% |
| X-1576 | <0.001 | 100.00% |
| X-1593 | 0.003 | 100.00% |
| X-1595 | <0.001 | 100.00% |
| X-1597 | 0.001 | 100.00% |
| X-1608 | 0.005 | 100.00% |
| X-1609 | 0.002 | 100.00% |
| X-1679 | <0.001 | 100.00% |
| X-1843 | <0.001 | 100.00% |
| X-1963 | <0.001 | 100.00% |
| X-1977 | <0.001 | 100.00% |
| X-1979 | 0.005 | 92.90% |
| X-2055 | 0.008 | 83.30% |
| X-2074 | <0.001 | 100.00% |
| X-2105 | 0.005 | 90.50% |
| X-2108 | 0.005 | 100.00% |
| X-2118 | <0.001 | 100.00% |
| X-2141 | 0.007 | 88.10% |
| X-2143 | 0.002 | 100.00% |
| X-2181 | <0.001 | 100.00% |
| X-2237 | 0.001 | 100.00% |
| X-2272 | <0.001 | 100.00% |
| X-2292 | <0.001 | 100.00% |
| X-2466 | <0.001 | 100.00% |
| X-2548 | 0.003 | 97.60% |
| X-2607 | 0.005 | 100.00% |
| X-2688 | 0.001 | 100.00% |
| X-2690 | <0.001 | 100.00% |
| X-2697 | 0.001 | 100.00% |
| X-2766 | <0.001 | 100.00% |
| X-2806 | <0.001 | 100.00% |
| X-2867 | <0.001 | 100.00% |
| X-2973 | <0.001 | 100.00% |
| X-3003 | 0.001 | 100.00% |
| X-3044 | 0.001 | 100.00% |
| X-3056 | <0.001 | 100.00% |
| X-3102 | <0.001 | 100.00% |
| X-3129 | <0.001 | 100.00% |
| X-3138 | <0.001 | 100.00% |
| X-3139 | <0.001 | 100.00% |
| X-3176 | <0.001 | 100.00% |
| X-3220 | 0.001 | 100.00% |
| X-3238 | <0.001 | 100.00% |
| X-3379 | <0.001 | 100.00% |
| X-3390 | <0.001 | 100.00% |
| X-3489 | 0.001 | 100.00% |
| X-3771 | <0.001 | 100.00% |
| X-3778 | <0.001 | 100.00% |
| X-3807 | <0.001 | 100.00% |
| X-3808 | <0.001 | 100.00% |
| X-3810 | <0.001 | 100.00% |
| X-3816 | <0.001 | 100.00% |
| X-3833 | 0.002 | 100.00% |
| X-3893 | <0.001 | 100.00% |
| X-3952 | 0.001 | 100.00% |
| X-3955 | <0.001 | 100.00% |
| X-3960 | <0.001 | 100.00% |
| X-3992 | <0.001 | 100.00% |
| X-3997 | 0.002 | 100.00% |
| X-4013 | <0.001 | 100.00% |
| X-4015 | <0.001 | 100.00% |
| X-4018 | <0.001 | 100.00% |
| X-4027 | <0.001 | 100.00% |
| X-4051 | <0.001 | 100.00% |
| X-4075 | <0.001 | 100.00% |
| X-4084 | <0.001 | 100.00% |
| X-4096 | <0.001 | 100.00% |
| X-4117 | 0.003 | 100.00% |
| X-4365 | <0.001 | 100.00% |
| X-4428 | 0.002 | 100.00% |
| X-4514 | <0.001 | 100.00% |
| X-4567 | 0.003 | 95.20% |
| X-4611 | <0.001 | 100.00% |
| X-4615 | <0.001 | 100.00% |
| X-4616 | 0.005 | 95.20% |
| X-4617 | 0.001 | 100.00% |
| X-4620 | <0.001 | 100.00% |
| X-4624 | 0.003 | 85.70% |
| X-4649 | <0.001 | 100.00% |
| X-4866 | 0.001 | 100.00% |
| X-4869 | <0.001 | 100.00% |
| X-5107 | 0.001 | 100.00% |
| X-5109 | 0.004 | 100.00% |
| X-5110 | 0.004 | 81.00% |
| X-5128 | <0.001 | 100.00% |
| X-5187 | <0.001 | 100.00% |
| X-5207 | <0.001 | 100.00% |
| X-5208 | <0.001 | 100.00% |
| X-5209 | <0.001 | 100.00% |
| X-5210 | <0.001 | 100.00% |
| X-5212 | <0.001 | 100.00% |
| X-5214 | 0.003 | 100.00% |
| X-5215 | <0.001 | 100.00% |
| X-5229 | 0.003 | 100.00% |
| X-5232 | 0.002 | 97.60% |

**Table 5**

| **Tissue type** | **Number of samples** | **Number of patients** |
|---|---|---|
| Benign adjacent prostate tissue | 25 | 20 |
| Local tumor (PCA) tissue | 36 | 36 |
| Metastatie tumor tissue | 28 | 19 |
| Metastasis site: adrenal | 1 | 1 |
| Liver | 14 | 12 |
| Lung | 1 | 1 |
| Mesentary | 2 | 1 |
| Pancreas | 1 | 1 |
| Periaostic lymph | 3 | 2 |
| Soft tissue | 2 | 2 |
| Unknown | 4 | 4 |

**Table 6**

| **Characteristic** | **Urine Supernatant Samples (n=110)** | **Urine Sediment Samples (n=93)** |
|---|---|---|
| | | |
| **Biopsy Negative** | | |
| No. of patients | 51* | 44** |
| Age at biopsy (years) | 63.4 ± 9.9 [4.2, 82] | 60.7 + 9.6 [40,77] |
| Baseline PSA (ng/ml) | 6.1 ± 3.8 [0.8, 20.8] | 5.3 + 2.3 [1.1, 10.0] |
| | | |

| **Biopsy Positive** | | |
|---|---|---|
| No. of patients | 59 # | 49 ## |
| Age at biopsy (years) | 68.0 ± 8.9 [51, 85] | 63.8 + 9 . [47, 81] |
| Baseline PSA (ng/ml) | 11.9 ± 19.6 [2.7, 111] | 11.4 + 23.5 [2.7, 111.0] |
| Gleason Sam | | |
| 6 | 25 (42.4%) | 19 (41.33%) |
| 7 | 25 (42.4%) | 20(43.5%) |
| 8 | 3 (5.1%) | 2 (4.4%) |
| 9 | 5 (8.5%) | 5(10.9%) |
| 10 | 1 (1.7%) | 0 (0%) |
| Maximum tumor diameter | 1.7 ± 1.0 [0.5, 4.3] | |
| Gland weight | 49.1 + 12.2 [28.2, 75.1] | 49.9+ 14.6 [28.2, 77.6] |

| **Characteristic** | **Urine Supernatant Samples** | **Urine Sediment Samples** |
|---|---|---|
| **Correlation with Sarcosine (log2)** | | |
| Age | 0.18 | 0.19 |
| PSA (log) | 0.22 | -0.06 |
| Gland weight | -0.09 | -0.17 |

| **Two-tailed Wilcoxon rauk-sum test of sarcosine (log2)** | | |
|---|---|---|
| Diagnosis (neg v pos) | P=0.0025 | P=0.0004 |
| Gleason (6v 7+) | P=0.5756 | P=0.6880 |

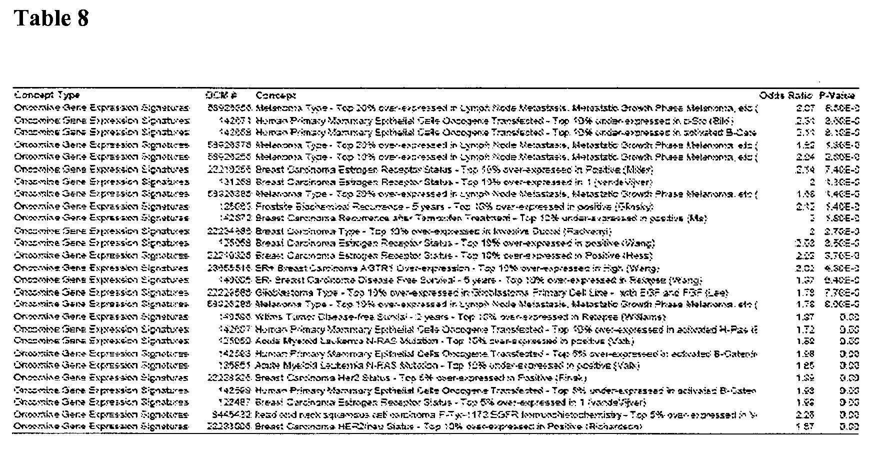

Table 9 below includes analytical characteristics of each of the unnamed metabolites listed in Table 4 above. The table includes, for each listed Metabolite 'X', the compound identifier (COMP_ID), retention time (RT), retention index (RI), mass, quant mass, and polarity obtained using the analytical methods described above. "Mass" refers to the mass of the C12 isotope of the parent ion used in quantification of the compound. The values for "Quant Mass" give an indication of the analytical method used for quantification: "Y" indicates GC-MS and "1" indicates LC-MS. "Polarity" indicates the polarity of the quantitative ion as being either positive (+) or negative (-).

**Table 9. Analytical characteristics of unnamed metabolites.**

| **COMP_ID** | **Metabolite** | **RT** | **RI** | **MASS** | **QUANT _MASS** | **Polarity** |
|---|---|---|---|---|---|---|
| 5669 | X-1104 | 2.43 | 2410 | 201 | 1 | - |
| 5689 | X-1111 | 2.69 | 2700 | 148.1 | 1 | + |
| 5702 | X-1114 | 2.19 | 2198 | 104.1 | 1 | + |
| 5765 | X-1142 | 8.54 | 8739 | 163 | 1 | - |
| 5797 | X-1186 | 8.83 | 9000 | 529.6 | 1 | + |
| 6379 | X-1329 | 2.69 | 2791 | 210.1 | 1 | + |
| 6396 | X-1333 | 3.05 | 3794 | 321.9 | 1 | + |
| 6413 | X-1342 | 9.04 | 9459.4 | 265.2 | 1 | + |
| 6437 | X-1349 | 3.5 | 3876 | 323.9 | 1 | + |
| 6443 | X-1351 | 1.77 | 1936.5 | 177.9 | 1 | + |
| 6787 | X-1465 | 3.45 | 3600 | 162.1 | 1 | + |
| 6997 | X-1575 | 2.25 | 2243.5 | 219.1 | 1 | + |
| 7002 | X-1576 | 2.51 | 2530 | 247.1 | 1 | + |
| 7018 | X-1593 | 2.67 | 2690 | 395.9 | 1 | - |
| 7023 | X-1595 | 3.14 | 3400 | 290.1 | 1 | + |
| 7029 | X-1597 | 3.66 | 4100 | 265.9 | 1 | + |
| 7073 | X-1608 | 8.08 | 8253 | 348.1 | 1 | - |
| 7081 | X-1609 | 8.31 | 8529 | 378 | 1 | + |
| 7272 | X-1679 | 8.52 | 8705.8 | 283.1 | 1 | - |
| 7672 | X-1843 | 3.25 | 3295 | 288.7 | 1 | - |
| 8107 | X-1963 | 13.15 | 13550.8 | 464.1 | 1 | + |
| 8189 | X-1977 | 3.56 | 4060 | 260.9 | 1 | + |
| 8196 | X-1979 | 1.52 | 1690.3 | 199 | 1 | - |
| 8669 | X-2055 | 1.37 | 1502 | 269.9 | 1 | + |
| 8796 | X-2074 | 2.24 | 2380.9 | 280.1 | 1 | + |
| 8991 | X-2105 | 8.15 | 8442 | 433.6 | 1 | + |
| 9007 | X-2108 | 8.76 | 8800 | 277.1 | 1 | + |
| 9038 | X-2118 | 13.1 | 13367.8 | 547.1 | 1 | + |
| 9137 | X-2141 | 9.39 | 9605 | 409.1 | 1 | + |
| 9143 | X-2143 | 10.11 | 10327 | 585.1 | 1 | + |
| 9458 | X-2181 | 8.37 | 8715.5 | 298 | 1 | + |
| 10047 | X-2237 | 10.14 | 10039 | 453.1 | 1 | + |
| 10286 | X-2272 | 7.96 | 8377 | 189.1 | 1 | - |
| 10424 | X-2292 | 2.4 | 2900 | 343.9 | 1 | - |
| 10774 | X-2466 | 9.19 | 8760 | 624.8 | 1 | + |
| 10850 | X-2548 | 5.97 | 6430 | 202.9 | 1 | - |
| 11173 | X-2607 | 10.01 | 10354 | 578.2 | 1 | + |
| 11222 | X-2688 | 1.42 | 1614 | 182 | 1 | - |
| 11235 | X-2690 | 1.62 | 1786.2 | 441.1 | 1 | + |
| 11262 | X-2697 | 3.77 | 4241.2 | 209.9 | 1 | + |
| 11544 | X-2766 | 8.09 | 8395 | 397 | 1 | + |
| 11770 | X-2806 | 1.38 | 1491 | 185.1 | 1 | + |
| 12298 | X-2867 | 9.65 | 9908 | 235.3 | 1 | + |
| 12593 | X-2973 | 4.74 | 1213.4 | 281 | Y | + |
| 12603 | X-2980 | 5.17 | 1261.3 | 266.1 | Y | + |
| 12626 | X-3003 | 6.79 | 1446.6 | 218.1 | Y | + |
| 12682 | X-3044 | 1.52 | 1615.3 | 150.1 | 1 | + |
| 12720 | X-3056 | 9.19 | 9432 | 185.2 | 1 | + |
| 12770 | X-3090 | 11.31 | 1954.7 | 243.1 | Y | + |
| 12784 | X-3102 | 11.99 | 2028.2 | 217.1 | Y | + |
| 12785 | X-3103 | 12.09 | 2039.2 | 290.1 | Y | + |
| 12912 | X-3129 | 8.8 | 9012 | 337.1 | 1 | + |
| 13018 | X-3138 | 8.63 | 8749 | 229.2 | 1 | + |
| 13024 | X-3139 | 8.82 | 8934.5 | 176.1 | 1 | + |
| 13179 | X-3176 | 1.42 | 1750 | 132 | 1 | + |
| 13262 | X-3220 | 3.73 | 4044.1 | 233.1 | 1 | + |
| 13328 | X-3238 | 11.77 | 11827.4 | 220 | 1 | + |
| 13810 | X-3379 | 1.51 | 1539 | 414.1 | 1 | + |
| 13853 | X-3390 | 8.14 | 8800 | 595.9 | 1 | - |
| 14368 | X-3489 | 3.26 | 3840 | 226 | 1 | + |
| 15057 | X-3771 | 1.68 | 1761 | 227 | 1 | - |
| 15098 | X-3778 | 7.37 | 7200 | 307.3 | 1 | + |
| 15211 | X-3807 | 3 | 3398.5 | 245 | 1 | + |
| 15213 | X-3808 | 3.28 | 3719 | 288.8 | 1 | - |
| 15215 | X-3810 | 3.74 | 4500 | 188.1 | 1 | - |
| 15227 | X-3816 | 4.16 | 5310 | 173.1 | 1 | - |
| 15255 | X-3833 | 8.81 | 9100 | 261.1 | 1 | - |
| 15374 | X-3893 | 3.26 | 3724.5 | 409 | 1 | + |
| 15532 | X-3952 | 8.7 | 9150 | 297.2 | 1 | + |
| 15535 | X-3955 | 8.68 | 8951.7 | 357.1 | 1 | - |
| 15571 | X-3960 | 8.49 | 8744.1 | 417.1 | 1 | + |
| 16002 | X-3992 | 1.4 | 1600 | 129.2 | 1 | - |
| 16027 | X-3997 | 2.87 | 2876 | 564.9 | 1 | - |
| 16057 | X-4013 | 8.05 | 8399.5 | 547 | 1 | - |
| 16062 | X-4015 | 7.37 | 1498.4 | 160 | Y | + |
| 16062 | X-4015 | 7.37 | 1497.8 | 160 | Y | + |
| 16068 | X-4018 | 8.35 | 8589.3 | 664 | 1 | - |
| 16082 | X-4027 | 8.67 | 1650.2 | 274.1 | Y | + |
| 16116 | X-4051 | 11.56 | 1970.2 | 357.1 | Y | + |
| 16131 | X-4075 | 13.27 | 2171.5 | 103 | Y | + |
| 16143 | X-4084 | 14.98 | 2393.9 | 441.3 | Y | + |
| 16186 | X-4096 | 8.6 | 8763.6 | 318.2 | 1 | + |
| 16219 | X-4117 | 14.7 | 15040.2 | 260.3 | 1 | + |
| 16666 | X-4365 | 11.05 | 1892.9 | 204 | Y | + |
| 16705 | X-4428 | 7.92 | 8236.5 | 229.2 | 1 | + |
| 16821 | X-4498 | 7.06 | 1434.9 | 103 | Y | + |
| 16822 | X-4499 | 7.22 | 1453 | 189 | Y | + |
| 16829 | X-4503 | 8.39 | 1589.3 | 227.2 | Y | + |
| 16831 | X-4504 | 8.46 | 1597.1 | 244.1 | Y | + |
| 16837 | X-4507 | 8.89 | 1644.9 | 245 | Y | + |
| 16853 | X-4514 | 10.31 | 1812.3 | 342.2 | Y | + |
| 16866 | X-4523 | 12.46 | 2048.1 | 258.1 | Y | + |
| 16925 | X-4567 | 3.5 | 3910.5 | 203.2 | 1 | + |
| 16984 | X-4599 | 7.42 | 1471.1 | 113 | Y | + |
| 17028 | X-4611 | 8.07 | 1546.6 | 292.1 | Y | + |
| 17043 | X-4615 | 7.93 | 8250 | 222.1 | 1 | + |
| 17044 | X-4616 | 8.12 | 8427 | 276.2 | 1 | + |
| 17048 | X-4617 | 8.39 | 8588 | 241.3 | 1 | + |
| 17050 | X-4618 | 8.93 | 1651.1 | 349.2 | Y | + |
| 17053 | X-4620 | 8.82 | 9001 | 312.1 | 1 | + |
| 17064 | X-4624 | 10.01 | 1779.1 | 342.2 | Y | + |
| 17064 | X-4624 | 10.01 | 1779.2 | 342.2 | Y | + |
| 17072 | X-4628 | 10.11 | 1786.4 | 267.1 | Y | + |
| 17074 | X-4629 | 10.29 | 1806.9 | 274.1 | Y | + |
| 17086 | X-4637 | 11.95 | 1988.1 | 193 | Y | + |
| 17088 | X-4639 | 12.87 | 2092.4 | 156.1 | Y | + |
| 17130 | X-4649 | 5.33 | 5997 | 164.1 | 1 | + |
| 17444 | X-4866 | 9.18 | 9069 | 506.7 | 1 | + |
| 17454 | X-4869 | 10.25 | 10112.8 | 534.5 | 1 | + |
| 17844 | X-5107 | 11.87 | 11986 | 516.7 | 1 | + |
| 17846 | X-5109 | 12.12 | 12248.5 | 560.7 | 1 | + |
| 17847 | X-5110 | 12.24 | 12350.5 | 582.6 | 1 | + |
| 17862 | X-5128 | 3.12 | 3462.8 | 558 | 1 | - |
| 17919 | X-5187 | 3.53 | 3985.5 | 489.1 | 1 | + |
| 17960 | X-5207 | 7.41 | 1493.6 | 151 | Y | + |
| 17962 | X-5208 | 7.83 | 1542.3 | 84 | 1 | |
| 17969 | X-5209 | 8.1 | 1573.6 | 218.2 | Y | + |
| 17971 | X-5210 | 8.47 | 1616.4 | 254.1 | Y | + |
| 17977 | X-5212 | 8.88 | 1665.1 | 306.1 | Y | + |
| 17979 | X-5214 | 11.54 | 1960 | 117 | Y | + |
| 17980 | X-5215 | 11.98 | 2008 | 163 | Y | + |
| 17989 | X-5229 | 7.13 | 1461.6 | 211.1 | Y | + |
| 18017 | X-5232 | 12.19 | 2031.5 | 134 | Y | + |
| 18232 | X-5403 | 5.92 | 1301.2 | 319 | Y | + |
| 18251 | X-5409 | 7.46 | 1477.9 | 128 | Y | + |
| 18253 | X-5410 | 7.53 | 1484 | 259.1 | Y | + |
| 18257 | X-5412 | 7.98 | 1538.7 | 128.9 | Y | + |
| 18264 | X-5414 | 8.59 | 1608.2 | 217.1 | Y | + |
| 18265 | X-5415 | 8.83 | 1639.9 | 205 | Y | + |
| 18271 | X-5418 | 9.01 | 1659.7 | 117 | Y | + |
| 18272 | X-5419 | 9.05 | 1664.1 | 349.2 | Y | + |
| 18273 | X-5420 | 9.09 | 1669 | 417.1 | Y | + |
| 18307 | X-5431 | 11.53 | 1946.5 | 453.2 | Y | + |
| 18309 | X-5433 | 11.6 | 1953.5 | 294 | Y | + |
| 18316 | X-5437 | 12.17 | 2017.3 | 337.1 | Y | + |
| 18388 | X-5491 | 8.3 | 1575.3 | 129 | Y | + |
| 18390 | X-5492 | 8.39 | 1584.6 | 122 | Y | + |
| 18419 | X-5506 | 8.66 | 1616 | 334.1 | Y | + |
| 18430 | X-5511 | 9.73 | 1745 | 128.9 | Y | + |
| 18438 | X-5518 | 11.94 | 1991.3 | 331.1 | Y | + |
| 18442 | X-5522 | 13.05 | 2119.8 | 259 | Y | + |
| 19954 | X-6906 | 9.13 | 1675.7 | 175 | Y | + |
| 19960 | X-6912 | 9.5 | 1721.6 | 292.1 | Y | + |
| 19965 | X-6928 | 10.04 | 1785.5 | 117 | Y | + |
| 19969 | X-6931 | 10.35 | 1819.6 | 267.1 | Y | + |
| 19973 | X-6946 | 10.76 | 1865 | 281.1 | Y | + |
| 19984 | X-6956 | 11.65 | 1961 | 323.1 | Y | + |
| 19990 | X-6962 | 11.9 | 1986.5 | 267.1 | Y | + |
| 19997 | X-6969 | 12.36 | 2040 | 584.4 | Y | + |
| 20014 | X-6985 | 13.75 | 2209.4 | 277.1 | Y | + |
| 20020 | X-6991 | 13.97 | 2238.8 | 292.1 | Y | + |
| 22308 | X-8886 | 8.24 | 1589.9 | 198.1 | Y | + |
| 22320 | X-8889 | 8.62 | 1634.3 | 521.2 | Y | + |
| 22494 | X-8994 | 10.76 | 1878.7 | 447.2 | Y | + |
| 22548 | X-9026 | 8.45 | 1599.5 | 156 | Y | + |
| 22570 | X-9033 | 9.61 | 1735.6 | 217.1 | Y | + |
| 22881 | X-9287 | 9.1 | 1656.8 | 271 | Y | + |
| 24074 | X-9706 | 4.39 | 1107 | 190 | Y | + |
| 24076 | X-9726 | 4.91 | 1167.5 | 245 | Y | + |
| 24332 | X-10128 | 8.8 | 1613.2 | 231 | Y | + |
| 24469 | X-10266 | 9.17 | 1655 | 328 | Y | + |
| 25401 | X-10359 | 9.85 | 1734.3 | 292.1 | Y | + |
| 25402 | X-10360 | 10.23 | 1781.9 | 204 | Y | + |
| 25449 | X-10385 | 13.25 | 2128.9 | 254 | Y | + |
| 25607 | X-10437 | 8.43 | 1596.4 | 331.1 | Y | + |
| 27883 | X-10604 | 10.7 | 1854.2 | 173 | Y | + |
| 27884 | X-10605 | 11.07 | 1892.6 | 173 | Y | + |
| 30275 | X-10738 | 11.67 | 1986.1 | 382.1 | Y | + |
| 30276 | X-10739 | 11.79 | 1999 | 469.2 | Y | + |
| 31022 | X-10831 | 10.33 | 1818.4 | 257.1 | Y | + |
| 31041 | X-10835 | 10.7 | 1858.4 | 358.2 | Y | + |
| 31053 | X-10841 | 11.6 | 1952 | 257.1 | Y | + |
| 31203 | X-10850 | 10.25 | 1817 | 179 | Y | + |
| 31489 | X-10914 | 6.82 | 1389 | 241.1 | Y | + |
| 31750 | X-11011 | 10.07 | 1777 | 287.1 | Y | + |
| 31751 | X-11012 | 10.48 | 1825 | 175 | Y | + |
| 31754 | X-11015 | 12.67 | 2071 | 285 | Y | + |
| 31757 | X-11018 | 13.68 | 2200 | 599.7 | Y | + |
| 32026 | X-11072 | 10.15 | 1802 | 287.2 | Y | + |
| 32120 | X-11096 | 8.4 | 1596 | 103.1 | Y | + |
| 32127 | X-11103 | 9.48 | 1732 | 217.1 | Y | + |
| 32550 | X-02272_201 | 1.97 | 1958 | 189 | 1 | - |
| 32557 | X-06126_201 | 2.69 | 2684 | 203.1 | 1 | - |
| 32562 | X-11245 | 3.91 | 3902 | 238.3 | 1 | - |
| 32578 | X-11261 | 3.69 | 3600 | 286.2 | 1 | + |
| 32599 | X-11282 | 4.77 | 4763 | 254.8 | 1 | - |
| 32631 | X-11314 | 0.64 | 634 | 243 | 1 | + |
| 32649 | X-11332 | 0.92 | 935 | 212.1 | 1 | + |
| 32650 | X-11333 | 1 | 1019 | 212.1 | 1 | + |
| 32651 | X-11334 | 0.96 | 982 | 259.1 | 1 | + |
| 32652 | X-11335 | 0.97 | 991 | 229.2 | 1 | + |
| 32653 | X-03249 200 | 1.03 | 1049 | 141.1 | 1 | + |
| 32664 | X-11347 | 2.6 | 2641 | 413 | 1 | + |
| 32665 | X-11348_200 | 2.62 | 2664 | 160.1 | 1 | + |
| 32669 | X-11352 | 0.86 | 879 | 189.2 | 1 | + |
| 32672 | X-02546_200 | 0.75 | 764 | 129.2 | 1 | + |
| 32674 | X-11357 | 1.71 | 1750 | 232.1 | 1 | + |
| 32675 | X-03951_200 | 1.87 | 1912 | 367.1 | 1 | + |
| 32709 | X-03056_200 | 2.21 | 2264 | 185.2 | 1 | + |
| 32714 | X-11397 | 2.59 | 2634 | 300.1 | 1 | + |
| 32735 | X-01911 200 | 4.26 | 4275 | 464.1 | 1 | + |
| 32738 | X-11421 | 4.54 | 4575 | 314.2 | 1 | + |
| 32740 | X-11423 | 1.05 | 1038 | 260.1 | 1 | - |
| 32754 | X-11437 | 2.89 | 2888 | 231 | 1 | - |
| 32761 | X-11444 | 3.99 | 3983 | 541.2 | 1 | - |
| 32767 | X-11450 | 4.11 | 4103 | 224.2 | 1 | - |
| 32769 | X-11452 | 4.12 | 4109 | 352.1 | 1 | - |
| 32781 | X-11464 | 2.96 | 3014 | 402.4 | 1 | + |
| 32787 | X-11470 | 4.16 | 4151 | 525.2 | 1 | - |
| 32792 | X-11475 | 4.25 | 4240 | 383.2 | 1 | - |
| 32807 | X-11490 | 4.77 | 4762 | 279.8 | 1 | - |
| 32827 | X-11510 | 3.92 | 3925 | 385.2 | 1 | - |
| 32878 | X-11561 | 1.26 | 1252 | 267.1 | 1 | - |
| 32881 | X-11564 | 1.2 | 1188 | 177.1 | 1 | - |
| 32910 | X-11593 | 0.79 | 790 | 189.2 | 1 | - |
| 32937 | X-03951_201 | 1.77 | 1773 | 365.2 | 1 | - |
| 32957 | X-11640 | 3.78 | 3776 | 377.1 | 1 | - |
| 32978 | X-11656 | 0.6 | 612 | 227 | 1 | + |
| 32996 | X-11668 | 1.37 | 1367 | 215.2 | 1 | - |
| 33009 | X-01981_200 | 1.19 | 1199 | 158.2 | 1 | + |
| 33014 | X-10457_200 | 1.47 | 1515 | 261.2 | 1 | + |
| 33031 | X-11687 | 2.16 | 2182 | 384.1 | 1 | + |
| 33033 | X-11689 | 3.11 | 3142 | 432.2 | 1 | + |
| 33090 | X-11745 | 8.37 | 1581 | 311.1 | Y | + |
| 33094 | X-11749 | 9.12 | 1668 | 218.2 | Y | + |
| 33100 | X-11755 | 10.39 | 1820 | 318.2 | Y | + |
| 33103 | X-11758 | 11.3 | 1917 | 397.2 | Y | + |
| 33106 | X-11761 | 11.97 | 1991 | 469.4 | Y | + |
| 33127 | X-11782 | 13.71 | 2205 | 294.2 | Y | + |
| 33171 | X-11826 | 1.48 | 1489 | 194.1 | 1 | - |
| 33188 | X-11843 | 2.69 | 2710 | 230.1 | 1 | - |
| 33195 | X-11850 | 3.2 | 3228 | 226.1 | 1 | - |
| 33280 | X-11935 | 1.88 | 1945 | 298.1 | 1 | + |
| 33281 | X-11936 | 2.07 | 2150 | 312.1 | 1 | + |
| 33290 | X-11945 | 1.83 | 1896 | 283.1 | 1 | + |
| 33291 | X-11946 | 1.52 | 1595 | 259.2 | 1 | + |
| 33295 | X-11949 | 3.76 | 3830 | 220.1 | 1 | + |
| 33325 | X-11979 | 2.01 | 2088 | 251.1 | 1 | + |
| 33347 | X-12001 | 1.57 | 1592 | 229.2 | 1 | - |
| 33352 | X-12006 | 2.18 | 2201 | 310.2 | 1 | - |
| 33356 | X-12010 | 1.68 | 1707 | 203.1 | 1 | - |
| 33359 | X-12013 | 2.07 | 2094 | 242.1 | 1 | - |
| 33361 | X-12015 | 1.3 | 1318 | 216.2 | 1 | - |
| 33393 | X-12042 | 1.31 | 1313 | 294 | 1 | - |
| 33398 | X-12047 | 2.65 | 2660 | 362.2 | 1 | + |
| 33405 | X-12053 | 3.24 | 3272 | 476.3 | 1 | + |
| 33511 | X-12096 | 1.53 | 1578 | 174.2 | 1 | + |
| 33512 | X-12097 | 1.48 | 1526 | 174.2 | 1 | + |
| 33514 | X-12099 | 1.35 | 1384 | 262.1 | 1 | + |
| 33515 | X-12100 | 1.76 | 1793 | 221.1 | 1 | + |
| 33516 | X-12101 | 1.6 | 1646 | 164.1 | 1 | + |
| 33519 | X-12104 | 1.72 | 1755 | 271.1 | 1 | + |
| 33523 | X-12108 | 1.42 | 1468 | 160.2 | 1 | + |
| 33528 | X-12113 | 1.69 | 1728 | 321 | 1 | + |
| 33530 | X-12115 | 1.54 | 1587 | 260.2 | 1 | + |
| 33532 | X-12117 | 1.44 | 1486 | 204.2 | 1 | + |
| 33537 | X-12122 | 1.76 | 1795 | 276.2 | 1 | + |
| 33539 | X-12124 | 1.4 | 1442 | 469.1 | 1 | + |
| 33542 | X-12127 | 1.22 | 1235 | 226.1 | 1 | + |
| 33543 | X-12128 | 1.69 | 1725 | 162.1 | 1 | + |
| 33546 | X-12131 | 3 | 3104 | 340.1 | 1 | + |
| 33590 | X-12170_200 | 2.45 | 2534 | 181.1 | 1 | + |
| 33594 | X-12173 | 1.41 | 1500 | 202.2 | 1 | + |
| 33609 | X-12188 | 2.83 | 2866 | 228.2 | 1 | - |
| 33614 | X-12193 | 3.45 | 3533 | 220 | 1 | + |
| 33620 | X-12199 | 2.94 | 3038 | 263.1 | 1 | + |
| 33627 | X-12206 | 0.64 | 654 | 255.1 | 1 | - |
| 33632 | X-12211 | 2.55 | 2582 | 295.2 | 1 | - |
| 33633 | X-12212 | 3.57 | 3607 | 229.1 | 1 | - |
| 33637 | X-12216 | 1.68 | 1701 | 228.1 | 1 | - |
| 33638 | X-12217 | 2.32 | 2343 | 203.1 | 1 | - |
| 33646 | X-12225 | 0.97 | 1009 | 143.2 | 1 | + |
| 33658 | X-12236 | 1.31 | 1321 | 245.1 | 1 | - |
| 33665 | X-12243 | 3.45 | 3533 | 279.1 | 1 | + |
| 33669 | X-12247 | 0.82 | 823 | 166.1 | 1 | - |
| 33676 | X-12254 | 2.57 | 2604 | 240 | 1 | - |
| 33683 | X-12261 | 1.83 | 1850 | 258.1 | 1 | - |
| 33704 | X-12282 | 1.31 | 1341 | 166.1 | 1 | + |
| 33728 | X-12306 | 2.34 | 2364 | 247.1 | 1 | - |
| 33745 | X-12323 | 1.31 | 1327 | 230.2 | 1 | - |
| 33764 | X-12339 | 1.02 | 1055 | 174.1 | 1 | + |
| 33765 | X-12340 | 3.3 | 3391 | 278 | 1 | + |
| 33774 | X-12349 | 0.71 | 699 | 222.2 | 1 | - |
| 33786 | X-12358 | 2.78 | 2796 | 239.9 | 1 | + |
| 33787 | X-12359 | 1.42 | 1451 | 218.1 | 1 | + |
| 33792 | X-12364 | 1.79 | 1800 | 204.1 | 1 | + |
| 33804 | X-12376 | 1.48 | 1514 | 245.2 | 1 | + |
| 33807 | X-12379 | 3.29 | 3304 | 297.2 | 1 | + |
| 33814 | X-12386 | 1 | 1001 | 216.3 | 1 | - |
| 33835 | X-12407 | 1.9 | 1902 | 205.1 | 1 | - |
| 33839 | X-12411 | 1.08 | 1077 | 195.2 | 1 | - |
| 33903 | X-12458 | 0.69 | 700 | 189.1 | 1 | + |
| 33910 | X-12465 | 1.41 | 1475 | 248.2 | 1 | + |
| 34041 | X-12511 | 4.61 | 4697 | 202.1 | 1 | + |
| 34094 | X-12534 | 9.11 | 1687 | 185.1 | Y | + |
| 34123 | X-12556 | 6.61 | 1374 | 116.9 | Y | + |
| 34124 | X-12557 | 10.12 | 1782 | 287 | Y | + |
| 34137 | X-12570 | 9.83 | 1748 | 312 | Y | + |
| 34138 | X-12571 | 2.36 | 2400 | 256.1 | 1 | + |
| 34146 | X-12579 | 6.89 | 1406 | 393 | Y | + |
| 34170 | X-12602 | 1.42 | 1456 | 204.2 | 1 | + |
| 34197 | X-12603 | 1.99 | 1878 | 397.3 | 1 | - |
| 34200 | X-12606 | 1.78 | 1673 | 353.2 | 1 | - |
| 34205 | X-12611 | 1.82 | 1860 | 290.2 | 1 | + |
| 34206 | X-12612 | 2.96 | 3020 | 416.2 | 1 | + |
| 34223 | X-12629 | 3.33 | 3396 | 520.3 | 1 | + |
| 34229 | X-12632 | 3.23 | 3290 | 490.3 | 1 | + |
| 34231 | X-12634 | 3.35 | 3409 | 548.3 | 1 | + |
| 34235 | X-12636 | 3.86 | 3890 | 259.2 | 1 | + |
| 34253 | X-12650 | 3.11 | 3147 | 446.2 | 1 | + |
| 34268 | X-12663 | 11.07 | 1895 | 359.2 | Y | + |
| 34289 | X-12680 | 0.81 | 819 | 229.3 | 1 | + |
| 34290 | X-12681 | 0.92 | 931 | 176.2 | 1 | + |
| 34291 | X-12682 | 0.93 | 939 | 589.2 | 1 | + |
| 34292 | X-12683 | 0.99 | 1004 | 675.1 | 1 | + |
| 34294 | X-12685 | 1.05 | 1060 | 154.2 | 1 | + |
| 34295 | X-12686 | 1.09 | 1101 | 181.1 | 1 | + |
| 34297 | X-12688 | 1.2 | 1210 | 203.2 | 1 | + |
| 34298 | X-12689 | 1.17 | 1183 | 278.2 | 1 | + |
| 34299 | X-12690 | 1.35 | 1386 | 346.1 | 1 | + |
| 34300 | X-12691 | 1.35 | 1405 | 360.2 | 1 | + |
| 34304 | X-12694 | 0.72 | 719 | 105.1 | 1 | - |
| 34305 | X-12695 | 0.72 | 722 | 144.1 | 1 | - |
| 34310 | X-12700 | 1.07 | 1060 | 227.1 | 1 | - |
| 34311 | X-12701 | 1.08 | 1100 | 319.1 | 1 | - |
| 34314 | X-12704 | 1.23 | 1252 | 274 | 1 | - |
| 34316 | X-12706 | 1.27 | 1280 | 223 | 1 | - |
| 34318 | X-12708 | 1.28 | 1295 | 269 | 1 | - |
| 34322 | X-12712 | 1.65 | 1690 | 219 | 1 | - |
| 34323 | X-12713 | 1.62 | 1645 | 263.1 | 1 | - |
| 34325 | X-12715 | 1.68 | 1700 | 279.1 | 1 | - |
| 34327 | X-12717 | 1.68 | 1717 | 194.1 | 1 | - |
| 34332 | X-12722 | 1.89 | 1915 | 249.1 | 1 | - |
| 34336 | X-12726 | 2.01 | 1993 | 233.1 | 1 | - |
| 34339 | X-12729 | 2.1 | 2077 | 228.1 | 1 | - |
| 34343 | X-12733 | 2.1 | 2079 | 339.2 | 1 | - |
| 34349 | X-12739 | 2.44 | 2414 | 241.2 | 1 | - |
| 34350 | X-12740 | 2.52 | 2499 | 287.1 | 1 | - |
| 34352 | X-12742 | 2.56 | 2534 | 241.2 | 1 | - |
| 34353 | X-12743 | 2.57 | 2544 | 302.2 | 1 | - |
| 34355 | X-12745 | 2.54 | 2541 | 350.1 | 1 | - |
| 34358 | X-12748 | 1.49 | 1538 | 322.1 | 1 | + |
| 34359 | X-12749 | 1.51 | 1562 | 262.1 | 1 | + |
| 34360 | X-12750 | 1.53 | 1580 | 276.2 | 1 | + |
| 34362 | X-12752 | 1.66 | 1696 | 262.2 | 1 | + |
| 34370 | X-12760 | 1.98 | 2001 | 302.2 | 1 | + |
| 34372 | X-12762 | 1.96 | 1990 | 396.1 | 1 | + |
| 34375 | X-12765 | 2.04 | 2067 | 281.2 | 1 | + |
| 34485 | X-12802 | 2.72 | 2731 | 318.2 | 1 | + |
| 34497 | X-12814 | 2.59 | 2597 | 405.2 | 1 | - |
| 34498 | X-12815 | 2.65 | 2659 | 271.1 | 1 | - |
| 34503 | X-12820 | 2.72 | 2727 | 405.2 | 1 | - |
| 34505 | X-12822 | 2.78 | 2786 | 389.1 | 1 | - |
| 34511 | X-12828 | 2.99 | 2995 | 237.2 | 1 | - |
| 34524 | X-12841 | 3.9 | 3937 | 200.2 | 1 | - |
| 34526 | X-12843 | 3.9 | 3938 | 347.2 | 1 | - |
| 34527 | X-12844 | 4.12 | 4168 | 539.3 | 1 | - |
| 34528 | X-12845 | 4.19 | 4234 | 461.3 | 1 | - |
| 34529 | X-12846 | 4.17 | 4218 | 481.3 | 1 | - |
| 34530 | X-12847 | 4.19 | 4240 | 227.1 | 1 | - |
| 34531 | X-12848 | 4.24 | 4288 | 350.1 | 1 | - |
| 34532 | X-12849 | 4.69 | 4726 | 331.2 | 1 | - |
| 34533 | X-12850 | 4.82 | 4847 | 263.8 | 1 | - |

### Example 2

### Biomarkers of Tumor Aggressiveness

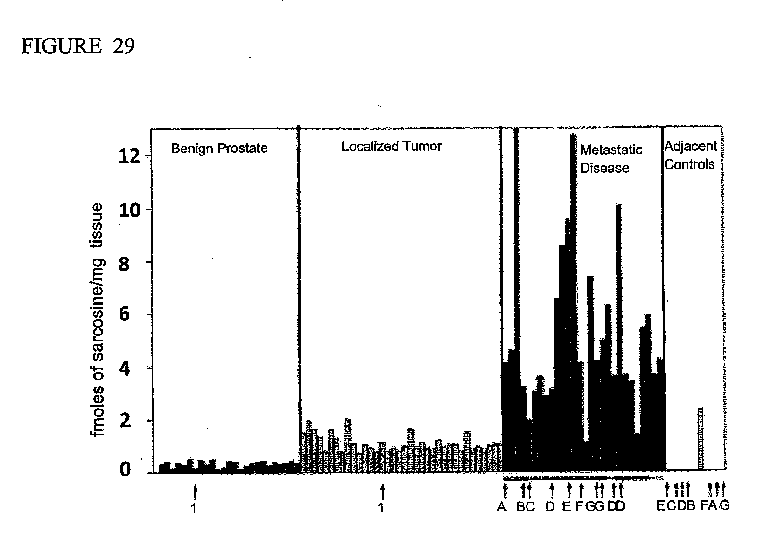

This example describes biomarkers that are useful in combination to distinguish prostate cancer tumors based on the level of tumor aggressiveness. The tissue samples used in the analysis ranged from non-aggressive (i.e., benign) to extremely aggressive (i.e., metastatic). Biomarkers were measured in benign prostate tissues (N=16), Gleason score major 3 (GS3) tumors (N=8), Gleason score major 4 (GS 4) tumors (N=4) and metastatic tumors (N=14). The levels of a four biomarker panel comprised of citrate, malate, N-acetylaspartate (NAA) and sarcosine (methylglycine) were measured in each sample. The ratio of the biomarkers citrate and malate was determined (citrate/malate). The results of the analysis show that a metabolite panel can be used to distinguish between more aggressive and less aggressive tumors and are presented in Figure 29). The metastatic tumors (most aggressive) were grouped together and were separated from the benign (non-aggressive) samples. The GS3 and GS4 samples were intermediate to the metastatic and benign, with GS4 more aggressive than GS3. The GS4 samples were closer to the metastatic samples while the GS3 were closer to the benign samples. Three GS3 samples (denoted by numbered arrows on the figure) were more closely associated with the more aggressive tumors (GS4 and metastatic). The biomarker analysis predicts that these tumors were more aggressive (higher aggressivity) than the GS3 samples that were more closely associated with the benign tissue. This prediction was supported by the clinical data associated with these samples. Based upon the clinical data, samples #1 and #2 had extra-prostatic extensions; clinically tissues were judged to be more aggressive if they have extra-prostatic extensions. None of the samples that clustered more closely to the77 benign samples had extra-prostatic extensions. Taken together, these results show that a metabolite panel can be used to distinguish benign from cancer tumors and to distinguish more aggressive from less aggressive tumors (i.e., determine cancer tumor aggressiveness).

The markers selected in the panel presented are an example of a biomarker panel combining sarcosine with other mechanism-based biomarkers. NAA is a membrane associated prostate-specific marker and citrate and malate are intermediates of the TCA cycle. In addition, this result illustrates the utility of biomarker ratios. Different combinations of metabolites, differing in number and composition and selected from the biomarkers described herein or elsewhere (e.g., PCT US2007/078805), may also be used to generate panels of metabolite77s that are useful for predicting tumor aggressiveness.

### Example 3

### Biomarkers discovered in urine

### I. General Methods

### A. Identification of Metabolic profiles for prostate cancer

Each sample was analyzed to determine the concentration of several hundred metabolites. Analytical techniques such as GC-MS (gas chromatography-mass spectrometry) and UHPLC-MS (ultra high performance liquid chromatography-mass spectrometry) were used to analyze the metabolites. Multiple aliquots were simultaneously, and in parallel, analyzed, and, after appropriate quality control (QC), the information derived from each analysis was recombined. Every sample was characterized according to several thousand characteristics, which ultimately amount to several hundred chemical species. The techniques used were able to identify novel and chemically unnamed compounds.

### B. Statistical Analysis

The data was analyzed using T-tests to identify molecules (either known, named metabolites or unnamed metabolites) present at differential levels in a definable population or subpopulation (e.g., biomarkers for prostate cancer biological samples compared to control biological samples) useful for distinguishing between the definable populations (e.g., prostate cancer and control, low grade prostate cancer and high grade prostate cancer). Other molecules (either known, named metabolites or unnamed metabolites) in the definable population or subpopulation were also identified. In some analyses the data was normalized according to creatinine levels in the samples while in other analyses the samples were not normalized. Results of both analyses are included.

### C. Biomarker identification

Various peaks identified in the analyses (e.g. GC-MS, UHPLC-MS, MS-MS), including those identified as statistically significant, were subjected to a mass spectrometry based chemical identification process. Biomarkers were discovered by (1) analyzing urine samples from different groups of human subjects to determine the levels of metabolites in the samples and then (2) statistically analyzing the results to determine those metabolites that were differentially present in the two groups.

### Biomarkers that distinguish cancer from non-cancer:

The urine samples used for the analysis were from 51 control individuals with negative biopsies for prostate cancer, and 59 individuals with prostate cancer. After the levels of metabolites were determined, the data was analyzed using the Wilcoxon test to determine differences in the mean levels of metabolites between two populations (i.e., Prostate cancer vs. Control).

As listed below in Table 10, biomarkers were discovered that were differentially present between plasma samples from subjects with prostate cancer and Control subjects with negative prostate biopsies (i.e. not diagnosed with prostate cancer).
Table 10 includes, for each listed biomarker, the p-value determined in the statistical analysis of the data concerning the biomarkers, the compound ID useful to track the compound in the chemical database and the analytical platform used to identify the compounds (GC refers to GC/MS and LC refers to UHPLC/MS/MS2). P-values that are listed as 0.000 are significant at p<0.0001.

**Table 10. Biomarkers useful to distinguish cancer from non-cancer.**

| **COMP_ID** | **COMPOUND** | **LIB_ID** | **p-value** | **% change in PCA** |
|---|---|---|---|---|
| 34404 | 1,3-7-trimethyluric acid | LCneg | 0.0457 | -61.6700 |
| 32391 | 1,3-dimethylurate | GC | 0.0188 | 264.8018 |
| 34400 | 1-7-dimethylurate | LCneg | 0.0442 | -55.8508 |
| 15650 | 1-methyladenosine | LCpos | 0.0156 | 61.7971 |
| 31609 | 1-methylguanosine | LCpos | 0.0181 | 10.9223 |
| 34395 | 1-methylurate | LCpos | 0.047 | -30.4105 |
| 22030 | 2-hydroxyisobutyrate | GC | 0.0039 | 62.9593 |
| 1432 | 2-hydroxyphenylacetate | LCneg | 0.0344 | 59.6277 |
| 32776 | 2-methylbutyroylcarnitine- | LCpos | 0.0444 | 72.8112 |
| 1431 | 3-(4-hydroxyphenyl)lactate | GC | 0.003 | 33.8077 |
| 18296 | 3-4-dihydroxyphenylacetate | GC | 0.001 | 147.8039 |
| 1566 | 3-amino-isobutyrate | GC | 0.0167 | 272.4645 |
| 32654 | 3-dehydrocarnitine- | LCpos | 0.0188 | 56.2816 |
| 32397 | 3-hydroxy-2-ethylpropionate | GC | 0.0477 | 40.3754 |
| 531 | 3-hydroxy-3-methylglutarate | GC | 4.03E-05 | 37.8097 |
| 15673 | 3-hydroxybenzoate | LCneg | 3.00E-04 | 196.7772 |
| 12017 | 3-methoxytyrosine | LCpos | 0.0069 | 95 6504 |
| 31940 | 3-methylcrotonylglycine | LCpos | 0.0102 | 62.5089 |
| 1557 | 3-methylglutarate | GC | 0.0134 | 36.0177 |
| 15677 | 3-methylhistidine | LCneg | 0.0203 | -42.0713 |
| 3155 | 3-ureidopropionate | LCpos | 0.0056 | 68.9399 |
| 1558 | 4-acetamidobutanoate | LCpos | 0.0143 | 77.3732 |
| 22115 | 4-acetylphenyl-sulfate | LCneg | 0.0467 | 100.8052 |
| 21133 | 4-hydroxybenzoate | GC | 0.0049 | 62.6825 |
| 1568 | 4-hydroxymandelate | GC | 0.0091 | 120.1023 |
| 541 | 4-hydroxyphenylacetate | GC | 0.0036 | 85.2767 |
| 22118 | 4-ureidobutyrate | LCpos | 0.0134 | 67.8751 |
| 1418 | 5,6-dihydrothymine | GC | 0.0057 | 140.1535 |
| 1559 | 5,6-dihydrouracil | GC | 0.004 | 80.4881 |
| 437 | 5-hydroxyindoleacetate | GC | 1.00E-04 | 61.2357 |
| 1419 | 5-methylthioadenosine (MTA) | LCpos | 5.00E-04 | 20.5901 |
| 1494 | 5-oxoproline | LCpos | 0.0047 | 17.9299 |
| 31580 | 7-methylguanosine | GC | 1.00E-04 | 75.7087 |
| 554 | adenine | GC | 1.00E-04 | 46.4734 |
| 555 | adenosine | LCpos | 0.0011 | 30.8684 |
| 2831 | adenosine-3',5'-cyclic-monophosphate (cAMP) | LCpos | 0.0038 | 75.5601 |
| 1126 | alanineQUM | GC | 0.0419 | 66.0477 |
| 22808 | allantoin | GC | 0.0085 | 47.1337 |
| 15142 | allo-threonine | GC | 0.0148 | 198.5838 |
| 31591 | androsterone sulfate | LCneg | 0.016 | 96.0684 |
| 575 | arabinose | GC | 2.00E-04 | 67.9778 |
| 15964 | arabitol | GC | 7.00E-04 | 46.2583 |
| 1640 | ascorbate (Vitamin C) | GC | 0.0327 | 55.6234 |
| 18362 | azelate (nonanedioate) | LCneg | 0.0478 | 118.3270 |
| 3141 | betaine | LCpos | 0.0093 | 91.2635 |
| 569 | caffeine | LCpos | 0.0179 | -70.6204 |
| 15506 | choline | LCpos | 0.0016 | 45.0093 |
| 12025 | cis-aconitate | LCpos | 0.0364 | 22.2510 |
| 22158 | citramalate | GC | 4.00E-04 | 59.4381 |
| 1564 | citrate | GC | 0.0019 | 139.2617 |
| 2132 | citrulline | GC | 4.00E-04 | 93.6606 |
| 27718 | creatine | LCpos | 4.00E-04 | 43.7043 |
| 20700 | cyanurate | GC | 0.0139 | 0.0000 |
| 31454 | cystine | GC | 0.0026 | 170.2201 |
| 32425 | dehydroisoandrosterone sulfate (DHEA-S) | LCneg | 0.0291 | 162.9464 |
| 15743 | dimethylarginine | LCpos | 2.00E-04 | 42.3710 |
| 5086 | dimethylglycine | GC | 0.0294 | 105.5877 |
| 32511 | EDTA* | LCneg | 0.005 | -10.4294 |
| 20699 | erythritol | GC | 2.45E-05 | 54.8561 |
| 33477 | erythronate* | GC | 3.10E-05 | 34.5359 |
| 577 | fructose | GC | 0.0373 | 152.8917 |
| 1643 | fumarate | GC | 3.81 E-05 | 61.1976 |
| 1117 | galactitol-dulcitol- | GC | 0.049 | -30.9639 |
| 34456 | gamma-glutamylisoleucine* | LCpos | 0.0032 | 12.7695 |
| 18369 | gamma-glutamylleucine | LCpos | 5.00E-04 | 202.0740 |
| 33422 | gammaglutamylphenylalanine | LCpos | 0.0013 | 170.8455 |
| 2734 | gamma-glutamyltyrosine | LCpos | 6.00E-04 | 199.6524 |
| 18280 | gentisate | LCneg | 0.0254 | 84.1857 |
| 1476 | glucarate (saccharate) | GC | 0.0163 | 93.0656 |
| 587 | gluconate | GC | 1.00E-04 | 49.6957 |
| 18534 | glucosamine | GC | 1.00E-04 | 56.1753 |
| 20488 | glucose | GC | 1.00E-04 | 57.0890 |
| 15443 | glucuronate | GC | 6.00E-04 | 49.1315 |
| 57 | glutamate | GC | 0.0332 | 15.2177 |
| 32393 | glutamylvaline | LCpos | 7.00E-04 | 82.6082 |
| 15990 | glycerophosphorylcholine (GPC) | LCpos | 0.0092 | 22.5740 |
| 11777 | glycineQUM | GC | 0.01 | 47.6937 |
| 15737 | glycolate (hydroxyacetate) | GC | 0.0125 | 115.3677 |
| 22171 | glycylproline | LCpos | 0.0156 | 64.5671 |
| 12359 | guanidinoacetate | GC | 3.00E-04 | 186.4843 |
| 418 | guanine | GC | 0.0129 | 80.4718 |
| 33454 | gulono-1-4-lactone | GC | 5.00E-04 | 39.8172 |
| 15753 | hippurate | LCpos | 0.032 | 50.4495 |
| 1101 | homovanillate (HVA) | GC | 0.0044 | 34.8863 |
| 3127 | hypoxanthine | LCpos | 0.0266 | 25.2729 |
| 15716 | imidazole lactate | LCpos | 4.00E-04 | 47.0735 |
| 33846 | indoleacetate* | LCpos | 0.0345 | 88.8776 |
| 18349 | indolelactate | GC | 0.0038 | 132.9586 |
| 33441 | isobutyrylcarnitine | LCpos | 0.0017 | 75.8028 |
| 1125 | isoleucine | LCpos | 0.0036 | 27.0710 |
| 34407 | isovalerylcarnitine | LCpos | 0.0046 | 42.2654 |
| 1417 | kynurenate | LCneg | 0.025 | 39.6023 |
| 15140 | kynurenine | LCpos | 0.0095 | 141.9643 |
| 11454 | lactose | GC | 0.0075 | 125.7434 |
| 60 | leucine | LCpos | 0.0088 | 26.6660 |
| 584 | mannose | GC | 0.0294 | 177.4984 |
| 18493 | mesaconate (methylfumarate) | GC | 0.008 | 85.1195 |
| 1302 | methionine | GC | 0.002 | 64.4250 |
| 34285 | monoethanolamine | GC | 0.0024 | 52.3196 |
| 33953 | N-acetylarginine | LCneg | 0.0014 | 116.6228 |
| 33942 | N-acetylasparagine | LCpos | 0.0134 | 79.3354 |
| 32195 | N-acetylaspartate (NAA) | GC | 0.0011 | 69.7707 |
| 15720 | N-acetylglutamate | LCpos | 0.009 | 41.1751 |
| 33943 | N-acetylglutamine | LCneg | 0.0294 | 65.6816 |
| 33946 | N-acetylhistidine | LCneg | 0.0046 | 81.9682 |
| 33967 | N-acetylisoleucine | LCpos | 0.0055 | 36.8144 |
| 1587 | N-acetylleucine | LCpos | 0.0042 | 107.1016 |
| 1592 | N-acetylneuraminate | GC | 0.0028 | 149.4873 |
| 33950 | N-acetylphenylalanine | LCpos | 0.0012 | 76.0267 |
| 33939 | N-acetylthreonine | LCpos | 0.026 | 89.8599 |
| 32390 | N-acetyltyrosine | LCpos | 3.00E-04 | 148.0601 |
| 1591 | N-acetylvaline | GC | 0.0035 | 148.2682 |
| 31850 | N-butyrylglycine | LCneg | 0.0356 | 46.9738 |
| 1598 | N-tigloylglycine | LCpos | 0.0186 | 36.7886 |
| 33936 | octanoylcarnitine | LCpos | 0.0063 | 32.2576 |
| 1505 | orotate | GC | 1.00E-04 | 57.3419 |
| 32558 | p-cresol sulfate* | LCneg | 0.0203 | 67.1842 |
| 32718 | phenylacetylglutamine- | LCpos | 0.0177 | 42.1472 |
| 33945 | phenylacetylglycine | LCpos | 0.0049 | 102.7455 |
| 64 | phenylalanine | LCpos | 0.0137 | 70.3716 |
| 11438 | phosphate | GC | 0.0112 | 66.4883 |
| 1512 | picolinate | GC | 0.0401 | 23.7291 |
| 1898 | proline | GC | 0.0084 | 49.8421 |
| 33442 | pseudouridine | LCpos | 0.0069 | 18.3476 |
| 1651 | pyridoxal | LCpos | 0.0212 | 77.6885 |
| 599 | pyruvate | GC | 0.0104 | 68.1170 |
| 18335 | quinate | GC | 0.0412 | 40.7535 |
| 1899 | quinolinate | LCpos | 0.0068 | 81.2769 |
| 27731 | ribonate | GC | 4.00E-04 | 61.5332 |
| 15948 | S-adenosylhomocysteine (SAH) | LCpos | 0.0108 | 84.3170 |
| 1516 | sarcosineQUM | GC | 0.0073 | 103.7037 |
| 32379 | scyllo-inositol | GC | 0.0435 | 154.8068 |
| 1648 | serine | GC | 3.00E-04 | 49.1580 |
| 485 | spermidine | LCpos | 0.0459 | -81.3755 |
| 2125 | taurine | GC | 0.0334 | 172.8511 |
| 12360 | tetrahydrobiopterin | GC | 0.0116 | 69.2047 |
| 27738 | threonate | GC | 0.0012 | 51.7428 |
| 1284 | threonine | GC | 0.0056 | 139.5883 |
| 604 | thymine | GC | 0.0034 | 161.2888 |
| 6104 | tryptamine | LCpos | 0.0372 | 62.1316 |
| 54 | tryptophan | LCpos | 0.0091 | 70.7395 |
| 1603 | tyramine | LCpos | 0.0493 | 35.8870 |
| 1299 | tyrosine | GC | 0.0011 | 58.4261 |
| 605 | uracil | GC | 0.0015 | 129.5276 |
| 607 | urocanate | LCpos | 0.0072 | 68.0070 |
| 34406 | valerylcarnitine | LCpos | 0.0306 | 120.0406 |
| 1649 | valine | LCpos | 2.00E-04 | 54.9329 |
| 1567 | vanillylmandelate-VMA- | LCneg | 0.0443 | 49.0489 |
| 3147 | xanthine | LCpos | 0.0331 | 44.5844 |
| 15136 | xanthosine | LCpos | 0.0156 | 85.5165 |
| 15679 | xanthurenate | LCpos | 0.0077 | 27.7713 |
| 15835 | xylose | GC | 0.0137 | 81.6462 |
| 32735 | X-01911_200 | LCpos | 0.0143 | 234.5459 |
| 33009 | X-01981_200 | LCpos | 0.0017 | 48.0588 |
| 32550 | X-02272_201 | LCneg | 0.0247 | 51.0244 |
| 32672 | X-02546_200 | LCpos | 5.00E-04 | 79.4250 |
| 32709 | X-03056_200 | LCpos | 0.0142 | 15.1147 |
| 32653 | X-03249_200 | LCpos | 0.0051 | 100.7635 |
| 32675 | X-03951_200 | LCpos | 6.00E-04 | 22.8452 |
| 32937 | X-03951_201 | LCneg | 4.00E-04 | 27.1295 |
| 32557 | X-06126_201 | LCneg | 0.023 | 106.4585 |
| 24332 | X-10128 | GC | 2.00E-04 | 52.5090 |
| 24469 | X-10266 | GC | 0.0032 | 38.3625 |
| 25401 | X-10359 | GC | 0.0024 | 33.6027 |
| 25402 | X-10360 | GC | 0.0262 | 44.6591 |
| 25449 | X-10385 | GC | 0.0136 | 49.8885 |
| 25607 | X-10437 | GC | 0.0474 | 86.7596 |
| 33014 | X-10457_200 | LCpos | 0.0476 | 22.6361 |
| 27883 | X-10604 | GC | 0.0077 | 43.5902 |
| 27884 | X-10605 | GC | 3.00E-04 | 40.8850 |
| 30275 | X-10738 | GC | 0.0049 | 55.5093 |
| 30276 | X-10739 | GC | 0.0034 | 82.2508 |
| 31022 | X-10831 | GC | 7.00E-04 | 67.9439 |
| 31041 | X-10835 | GC | 0.0051 | 108.0205 |
| 31053 | X-10841 | GC | 0.007 | 66.8101 |
| 31203 | X-10850 | GC | 0.0224 | 96.3934 |
| 31489 | X-10914 | GC | 0.0041 | 33.6270 |
| 31750 | X-11011 | GC | 1.00E-04 | 51.1781 |
| 31751 | X-11012 | GC | 1.00E-04 | 42.1647 |
| 31754 | X-11015 | GC | 0.002 | 43.7399 |
| 31757 | X-11018 | GC | 0.0188 | 209.6372 |
| 32026 | X-11072 | GC | 0.038 | 167.5549 |
| 32120 | X-11096 | GC | 0.0025 | 258.5659 |
| 32127 | X-11103 | GC | 0.026 | 288.9233 |
| 32562 | X-11245 | LCneg | 0.0419 | 116.4416 |
| 32578 | X-11261 | LCpos | 0.0357 | 53.5881 |
| 32599 | X-11282 | LCneg | 0.0211 | 124.6693 |
| 32649 | X-11332 | LCpos | 0.0303 | -41.3196 |
| 32650 | X-11333 | LCpos | 0.0359 | 53.6853 |
| 32664 | X-11347 | LCpos | 1.00E-04 | 30.8069 |
| 32665 | X-11348_200 | LCpos | 6.00E-04 | 37.7556 |
| 32669 | X-11352 | LCpos | 0.0163 | 51.3693 |
| 32674 | X-11357 | LCpos | 0.0314 | 55.2106 |
| 32714 | X-11397 | LCpos | 0.038 | 126.7154 |
| 32738 | X-11421 | LCpos | 0.0318 | 69.8841 |
| 32740 | X-11423 | LCneg | 0.0151 | 15.7989 |
| 32761 | X-11444 | LCneg | 3.00E-04 | 33.3214 |
| 32767 | X-11450 | LCneg | 0.0461 | 86.9345 |
| 32769 | X-11452 | LCneg | 0.0055 | 95.2700 |
| 32781 | X-11464 | LCpos | 0.0435 | 53.2915 |
| 32787 | X-11470 | LCneg | 0.027 | 13.3518 |
| 32792 | X-11475 | LCneg | 0.0032 | 292.2009 |
| 32807 | X-11490 | LCneg | 0.0092 | 91.7365 |
| 32881 | X-11564 | LCneg | 8.00E-04 | 31.9184 |
| 32910 | X-11593 | LCneg | 0.0435 | 45.1354 |
| 32957 | X-11640 | LCneg | 0.0209 | 111.1731 |
| 32996 | X-11668 | LCneg | 0.0196 | 39.8008 |
| 33031 | X-11687 | LCpos | 0.0016 | 27.7502 |
| 33033 | X-11689 | LCpos | 0.0199 | 46.8620 |
| 33090 | X-11745 | GC | 0.0318 | 35.4414 |
| 33094 | X-11749 | GC | 0.0082 | 63.4649 |
| 33100 | X-11755 | GC | 0.0023 | 48.7368 |
| 33103 | X-11758 | GC | 0.0157 | 30.5194 |
| 33106 | X-11761 | GC | 0.0034 | 61.6069 |
| 33127 | X-11782 | GC | 0.0083 | 314.9654 |
| 33171 | X-11826 | LCneg | 0.0042 | 178.7640 |
| 33188 | X-11843 | LCneg | 0.0076 | 460.0511 |
| 33195 | X-11850 | LCneg | 0.0394 | 210.3870 |
| 33280 | X-11935 | LCpos | 0.0016 | 19.1957 |
| 33281 | X-11936 | LCpos | 0.0151 | 12.3351 |
| 33290 | X-11945 | LCpos | 0.0012 | 32.5289 |
| 33291 | X-11946 | LCpos | 0.0439 | 90.4452 |
| 33325 | X-11979 | LCpos | 0.0052 | 22.8598 |
| 33347 | X-12001 | LCneg | 0.0019 | 1707811 |
| 33352 | X-12006 | LCneg | 2.00E-04 | 25.9733 |
| 33356 | X-12010 | LCneg | 0.0078 | 72.4838 |
| 33359 | X-12013 | LCneg | 0.022 | 405.5324 |
| 33393 | X-12042 | LCneg | 0.0095 | 93.4761 |
| 33398 | X-12047 | LCpos | 0.0046 | 48.5667 |
| 33405 | X-12053 | LCpos | 0.0276 | 70.0004 |
| 33511 | X-12096 | LCpos | 0.0266 | 38.6810 |
| 33512 | X-12097 | LCpos | 0.0333 | 58.4217 |
| 33514 | X-12099 | LCpos | 0.0072 | 47.4618 |
| 33515 | X-12100 | LCpos | 0.0089 | 21.6757 |
| 33516 | X-12101 | LCpos | 1.00E-04 | 83.2818 |
| 33519 | X-12104 | LCpos | 0.0177 | 11.4120 |
| 33523 | X-12108 | LCpos | 0.026 | 44.2185 |
| 33528 | X-12113 | LCpos | 0.025 | 146.1043 |
| 33532 | X-12117 | LCpos | 0.0483 | 21.8348 |
| 33537 | X-12122 | LCpos | 0.0029 | 66.5031 |
| 33539 | X-12124 | LCpos | 9.00E-04 | 29.0229 |
| 33542 | X-12127 | LCpos | 0.0068 | 123.3782 |
| 33543 | X-12128 | LCpos | 0.0167 | 43.0535 |
| 33546 | X-12131 | LCpos | 0.0086 | 0.0000 |
| 33590 | X-12170_200 | LCpos | 0.003 | 23.1150 |
| 33594 | X-12173 | LCpos | 0.0417 | -52.8764 |
| 33609 | X-12188 | LCneg | 0.0277 | 80.8620 |
| 33614 | X-12193 | LCpos | 0.0114 | 140.4048 |
| 33620 | X-12199 | LCpos | 0.0109 | 195.2826 |
| 33627 | X-12206 | LCneg | 0.0095 | 15.5730 |
| 33632 | X-12211 | LCneg | 0.0038 | 217.1225 |
| 33633 | X-12212 | LCneg | 0.0361 | 220.1253 |
| 33638 | X-12217 | LCneg | 0.0266 | 42.5603 |
| 33646 | X-12225 | LCpos | 6.00E-04 | 20.7575 |
| 33658 | X-12236 | LCneg | 0.0258 | 109.4350 |
| 33669 | X-12247 | LCneg | 0.0156 | 38.0283 |
| 33676 | X-12254 | LCneg | 0.0315 | 229.5867 |
| 33683 | X-12261 | LCneg | 0.0224 | 215.2098 |
| 33704 | X-12282 | LCpos | 0.0032 | 78.5452 |
| 33728 | X-12306 | LCneg | 0.0356 | 115.0007 |
| 33745 | X-12323 | LCneg | 0.0191 | 36.7940 |
| 33764 | X-12339 | LCpos | 0.023 | 50.4166 |
| 33765 | X-12340 | LCpos | 0.0386 | 131.2436 |
| 33786 | X-12358 | LCpos | 0.0019 | 39.9305 |
| 33787 | X-12359 | LCpos | 0.0022 | 108.4776 |
| 33792 | X-12364 | LCpos | 0.015 | 52.5728 |
| 33804 | X-12376 | LCpos | 0.0037 | 52.2176 |
| 33807 | X-12379 | LCpos | 0.0335 | 84.0021 |
| 33814 | X-12386 | LCneg | 0.0028 | 79.8037 |
| 33835 | X-12407 | LCneg | 0.0419 | 102.2921 |
| 33839 | X-12411 | LCneg | 0.0469 | 181.1927 |
| 33903 | X-12458 | LCpos | 0.0454 | 3.8204 |
| 34041 | X-12511 | LCpos | 0.014 | 67.0961 |
| 34094 | X-12534 | GC | 0.0114 | 23.0764 |
| 34123 | X-12556 | GC | 0.0014 | 38.9741 |
| 34124 | X-12557 | GC | 0.0069 | 133.5437 |
| 34137 | X-12570 | GC | 6.00E-04 | 23.4172 |
| 34146 | X-12579 | GC | 0.0166 | 36.6870 |
| 34197 | X-12603 | LCneg | 0.0486 | 93.9915 |
| 34200 | X-12606 | LCneg | 0.0239 | 84.7583 |
| 34205 | X-12611 | LCpos | 0.0024 | 36.6540 |
| 34206 | X-12612 | LCpos | 0.0403 | 100.6866 |
| 34223 | X-12629 | LCpos | 0.0228 | 64.2063 |
| 34229 | X-12632 | LCpos | 0.0345 | 65.5474 |
| 34231 | X-12634 | LCpos | 0.0339 | 74.2212 |
| 34235 | X-12636 | LCpos | 0.0113 | 30.6322 |
| 34253 | X-12650 | LCpos | 0.0228 | 70.5815 |
| 34268 | X-12663 | GC | 0.0186 | 149.0884 |
| 34289 | X-12680 | LCpos | 0.0249 | 116.7362 |
| 34290 | X-12681 | LCpos | 0.0345 | 53.3469 |
| 34291 | X-12682 | LCpos | 0.0266 | 25.1312 |
| 34292 | X-12683 | LCpos | 0.0025 | 36.9150 |
| 34294 | X-12685 | LCpos | 0.0474 | 70.8178 |
| 34295 | X-12686 | LCpos | 0.0052 | 15.6282 |
| 34297 | X-12688 | LCpos | 0.0029 | 124.9182 |
| 34298 | X-12689 | LCpos | 0.0256 | 20.8243 |
| 34299 | X-12690 | LCpos | 0.0019 | 16.8796 |
| 34300 | X-12691 | LCpos | 0.016 | 81.0894 |
| 34304 | X-12694 | LCneg | 0.0292 | 30.3117 |
| 34305 | X-12695 | LCneg | 0.0083 | 51.2191 |
| 34310 | X-12700 | LCneg | 0.005 | 85.1265 |
| 34311 | X-12701 | LCneg | 0.0451 | 63.6861 |
| 34314 | X-12704 | LCneg | 0.0252 | 243.6844 |
| 34316 | X-12706 | LCneg | 0.0413 | 156.8494 |
| 34318 | X-12708 | LCneg | 0.015 | 79.9730 |
| 34322 | X-12712 | LCneg | 0.0487 | 79.2438 |
| 34325 | X-12715 | LCneg | 0.0049 | 55.2094 |
| 34327 | X-12717 | LCneg | 0.012 | 203.4073 |
| 34336 | X-12726 | LCneg | 0.0146 | 66.2239 |
| 34339 | X-12729 | LCneg | 0.0299 | 117.3626 |
| 34343 | X-12733 | LCneg | 0.0108 | 43.8603 |
| 34349 | X-12739 | LCneg | 0.0014 | 89.0934 |
| 34350 | X-12740 | LCneg | 0.0282 | 405.1284 |
| 34352 | X-12742 | LCneg | 0.0199 | 70.2457 |
| 34353 | X-12743 | LCneg | 6.38E-06 | 70.0243 |
| 34355 | X-12745 | LCneg | 0.0045 | 1230.4546 |
| 34358 | X-12748 | LCpos | 1.09E-05 | 68.9382 |
| 34359 | X-12749 | LCpos | 0.0196 | 14.6434 |
| 34360 | X-12750 | LCpos | 0.0452 | 34.9301 |
| 34362 | X-12752 | LCpos | 0.002 | 28.4767 |
| 34370 | X-12760 | LCpos | 0.007 | 41.6076 |
| 34375 | X-12765 | LCpos | 0.0016 | 57.1255 |
| 34485 | X-12802 | LCpos | 0.0031 | 47.2186 |
| 34497 | X-12814 | LCneg | 0.0349 | 216.9783 |
| 34498 | X-12815 | LCneg | 0.0497 | 98.1436 |
| 34503 | X-12820 | LCneg | 0.0467 | 348.8805 |
| 34505 | X-12822 | LCneg | 0.012 | 64.5382 |
| 34511 | X-12828 | LCneg | 0.0107 | 74.3241 |
| 34524 | X-12841 | LCneg | 0.0049 | 165.1258 |
| 34526 | X-12843 | LCneg | 0.0018 | 432.1185 |
| 34527 | X-12844 | LCneg | 0.0029 | 30.9475 |
| 34528 | X-12845 | LCneg | 0.0161 | 162.3770 |
| 34529 | X-12846 | LCneg | 0.0306 | 27.5410 |
| 34530 | X-12847 | LCneg | 0.0306 | 254.3334 |
| 34531 | X-12848 | LCneg | 0.0147 | 259.3802 |
| 34532 | X-12849 | LCneg | 0.022 | 232.6990 |
| 34533 | X-12850 | LCneg | 0.0106 | 152.3123 |
| 12603 | X-2980 | GC | 0.0435 | 150.0623 |
| 12770 | X-3090 | GC | 0.047 | 49.3716 |
| 16062 | X-4015 | GC | 5.00E-04 | 97.5835 |
| 16821 | X-4498 | GC | 5.00E-04 | 59.0953 |
| 16822 | X-4499 | GC | 2.00E-04 | 65.9952 |
| 16829 | X-4503 | GC | 0.0389 | 448.9493 |
| 16831 | X-4504 | GC | 0.0017 | 34.7506 |
| 16837 | X-4507 | GC | 0.0104 | 33.7584 |
| 16866 | X-4523 | GC | 2.00E-04 | 163.4988 |
| 16984 | X-4599 | GC | 0.0033 | 76.7293 |
| 17050 | X-4618 | GC | 0.0085 | 32.9874 |
| 17064 | X-4624 | GC | 0.0052 | 55.2961 |
| 17072 | X-4628 | GC | 0.0075 | 272.1564 |
| 17074 | X-4629 | GC | 1.00E-04 | 57.5233 |
| 17086 | X-4637 | GC | 6.00E-04 | 181.6876 |
| 17088 | X-4639 | GC | 0.0064 | 88.5308 |
| 18232 | X-5403 | GC | 0.0032 | 32.1164 |
| 18251 | X-5409 | GC | 0.0042 | 39.1551 |
| 18253 | X-5410 | GC | 0.017 | 355.5448 |
| 18257 | X-5412 | GC | 0.0104 | 48.5322 |
| 18264 | X-5414 | GC | 0.0032 | 135.2663 |
| 18265 | X-5415 | GC | 0.0171 | 40.2508 |
| 18271 | X-5418 | GC | 3.00E-04 | 65.0484 |
| 18272 | X-5419 | GC | 0.0082 | 49.3174 |
| 18273 | X-5420 | GC | 2.00E-04 | 50.7034 |
| 18307 | X-5431 | GC | 0.0046 | 267.5213 |
| 18309 | X-5433 | GC | 0.0094 | 131.5460 |
| 18316 | X-5437 | GC | 0.0075 | 142.7695 |
| 18388 | X-5491 | GC | 4.19E-05 | 58.3225 |
| 18390 | X-5492 | GC | 8.00E-04 | 46.4359 |
| 18419 | X-5506 | GC | 0.027 | 65.4907 |
| 18430 | X-5511 | GC | 0.0199 | 107.8683 |
| 18438 | X-5518 | GC | 0.0117 | 1692.6298 |
| 18442 | X-5522 | GC | 0.002 | 45.8239 |
| 19954 | X-6906 | GC | 1.00E-04 | 34.3189 |
| 19960 | X-6912 | GC | 0.0031 | 36.2744 |
| 19965 | X-6928 | GC | 0.0191 | 38.2332 |
| 19969 | X-6931 | GC | 0.0136 | 225.7159 |
| 19973 | X-6946 | GC | 0.003 | 126.2096 |
| 19984 | X-6956 | GC | 4.00E-04 | 77.8832 |
| 19990 | X-6962 | GC | 0.0149 | 42.7975 |
| 19997 | X-6969 | GC | 0.0037 | 545.8663 |
| 20014 | X-6985 | GC | 0.0474 | 106.4077 |
| 20020 | X-6991 | GC | 0.015 | 49.2941 |
| 22308 | X-8886 | GC | 0.0452 | 118.3757 |
| 22494 | X-8994 | GC | 0.017 | 567.8661 |
| 22548 | X-9026 | GC | 0.002 | 125.0265 |
| 22570 | X-9033 | GC | 0.0329 | 85.2545 |
| 22881 | X-9287 | GC | 0.0101 | 85.5217 |
| 24074 | X-9706 | GC | 0.0042 | 46.6887 |
| 24076 | X-9726 | GC | 0.0331 | 50.6677 |

The cancer status (i.e. non-cancer or cancer) of individual subjects was determined using the biomarkers sarcosine and N-acetyl tyrosine. Using these two markers in combination resulted in cancer diagnosis with 83% sensitivity and 49% specificity. Assuming a 30% prevalence of cancer in a PSA positive population, these biomarkers gave a Negative Predictive Value (NPV) of 87% and a Positive Predictive Value (PPV) of 41%.

### Biomarkers that distinguish less aggressive cancer from more aggressive cancer:

The urine samples used for the analysis were obtained from individuals diagnosed with prostate cancer having biopsy scores of GS major 3 or GS major 4 and above. GSmajor3 indicates a lower grade of cancer that is typically less aggressive while GS major 4 indicates a higher grade of cancer that is typically more aggressive. In this analysis the GS major 3 subjects (N=45) were compared to subjects with a GS major 4 (N=13). After the levels of metabolites were determined, the data was analyzed using the Wilcoxon test to determine differences in the mean levels of metabolites between two populations (i.e., Prostate cancer vs. Control).

As listed below in Table 11, biomarkers were discovered that were differentially present between urine samples from subjects with less aggressive/lower grade prostate cancer and subjects with more aggressive/higher grade prostate cancer. Table 11 includes, for each listed biomarker, the p-value determined in the statistical analysis of the data concerning the biomarkers, the compound ID useful to track the compound in the chemical database and the analytical platform used to identify the compounds (GC refers to GC/MS and LC refers to UHPLC/MS/MS2). P-values that are listed as 0.000 are significant at p<0.0001.

**Table 11. Biomarkers that distinguish less aggressive from more aggressive prostate cancer.**

| **COMP_ID** | **COMPOUND** | **Platform** | **p-value** | **% Change in Aggressive PCA** |
|---|---|---|---|---|
| 34404 | 1,3-7-trimethyluric acid | LCneg | 0.0057 | -66.55113998 |
| 34400 | 1-7-dimethylurate | LCneg | 0.001 | -62.28917254 |
| 15650 | 1-methyladenosine | LCpos | 0.0254 | 43.02217774 |
| 34395 | 1-methylurate | LCpos | 4.00E-04 | -49.79665561 |
| 34389 | 1-methylxanthine | LCpos | 0.0138 | -67.90592259 |
| 15667 | 2-isopropylmalate | LCneg | 0.0469 | 166.2876883 |
| 18296 | 3-4-dihydroxyphenylacetate | GC | 0.0014 | 123.2216303 |
| 27672 | 3-indoxyl-sulfate | LCneg | 0.0138 | -23.7469546 |
| 12017 | 3-methoxytyrosine | LCpos | 0.0113 | 86.24357623 |
| 15677 | 3-methylhistidine | LCneg | 0.0059 | 102.3968054 |
| 32445 | 3-methylxanthine | LCpos | 0.0132 | -72.50497601 |
| 3155 | 3-ureidopropionate | LCpos | 0.022 | 27.56547555 |
| 1558 | 4-acetamidobutanoate | LCpos | 0.0166 | 59.98174305 |
| 15681 | 4-guanidinobutanoate | LCpos | 0.0297 | 174.6765122 |
| 21133 | 4-hydroxybenzoate | GC | 0.01 | 71.09064956 |
| 1568 | 4-hydroxymandelate | GC | 0.0208 | 89.80468995 |
| 22118 | 4-ureidobutyrate | LCpos | 0.017 | 60.30878737 |
| 437 | 5-hydroxyindoleacetate | GC | 0.0226 | 84.94805375 |
| 1494 | 5-oxoproline | LCpos | 0.0056 | -29.70497615 |
| 31580 | 7-methylguanosine | GC | 0.0347 | 84.95194026 |
| 555 | adenosine | LCpos | 0.0111 | 79.86819651 |
| 2831 | adenosine-3',5'-cyclic-monophosphate (cAMP) | LCpos | 0.0136 | 53.42430461 |
| 15142 | allo-threonine | GC | 5.00E-04 | 307.6014316 |
| 575 | arabinose | GC | 0.0079 | 148.4557 |
| 15964 | arabitol | GC | 0.0441 | 98.60829547 |
| 1640 | ascorbate (Vitamin C) | GC | 0.045 | 175.9986664 |
| 18362 | azelate (nonanedioate) | LCneg | 0.0186 | 207.3082051 |
| 3141 | betaineQUM | LCpos | 0.0019 | 111.1077205 |
| 569 | caffeine | LCpos | 0.0075 | -81.71522011 |
| 12025 | cis-aconitate | LCpos | 0.0369 | -25.83372809 |
| 1564 | citrate | GC | 0.0153 | 159.3164801 |
| 27718 | creatine | LCpos | 0.0062 | 239.6294824 |
| 513 | creatinine | LCpos | 0.0291 | 77.95100223 |
| 32425 | dehydroisoandrosterone sulfate (DHEA-S) | LCneg | 0.0272 | 153.7895042 |
| 5086 | dimethylglycine | GC | 0.0084 | 89.87003058 |
| 1643 | fumarate | GC | 0.023 | -27.15601216 |
| 1117 | galactitol-dulcitol- | GC | 0.0036 | 352.7349757 |
| 34456 | gamma-glutamylisoleucine* | LCpos | 0.0198 | 83.47303345 |
| 18369 | gamma-glutamylleucine | LCpos | 8.00E-04 | 100.8835487 |
| 33422 | gammaglutamylphenylalanine | LCpos | 8.00E-04 | 116.4623197 |
| 2734 | gamma-glutamyltyrosine | LCpos | 0.0018 | 199.6523546 |
| 1476 | glucarate (saccharate) | GC | 0.0413 | 78.73546464 |
| 587 | gluconate | GC | 0.0337 | 135.3595762 |
| 15443 | glucuronate | GC | 0.048 | 79.98123372 |
| 32393 | glutamylvaline | LCpos | 0.005 | 53.61399238 |
| 15365 | glycerol 3-phosphate (G3P) | GC | 0.0095 | 96.65755153 |
| 15990 | glycerophosphorylcholine (GPC) | LCpos | 0.043 | -30.99560024 |
| 11777 | glycine | GC | 0.0047 | 51.51603573 |
| 15737 | glycolate (hydroxyacetate) | GC | 0.0219 | 103.7720467 |
| 22171 | glycylproline | LCpos | 0.0081 | 81.31832313 |
| 12359 | guanidinoacetate | GC | 0.0015 | 163.1261154 |
| 33454 | gulono-1-4-lactone | GC | 0.0413 | 61.59491649 |
| 1101 | homovanillate (HVA) | GC | 0.0081 | 87.32242401 |
| 21025 | iminodiacetate-IDA- | GC | 0.021 | 44.48398584 |
| 33846 | indoleacetate* | LCpos | 0.0362 | 105.8783175 |
| 18349 | indolelactate | GC | 0.0332 | 101.7860312 |
| 33441 | isobutyrylcarnitine | LCpos | 0.0279 | 55.35226019 |
| 12110 | isocitrate | LCpos | 0.0422 | -41.41198939 |
| 1125 | isoleucine | LCpos | 0.0208 | 54.70179416 |
| 15140 | kynurenine | LCpos | 0.0191 | 132.392076 |
| 527 | lactate | GC | 0.0337 | -29.28603115 |
| 11454 | lactose | GC | 0.0117 | 108.8417975 |
| 60 | leucine | LCpos | 0.0332 | 44.16653491 |
| 584 | mannose | GC | 0.0158 | 108.0495974 |
| 18493 | mesaconate (methylfumarate) | GC | 0.0452 | -48.02028356 |
| 1302 | methionine | GC | 0.01 | 93.23111101 |
| 34285 | monoethanolamine | GC | 0.0363 | 159.4495524 |
| 33953 | N-acetylarginine | LCneg | 0.0317 | 85.9617038 |
| 32195 | N-acetylaspartate (NAA) | GC | 0.0379 | 94.62417064 |
| 33946 | N-acetylhistidine | LCneg | 0.0058 | 59.11465726 |
| 1587 | N-acetylleucine | LCpos | 0.0227 | 85.37871881 |
| 33950 | N-acetylphenylalanine | LCpos | 0.0095 | 66.64423652 |
| 33939 | N-acetylthreonine | LCpos | 0.0332 | 78.16412969 |
| 32390 | N-acetyltyrosine | LCpos | 0.0057 | 133.7952527 |
| 1591 | N-acetylvaline | GC | 0.0463 | 66.01491718 |
| 18254 | paraxanthine | LCpos | 0.0219 | -63.90495686 |
| 33945 | phenylacetylglycine | LCpos | 0.006 | 90.17463794 |
| 64 | phenylalanine | LCpos | 0.0254 | 57.32016167 |
| 33442 | pseudouridine | LCpos | 0.0231 | 54.52078056 |
| 1651 | pyridoxal | LCpos | 0.0268 | 54.86441025 |
| 599 | pyruvate | GC | 0.0071 | 62.1494331 |
| 1899 | quinolinate | LCpos | 0.006 | 61.91679621 |
| 27731 | ribonate | GC | 0.0394 | 100.3888599 |
| 15948 | S-adenosylhomocysteine (SAH) | LCpos | 0.0344 | 62.81234124 |
| 1516 | sarcosine | GC | 0.0021 | 89.65517241 |
| 1648 | serine | GC | 0.0337 | 80.59915169 |
| 603 | spermine | LCpos | 0.0247 | -78.26667362 |
| 18392 | theobromine | LCpos | 0.0165 | -80.1429027 |
| 27738 | threonate | GC | 0.0396 | 94.31081416 |
| 1284 | threonine | GC | 0.0118 | 77.88106938 |
| 604 | thymine | GC | 0.0157 | 71.13143504 |
| 54 | tryptophan | LCpos | 0.0162 | 80.30828074 |
| 1299 | tyrosine | GC | 0.008 | 99.33740457 |
| 605 | uracil | GC | 0.0318 | 75.86987921 |
| 32701 | urate- | LCpos | 0.0482 | -49.86065084 |
| 607 | urocanate | LCpos | 0.0219 | 55.53807526 |
| 1649 | valine | LCpos | 0.0266 | 132.4327688 |
| 15835 | xylose | GC | 0.0219 | 79.58039821 |
| 32672 | X-02546_200 | LCpos | 0.0124 | 39.92995063 |
| 32653 | X-03249_200 | LCpos | 0.0347 | 50.52155844 |
| 32675 | X-03951_200 | LCpos | 0.0461 | 77.31945011 |
| 32937 | X-03951_201 | LCneg | 0.0404 | 84.92252578 |
| 24469 | X-10266 | GC | 0.0276 | 73.92296217 |
| 25402 | X-10360 | GC | 0.0347 | 79.71371779 |
| 33014 | X-10457_200 | LCpos | 0.0369 | 26.87901527 |
| 27884 | X-10605 | GC | 0.0379 | 117.0583917 |
| 31751 | X-11012 | GC | 0.0266 | 126.3470402 |
| 31754 | X-11015 | GC | 0.0396 | 60.66427028 |
| 32026 | X-11072 | GC | 0.0204 | 111.0816308 |
| 32120 | X-11096 | GC | 0.002 | 246.5355958 |
| 32562 | X-11245 | LCneg | 0.022 | 147.5795427 |
| 32631 | X-11314 | LCpos | 0.0347 | -38.84300738 |
| 32649 | X-11332 | LCpos | 0.0059 | 104.0484707 |
| 32651 | X-11334 | LCpos | 0.0321 | 69.54121645 |
| 32652 | X-11335 | LCpos | 0.0379 | 65.56679429 |
| 32665 | X-11348_200 | LCpos | 0.0369 | 71.33451227 |
| 32714 | X-11397 | LCpos | 0.0277 | -67.48708723 |
| 32754 | X-11437 | LCneg | 0.0047 | 1257.122467 |
| 32767 | X-11450 | LCneg | 0.0363 | 79.38640823 |
| 32792 | X-11475 | LCneg | 0.0031 | 366.4908828 |
| 32807 | X-11490 | LCneg | 0.0466 | 84.13891831 |
| 32827 | X-11510 | LCneg | 0.015 | 137.5062988 |
| 32878 | X-11561 | LCneg | 0.0347 | 39.08827189 |
| 32978 | X-11656 | LCpos | 0.045 | -55.75256194 |
| 33171 | X-11826 | LCneg | 0.0064 | 144.2554847 |
| 33280 | X-11935 | LCpos | 0.0293 | 61.44828759 |
| 33281 | X-11936 | LCpos | 0.0266 | 53.18088504 |
| 33290 | X-11945 | LCpos | 0.0461 | 51.88262935 |
| 33291 | X-11946 | LCpos | 0.0433 | 57.82662663 |
| 33295 | X-11949 | LCpos | 0.0321 | -26.25001217 |
| 33325 | X-11979 | LCpos | 0.0278 | 48.01647625 |
| 33352 | X-12006 | LCneg | 0.0304 | 73.56750455 |
| 33356 | X-12010 | LCneg | 0.0083 | 233.0064131 |
| 33361 | X-12015 | LCneg | 0.0158 | 106.0732039 |
| 33393 | X-12042 | LCneg | 0.0173 | 74.91590711 |
| 33398 | X-12047 | LCpos | 0.0219 | 55.34246459 |
| 33514 | X-12099 | LCpos | 0.0129 | 47.01102723 |
| 33516 | X-12101 | LCpos | 0.0103 | -36.00760478 |
| 33530 | X-12115 | LCpos | 0.0441 | -33.02940864 |
| 33537 | X-12122 | LCpos | 0.0253 | 49.52870476 |
| 33539 | X-12124 | LCpos | 0.0347 | 46.14882349 |
| 33542 | X-12127 | LCpos | 0.0254 | 89.89660466 |
| 33543 | X-12128 | LCpos | 0.0034 | -55.28552444 |
| 33609 | X-12188 | LCneg | 0.0071 | -77.72107587 |
| 33614 | X-12193 | LCpos | 0.0063 | 116.7744629 |
| 33620 | X-12199 | LCpos | 0.0254 | 161.7656256 |
| 33632 | X-12211 | LCneg | 0.0216 | 203.3196007 |
| 33633 | X-12212 | LCneg | 0.033 | 280.5910199 |
| 33637 | X-12216 | LCneg | 0.0118 | -52.22252608 |
| 33638 | X-12217 | LCneg | 0.0482 | -39.44206727 |
| 33646 | X-12225 | LCpos | 0.0075 | 59.98551337 |
| 33665 | X-12243 | LCpos | 0.0253 | -47.60623384 |
| 33676 | X-12254 | LCneg | 0.0191 | 415.8798474 |
| 33704 | X-12282 | LCpos | 0.0059 | 58.42472716 |
| 33764 | X-12339 | LCpos | 0.0413 | 40.70759506 |
| 33774 | X-12349 | LCneg | 0.0198 | -25.18575014 |
| 33787 | X-12359 | LCpos | 0.0111 | 93.83073384 |
| 33804 | X-12376 | LCpos | 0.0124 | 58.66527499 |
| 33814 | X-12386 | LCneg | 0.0136 | 108.2300401 |
| 33835 | X-12407 | LCneg | 0.0489 | 55.24997178 |
| 33839 | X-12411 | LCneg | 0.019 | 87.92801957 |
| 33910 | X-12465 | LCpos | 0.0218 | 0 |
| 34041 | X-12511 | LCpos | 0.0179 | 89.02312659 |
| 34094 | X-12534 | GC | 0.0369 | 15.74666369 |
| 34123 | X-12556 | GC | 0.0386 | 55.12702293 |
| 34137 | X-12570 | GC | 0.029 | 72.94401006 |
| 34138 | X-12571 | LCpos | 0.0461 | -51.97060823 |
| 34170 | X-12602 | LCpos | 0.0327 | 33.15918309 |
| 34268 | X-12663 | GC | 0.0265 | 82.0191453 |
| 34289 | X-12680 | LCpos | 0.045 | 93.83428843 |
| 34290 | X-12681 | LCpos | 0.0431 | 67.59059032 |
| 34292 | X-12683 | LCpos | 0.0468 | 76.11571819 |
| 34294 | X-12685 | LCpos | 0.0128 | 114.0988325 |
| 34295 | X-12686 | LCpos | 0.0461 | 54.50094449 |
| 34297 | X-12688 | LCpos | 0.0084 | 100.1303934 |
| 34299 | X-12690 | LCpos | 0.0353 | 74.54432605 |
| 34300 | X-12691 | LCpos | 0.0325 | 67.30133053 |
| 34305 | X-12695 | LCneg | 0.0321 | 52.64061636 |
| 34310 | X-12700 | LCneg | 0.0073 | 102.1108558 |
| 34311 | X-12701 | LCneg | 0.0428 | 159.9798899 |
| 34322 | X-12712 | LCneg | 0.0362 | 107.510855 |
| 34323 | X-12713 | LCneg | 0.0253 | 141.1585404 |
| 34332 | X-12722 | LCneg | 0.0181 | 120.1175671 |
| 34339 | X-12729 | LCneg | 0.0428 | 210.5959332 |
| 34343 | X-12733 | LCneg | 0.0037 | -57.78309079 |
| 34349 | X-12739 | LCneg | 0.0198 | -37.87433792 |
| 34350 | X-12740 | LCneg | 0.0158 | 441.3133411 |
| 34352 | X-12742 | LCneg | 0.0307 | -48.53620833 |
| 34353 | X-12743 | LCneg | 0.0138 | 155.1605436 |
| 34355 | X-12745 | LCneg | 0.0354 | 471.2309818 |
| 34358 | X-12748 | LCpos | 0.0461 | -13.09684771 |
| 34359 | X-12749 | LCpos | 0.0242 | -23.31492948 |
| 34360 | X-12750 | LCpos | 0.0297 | 26.42009682 |
| 34372 | X-12762 | LCpos | 0.0412 | 178.3117468 |
| 34497 | X-12814 | LCneg | 0.04 | 170.9153319 |
| 34498 | X-12815 | LCneg | 0.0242 | 98.14355773 |
| 34505 | X-12822 | LCneg | 0.0325 | 43.0072576 |
| 34524 | X-12841 | LCneg | 0.0182 | 189.4742509 |
| 34526 | X-12843 | LCneg | 0.0066 | 118.568709 |
| 34528 | X-12845 | LCneg | 0.023 | 162.3770256 |
| 34532 | X-12849 | LCneg | 0.0143 | 173.837207 |
| 34533 | X-12850 | LCneg | 0.0233 | 138.2604803 |
| 12785 | X-3103 | GC | 0.0482 | -47.31496658 |
| 16062 | X-4015 | GC | 0.0037 | 43.60275909 |
| 16831 | X-4504 | GC | 0.0321 | 120.6164818 |
| 17086 | X-4637 | GC | 0.0028 | 281.0902182 |
| 18251 | X-5409 | GC | 0.0191 | 71.87489485 |
| 18264 | X-5414 | GC | 0.015 | 90.0100388 |
| 18265 | X-5415 | GC | 0.0413 | 101.7549199 |
| 18316 | X-5437 | GC | 0.0053 | 128.193364 |
| 18388 | X-5491 | GC | 0.023 | -31.91685364 |
| 19960 | X-6912 | GC | 0.0242 | 129.4486593 |
| 19965 | X-6928 | GC | 0.0317 | 125.0950831 |
| 19969 | X-6931 | GC | 0.0278 | 180.8662725 |
| 19973 | X-6946 | GC | 0.0061 | 149.537457 |
| 19990 | X-6962 | GC | 0.0413 | 34.36068338 |
| 19997 | X-6969 | GC | 0.0145 | 545.8663231 |
| 22320 | X-8889 | GC | 0.0441 | 41.201698 |
| 22494 | X-8994 | GC | 0.0236 | 805.8059769 |
| 22570 | X-9033 | GC | 0.0219 | -94.82653652 |
| 24074 | X-9706 | GC | 0.0482 | 35.47108011 |

### Example 4

### Role of Sarcosine in Prostate Cancer Progression

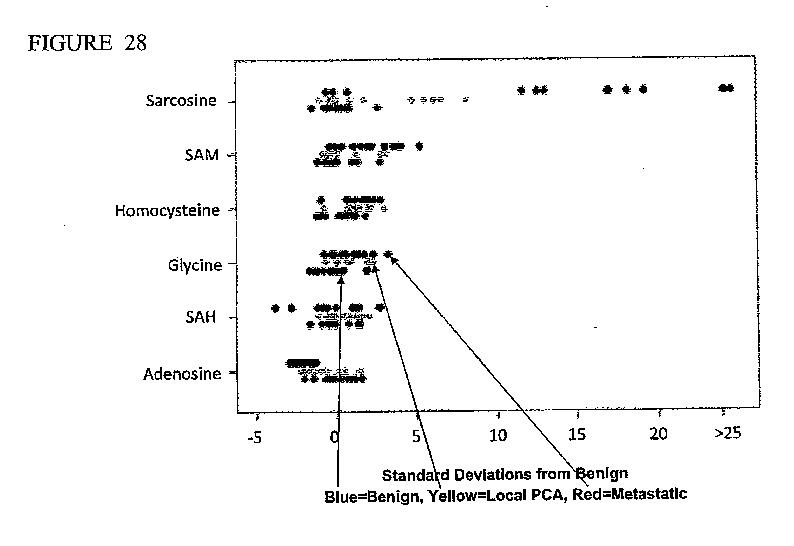

The studies described above on metabolomic profiling of prostate cancer identified sarcosine, a.k.a. N-methylglycine as being upregulated during prostate cancer progression (Figure 28). This was validated in independent tissue specimens using isotope dilution GC-MS (Figure 29). The biomarker potential of sarcosine was reflected in its elevated levels in urine (both sediment and supernatant) from biopsy positive prostate cancer patients compared to biopsy negative controls (Figures 12 and 13). To understand the role of sarcosine in prostate cancer progression, levels of the metabolite was measured in a panel of prostate-derived cell lines. Elevated level of sarcosine was found in prostate cancer cell lines compared to their benign counterparts). Sarcosine levels correlated well with the extent of invasion exhibited by the prostate cancer cell lines in an invitro Boyden chamber assay. Further, sarcosine levels were elevated upon overexpression of either EZH2 or ETS family of transcription factors in benign epithelial cells, both of which made the cells invasive (Figure 4b,c). This confirmed the role of sarcosine in inducing an invasive phenotype in prostate cancer cells. Addition of sarcosine to benign prostate epithelial cells made them invasive strengthening its role as an inducer of invasive phenotype in tumors (Figure 4 d). To characterize this observation further knock down studies of enzymes that lead to sarcosine generation or breakdown were performed in prostate derived cell lines. The knock down studies were carried out using specific siRNA and the extent of target inhibition was assessed using Q-PCR. The in vitro Boyden chamber assay was used to qualify the modulation in the invasiveness of the knock down cells. 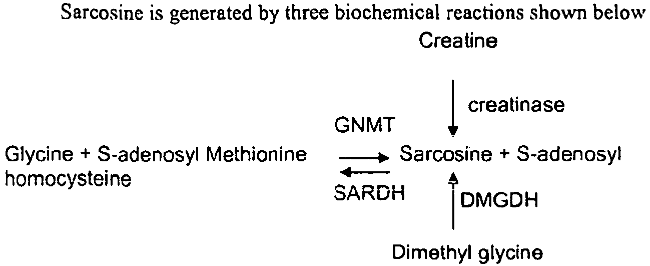 Of these glycine-N-methyl transferase (GNMT) acts as the major biosynthetic enzyme for sarcosine generation while sarcosine dehydrogenase is the predominant demethylating enzyme.

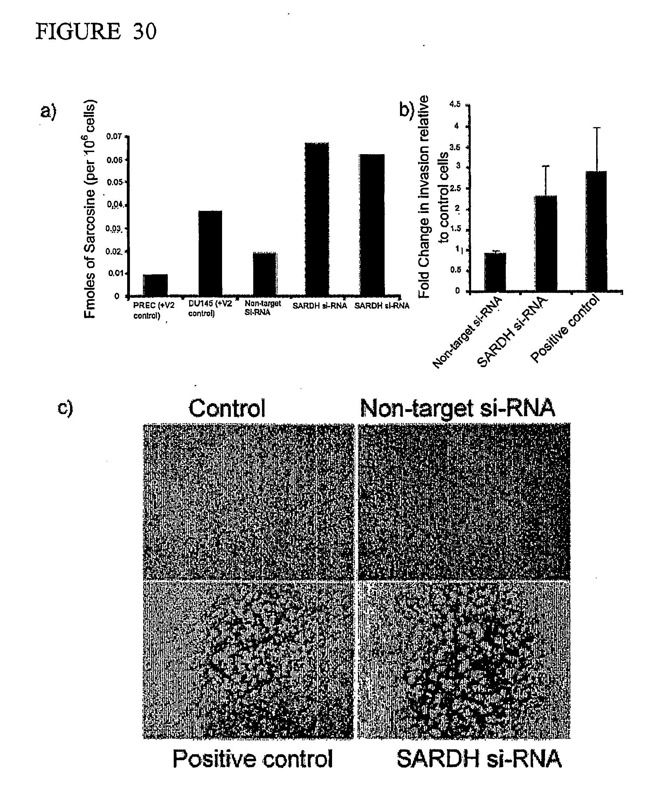

Knock down of GNMT in invasive prostate cancer cell line (DU145) resulted in a significant reduction in the invasiveness with a concomitant decrease in the levels of sarcosine (Figure 4 e). In a similar experiment RWPE cells harboring GNMT knockdown could be made invasive only upon addition of sarcosine but not glycine highlighting the importance of sarcosine in inducing tumor invasion (Figure 4 f). Further, knock down of SARDH (an enzyme that catalyzes sarcosine breakdown) in RWPE cells imparted an invasive phenotype to these benign epithelial cells with a concomitant accumulation of sarcosine (Figure 30). These data demonstrate the importance of sarcosine in potentiating invasion in prostate cancer tumors.

## Claims

1. A method of diagnosing prostate cancer, comprising:
a) detecting the presence or absence or the level of sarcosine and N-acetyl tyrosine in a tissue or urine sample from a subject; and
b) diagnosing prostate cancer based on the presence, absence or level of sarcosine and N-acetyl tyrosine in the sample.

2. The method of claim 1, wherein said tissue sample is a biopsy sample.

3. The method of claim 1, wherein step a) further comprises detecting the presence or absence or the level of citrate and/or malate.

## Patentansprüche

1. Verfahren zur Diagnose von Prostatakrebs, umfassend:
a) die Detektion der Anwesenheit oder der Abwesenheit oder des Spiegels von Sarcosin und N-Acetyltyrosin in einer Gewebeprobe oder einer Urinprobe von einem Subjekt; und
b) die Diagnose von Prostatakrebs basierend auf der Anwesenheit, der Abwesenheit oder dem Spiegel von Sarcosin- und N-Acetyltyrosin in der Probe.

2. Verfahren nach Anspruch 1, wobei die Gewebeprobe eine Biopsieprobe ist.

3. Verfahren nach Anspruch 1, wobei Schritt a) des Weiteren die Detektion der Anwesenheit oder Abwesenheit oder des Spiegels von Citrat- und/oder Malat umfasst.

## Revendications

1. Procédé de diagnostic d'un cancer de la prostate, comprenant :
a) la détection de la présence ou de l'absence ou du niveau de sarcosine et de N-acétyl tyrosine dans un échantillon de tissu ou d'urine d'un sujet ; et
b) le diagnostic du cancer de la prostate sur la base de la présence, de l'absence ou du niveau de sarcosine et de N-acétyl tyrosine dans l'échantillon.

2. Procédé selon la revendication 1, dans lequel ledit échantillon de tissu est un échantillon de biopsie.

3. Procédé selon la revendication 1, dans lequel l'étape a) comprend en outre la détection de la présence ou de l'absence ou du niveau de citrate et/ou de malate.
